# EUROPÄISCHE PATENTSCHRIFT

(11) **EP 1 432 388 B1**
(45) Veröffentlichungstag und Bekanntmachung des Hinweises auf die Patenterteilung: **18.06.2008**
(21) Anmeldenummer: 02772344.4
(22) Anmeldetag: 25.09.2002
(51) Int. Cl.: A61K 8/49, A61Q 5/10

(54) **MITTEL ZUM FÄRBEN VON KERATINHALTIGEN FASERN**
AGENT FOR COLOURING FIBRES CONTAINING KERATIN
AGENT POUR COLORER DES FIBRES KERATINIQUES

(30) Priorität: 04.10.2001 DE 10148844
(43) Veröffentlichungstag der Anmeldung: 30.06.2004
(73) Patentinhaber: Henkel AG & Co. KGaA, 40589 Düsseldorf (DE)
(72) Erfinder: GROSS, Wibke, 40549 Düsseldorf (DE); OBERKOBUSCH, Doris, 40591 Düsseldorf (DE); MÜLLER, Helmut, 40591 Düsseldorf (DE); HÖFFKES, Horst, 40595 Düsseldorf (DE); MÖLLER, Hinrich, 40789 Monheim (DE)
(86) Internationale Anmeldenummer: PCT/EP2002/010736
(87) Internationale Veröffentlichungsnummer: WO 2003/030847

(56) Entgegenhaltungen:
- EP-A- 0 873 744
- WO-A-00/38633
- WO-A-01/10379
- WO-A-01/47483
- WO-A-01/85111
- DE-A- 19 936 911

## Beschreibung

Die Erfindung betrifft ein Mittel zum Färben von keratinhaltigen Fasern, insbesondere menschlichen Haaren, das kationische heterozyklische Verbindungen und CH-acide Verbindungen enthält, die Verwendung dieser Kombination in Mitteln zum Färben von keratinhaltigen Fasern sowie ein Verfahren zum Färben von keratinhaltigen Fasern, insbesondere menschlichen Haaren.

Für das Färben von keratinhaltigen Fasern kommen im Allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im Allgemeinen unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Der Verbraucher verlangt von einem Färbemittel eine lange anhaltende und gleichmäßige Färbung und eine gute physiologische Verträglichkeit. Gerade bei jungen Menschen sind zusätzlich ausgefallene Farbtöne und spezielle Effekte auf dem Haar beliebt. Einen solchen Spezialeffekt bieten beispielsweise fluoreszierende Haarfarben.

Aufgabe der vorliegenden Erfindung ist es, Färbemittel für Keratinfasern, insbesondere menschliche Haare, bereitzustellen, die eine ausgefallene und/oder eine fluoreszierende Färbung bewirken. Des weiteren sollen die Färbemittel hinsichtlich der Farbtiefe, der Grauabdeckung und den Echtheitseigenschaften qualitativ den üblichen Oxidationshaarfärbemitteln mindestens gleichwertig sein, ohne jedoch unbedingt Oxidationsmittel wie z. B. H₂O₂ zu benötigen. Darüber hinaus dürfen die Färbemittel kein oder lediglich ein sehr geringes Sensibilisierungspotential aufweisen.

Überraschenderweise wurde nun gefunden, daß sich die Kombination aus kationischen heterozyklischen Verbindungen gemäß Formel I bzw. II und CH-aciden Verbindungen auch in Abwesenheit von oxidierenden Agentien hervorragend zum Färben von keratinhaltigen Fasern eignet. Der Einsatz von oxidierenden Agentien soll jedoch nicht prinzipiell ausgeschlossen werden. Es ergeben sich Ausfärbungen mit hervorragender Brillanz und Farbtiefe in vielfältigen Farbnuancen, insbesondere über einen Nuancenbereich von gelb über gelbbraun, orange, braunorange, rot, rotbraun, violett bis hin zu blau. Die Färbungen können fluoreszieren, besonders unter der Zuhilfenahme von UV-Licht (Schwarzlicht).

Haarfärbemittel mit einer Kombination aus CH-aciden Verbindungen und kationischen heterozyklischen Verbindungen gemäß Formel I bzw. II sind dem Fachmann nicht bekannt.

Gegenstand der Erfindung ist ein Mittel zum Färben von keratinhaltigen Fasern, insbesondere menschlichen Haaren, enthaltend
A. mindestens eine quatemierte heterozyklische Verbindung gemäß Formel I, 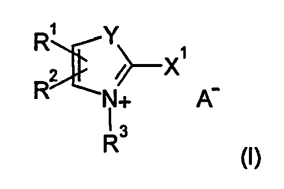 worin
   - R¹ und R² stehen unabhängig voneinander für ein Wasserstoffatom, ein Halogenatom, eine Hydroxygruppe, eine C₁-C₄-Hydroxyalkylgruppe, eine C₁-C₆-Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine Sulfonsäuregruppe, eine Carboxylgruppe, eine Nitrogruppe, eine Cyanogruppe oder eine Gruppe -NR⁴R⁵, wobei R⁴ und R⁵ stehen unabhängig voneinander für ein Wasserstoffatom, eine C₁-C₆-Alkylgruppe, eine C₂-C₆-Alkenylgruppe, eine Aryl-C₁-C₄-alkylgruppe oder eine C₁-C₄-Hydroxyalkylgruppe, wobei R¹ und R² zusammen einen ankondensierten 5- oder 6-gliedrigen, aliphatischen oder aromatischen Ring bilden können, welcher wiederum mit den Resten R⁶ und R⁷ substituiert ist; wobei R⁶ und R⁷ stehen unabhängig voneinander für die Reste, die unter R¹ definiert sind,
   - R³ steht für eine C₁-C₄-Hydroxyalkylgruppe, eine C₁-C₆-Aminoalkylgruppe, eine C₁-C₄-Dialkylamino-C₁-C₄-alkylgruppe, eine Di(C₁-C₄-hydroxyalkyl)amino-C₁-C₄-alkylgruppe, eine lineare oder verzweigte C₁-C₆-Alkylgruppe, eine C₂-C₆-Alkenylgruppe, eine gegebenenfalls substituierte Arylgruppe,
   - A⁻ steht für ein Chlorid, Bromid, lodid, Hexafluorophosphat, Tetrachlorozinkat, Tetrafluoroborat, Trifluormethylsulfonat, Methylsulfonat oder p-Toluolsulfonat,
   - Y steht für ein Sauerstoffatom, ein Schwefelatom, eine Gruppe -N=CH-, eine gegebenenfalls substituierte Methylen- oder eine gegebenenfalls substituierte Vinylengruppe oder eine Gruppe NR⁸, wobei R⁸ für die gleichen Gruppen stehen kann, die unter R⁵ definiert sind,
   - X¹ steht für ein Halogenatom, eine C₁-C₄-Alkoxygruppe, eine C₁-C₄-Alkylmercaptogruppe, eine Sulfonsäuregruppe oder eine p-Toluolsulfonylgruppe,
   mit der Maßgabe, daß wenn Y eine gegebenenfalls substituierte Vinylengruppe ist, die Verbindungen der Formel II 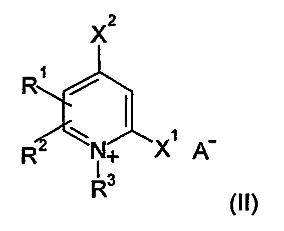 wobei
   - R¹, R², R³ und A⁻ wie oben definiert sind,
   - einer der Reste X¹ oder X² steht für ein Halogenatom, eine C₁-C₄-Alkoxygruppe, eine C₁-C₄-Alkylmercaptogruppe, eine Sulfonsäuregruppe oder eine p-Toluolsulfonylgruppe und der andere für ein Wasserstoffatom oder die Gruppen, die unter R¹ und R² definiert sind,
      mitumfaßt sind,
   sowie entsprechenden inneren Salzen, wobei A⁻ entfällt, und
B. mindestens eine CH-acide Verbindung gemäß eines des Formeln C1 bis C21.

Beispiele für die als Substituenten im Rahmen dieser Anmeldung genannten C₁-C₆-Alkylreste sind die Gruppen Methyl, Ethyl, Propyl, Isopropyl, n-Butyl, sec-Butyl, tert.-Butyl, n-Pentyl, Neopentyl und Hexyl. Ethyl und Methyl sind bevorzugte Alkylreste. Beispiele für bevorzugte C₂-C₆-Alkenylreste sind Vinyl und Allyl. Erfindungsgemäß bevorzugte C₁-C₄-Alkoxyreste sind beispielsweise eine Methoxy- oder eine Ethoxygruppe. Bevorzugte C₁-C₄-Mercaptogruppen sind die Methylmercapto oder die Ethylmercaptogruppe; die Methylmercaptogruppe ist besonders bevorzugt. Weiterhin können als bevorzugte Beispiele für eine C₁-C₆-Hydroxyalkylgruppe eine Hydroxymethyleine 2-Hydroxyethyl-, eine 2-Hydroxypropyl, eine 3-Hydroxypropyl-, eine 4-Hydroxybutylgruppe, eine 1,2-Dihydroxyethylgruppe und die 2,3-Dihydroxypropylgruppe genannt werden. Eine 2-Hydroxyethylgruppe und eine 2,3-Dihydroxypropylgruppe sind besonders bevorzugt. Bevorzugte Arylgruppen sind Phenyl, Naphthyl und Biphenyl. Beispiele für Halogenatome sind F-, Cl-, oder Br-Atome, wobei Cl-Atome ganz besonders bevorzugt sind. Bevorzugte C₁-C₄-Aminoalkylgruppen sind die Aminomethyl-, die Aminoethyl und die Aminopropylgruppe. Beispiele für bevorzugte C₁-C₄-DialkylaminO-C₁-C₄-alkylgruppen sind 2-(N,N-Dimethylamino)ethyl, 2-(N,N-Diethylamino)ethyl, 3-(N,N-Dimethylamino)propyl und 3-(N,N-Diethylamino)propyl. Beispiele für eine Aryl-C₁-C₄-alkylgruppe sind Benzyl und 2-Phenylethyl. Die weiteren verwendeten Begriffe leiten sich erfindungsgemäß von den hier gegebenen Definitionen ab.

Die Verbindungen mit den Formeln 1 und 11 sind zum großen Teil literaturbekannt, im Handel erhältlich oder nach bekannten Syntheseverfahren herstellbar.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Bevorzugte Verbindungen gemäß Formel I bzw. Formel II sind Substanzen, in denen das Kation ausgewählt ist aus 2-Chlor-1-ethyl-chinolinium, 4-Chlor-1-ethyl-chinolinium, 2-Chlor-1-methyl-pyridinium, 4-Chlor-1-methyl-pyridinium, 3-Ethyl-2-methylmercaptobenzothiazolium, 3-Ethyl-2-methylmercapto-benzoxazolium, 2-Chlor-1,3-diethylbenzimidazolium, 2-Chlor-3-ethyl-benzoxazolium oder 2-Chlor-3-ethyl-benzothiazolium und das Gegenion A⁻ ausgewählt ist aus Chlorid, Bromid, lodid, Hexafluorophosphat, Tetrachlorozinkat, Tetrafluoroborat, Trifluormethylsulfonat, Methylsulfonat oder p-Toluolsulfonat sowie den inneren Salzen 3-Ethyl-benzothiazolium-2-sulfonat, 1-Ethyl-chinolinium-4-sulfonat und 1-Ethyl-chinolinium-2-sulfonat.

Besonders bevorzugte Verbindungen gemäß Formel I bzw. II sind 4-Chlor-1-ethylchinolinium-tetrafluororborat, 3-Ethyl-2-methylmercapto-benzothiazolium-tetrafluoroborat und 3-Ethyl-benzothiazolium-2-sulfonat.

Die CH-acide Verbindung ist ausgewählt aus Verbindungen gemäß einer der Formeln C1 bis C21

M¹-CH₂-M² (C1)

worin
- M¹ eine Gruppe -COM³, COOM³, S(O)M³ oder SO₂M³ ist, worin M³ für ein Wasserstoffatom oder eine C₁-C₆-Alkylgruppe steht, oder eine Gruppe - C(M⁴)=C(C≡N)₂ bedeutet, worin M⁴ für ein Wasserstoffatom, eine C₁-C₄-Alkylgruppe oder eine Arylgruppe steht,
- M² die gleiche Bedeutung wie M¹ hat oder eine Cyanogruppe, eine substituierte oder unsubstituierte Arylgruppe oder Aryl-C₁-C₄-alkylgruppe, ein substituierter oder unsubstituierter, gesättigter oder ungesättigter Heterozyklus ist,

Verbindungen mit der Formel C2 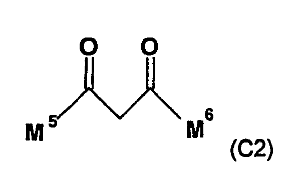 worin
- M⁵ eine Cyanogruppe, eine substituierte oder unsubstituierte Arylgruppe oder eine Aryl-C₁-C₄-alkylgruppe, ein substituierter oder unsubstituierter, gesättigter oder ungesättigter Heterozyklus oder eine Gruppe -COM⁷ oder COOM⁷ ist, worin M⁷ für ein Wasserstoffatom oder eine C₁-C₆-Alkylgruppe steht,
- M⁶ eine substituierte oder unsubstituierte C₁-C₆-Alkylgruppe, eine Acetyloxygruppe, eine C₃-C₆-Cycloalkylgruppe, eine substituierte oder unsubstituierte Arylgruppe oder Aryl-C₁-C₄-alkylgruppe, eine substituierte oder unsubstituierte Aminoarylgruppe, ein substituierter oder unsubstituierter, gesättigter oder ungesättigter Heterozyklus ist,

Verbindungen mit der Formel C3 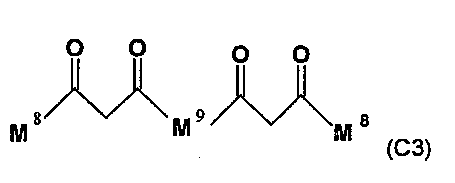 worin
- M⁸ eine Cyanogruppe, eine substituierte oder unsubstituierte Arylgruppe oder Aryl-C₁-C₄-alkylgruppe, ein substituierter oder unsubstituierter, gesättigter oder ungesättigter Heterozyklus oder eine Gruppe -COM¹⁰ oder COOM¹⁰ ist, worin M¹⁰ für ein Wasserstoffatom oder eine C₁-C₆-Alkylgruppe steht,
- M⁹ eine substituierte oder unsubstituierte Arylgruppe oder Aryl-C₁-C₄-alkylgruppe, eine substituierte oder unsubstituierte Aminoarylgruppe, ein substituierter oder unsubstituierter, gesättigter oder ungesättigter Heterozyklus ist,
   Pyrazolderivate (a), die ausgewählt sein können aus den folgenden Formeln C4 und C5 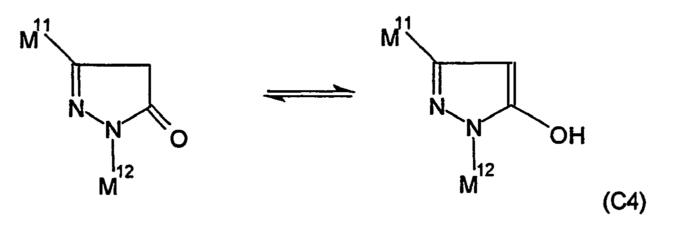 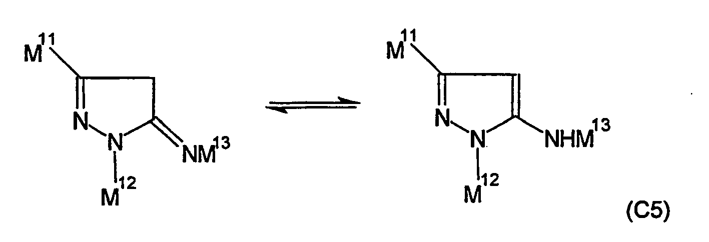
   worin
   - M¹¹ und M¹² unabhängig voneinander für eine substituierte oder unsubstituierte C₁-C₆-Alkylgruppe, eine Acetyloxygruppe, eine C₃-C₆-Cycloalkylgruppe, eine substituierte oder unsubstituierte Arylgruppe oder Aryl-C₁-C₄-alkylgruppe, eine substituierte oder unsubstituierte Aminoarylgruppe, einen substituierter oder unsubstituierter, gesättigten oder ungesättigten Heterozyklus stehen,
   - M¹³ ein Wasserstoffatom oder eine substituierte oder unsubstituierte C₁-C₆-Alkylgruppe ist,
      (b) zwei über M¹¹ oder M¹² gebundene Pyrazolringe mit der Formel C4 oder C5

Barbitursäurederivate mit der folgenden Formel C6 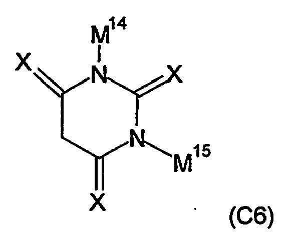 worin
- M¹⁴ und M¹⁵ stehen unabhängig voneinander für eine C₁-C₆-Alkylgruppe, eine C₂-C₆-Alkenylgruppe, eine C₃-C₆-Cycloalkylgruppe, eine Aryl-C₁-C₄-alkylgruppe, eine substituierte oder unsubstituierte Arylgruppe, oder einen über die Reste M¹⁴ oder M¹⁵ gebundenen Bicyclus,
- X steht für ein Sauerstoff- oder ein Schwefelatom,

Pyridinderivate mit der Formeln C7a und C7b 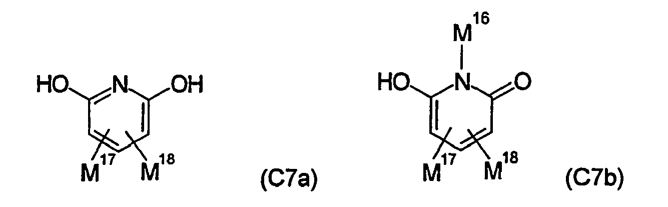 worin
- M¹⁶ eine substituierte oder unsubstituierte C₁-C₆-Alkylgruppe oder substituierte oder unsubstituierte Arylgruppe ist,
- M¹⁷ ein Wasserstoffatom, eine substituierte oder unsubstituierte C₁-C₆-Alkylgruppe oder eine substituierte oder unsubstituierte Arylgruppe ist,
- M¹⁸ ein Wasserstoffatom, eine Cyanogruppe, eine substituierte oder unsubstituierte C₁-C₆-Alkylgruppe, eine Gruppe COOM¹⁹, worin M¹⁹ ein Wasserstoffatom oder eine substituierte oder unsubstituierte C₁-C₆-Alkylgruppe bedeutet, ist,

Verbindungen mit der folgenden Formel C8 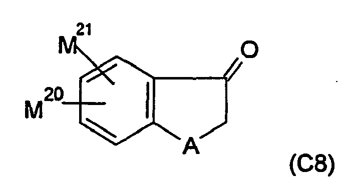 worin
- A steht für ein Sauerstoffatom, ein Schwefelatom, eine Sulfoxylgruppe, eine Sulfonylgruppe oder eine Gruppe NM^{20a}, worin M^{20a} ein Wasserstoffatom, eine substituierte oder unsubstituierte C₁-C₆-Alkylengruppe bedeutet,
- M²⁰ und M²¹ stehen unabhängig voneinander für ein Wasserstoff-, ein Chlor-, ein Bromatom, eine Hydroxygruppe, eine Nitrogruppe, eine C₁-C₆-Alkylgruppe, eine C₁-C₆-Alkoxy-, eine Carboxamid-, eine Sulfonamid-, eine Carboxyl-, eine C₁-C₄-Acyl, eine Cyanogruppe oder eine Aminogruppe -NM²²M²³, in der M²² und M²³ unabhängig voneinander stehen für ein Wasserstoffatom oder eine C₁-C₆-Alkylgruppe,

Verbindungen mit den Formeln C9 und C10 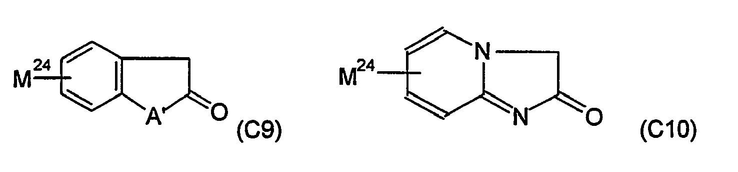 worin
- A' steht für ein Sauerstoffatom, ein Schwefelatom oder eine Gruppe NM²⁵, worin M²⁵ ein Wasserstoffatom oder eine substituierte oder unsubstituierte C₁-C₆-Alkylgruppe bedeutet,
- M²⁴ steht für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM²⁶M²⁷, in der M²⁶ und M²⁷ unabhängig voneinander stehen für Wasserstoff und eine C₁-C₆-Alkylgruppe

Verbindungen mit der Formel C11 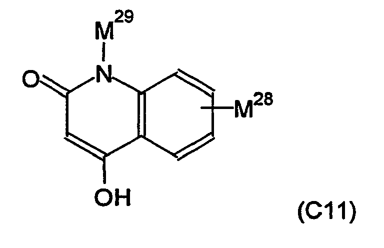 worin
- M²⁸ ein Wasserstoffatom, eine Hydroxygruppe, eine substituierte oder unsubstituierte C₁-C₆-Alkylgruppe oder eine substituierte oder unsubstituierte Aryl- oder C₁-C₆-Alkylarylgruppe ist;
- M²⁹ steht für ein Wasserstoffatom oder eine C₁-C₄-Alkylgruppe

Indandionderivate mit der Formel C12 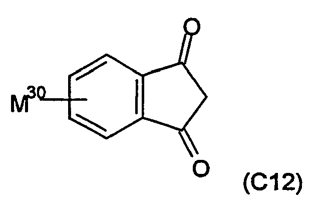 worin
- M³⁰ ein Wasserstoff-, ein Chlor-, ein Bromatom, eine Nitro-, eine C₁-C₆-Alkyl-, eine C₁-C₆-Alkoxy-, eine Carboxamid-, eine Sulfonamid- oder eine Cyanogruppe ist,

Verbindungen mit der Formel C13 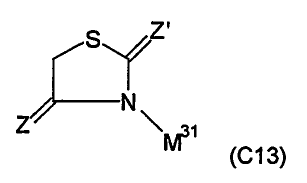 worin
- Z steht für ein Sauerstoffatom oder eine Gruppe NM³², worin M³² ein Wasserstoffatom oder eine C₁-C₆-Alkylgruppe bedeutet,
- Z' steht für ein Schwefelatom oder eine Gruppe NM³³, worin M³³ ein Wasserstoffatom oder eine C₁-C₆Alkylgruppe bedeutet,
- M³¹ steht für ein Wasserstoffatom, eine C₁-C₆-Alkylgruppe oder eine C₁-C₄-Carboxyalkylgruppe,

Dioxopyrazolverbindungen mit der Formel C14 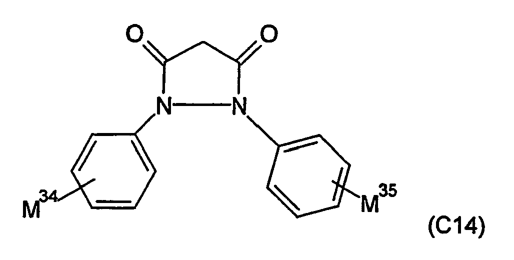 worin
- M³⁴ und M³⁵ stehen unabhängig voneinander für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM³⁶M³⁷, in der M³⁶ und M³⁷ unabhängig voneinander stehen für Wasserstoff oder eine C₁-C₆-Alkylgruppe,

5-Oxoimidazolderivate mit der Formel C15 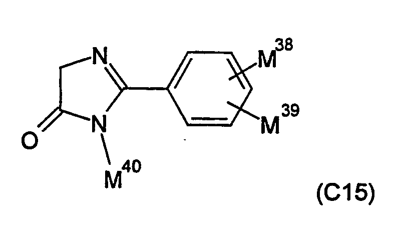 worin
- M³³ und M³⁹ stehen unabhängig voneinander für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM⁴¹M⁴², in der M⁴¹ und M⁴² unabhängig voneinander stehen für ein Wasserstoffatom oder eine C₁-C₆Alkylgruppe,
- M⁴⁰ steht für ein Waserstoffatom oder eine C₁-C₆-Alkylgruppe,

Derivate von Dehydrobutyrolacton mit der Formel C16 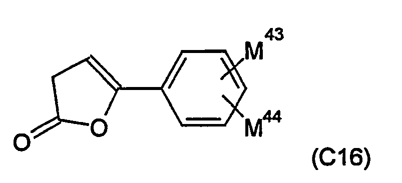 worin
- M⁴³ und M⁴⁴ unabhängig voneinander stehen für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM⁴⁵M⁴⁶, in der M⁴⁵ und M⁴⁶ unabhängig voneinander stehen für ein Wasserstoffatom oder eine C₁-C₆-Alkylgruppe

Verbindungen mit der Formel C17 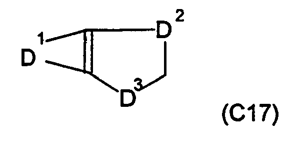 worin
- D¹ ist ein ankondensierter aromatischer oder heteroaromatischer Ring,
- D² ist eine Carbonylgruppe, eine Gruppe C=CD'D" oder eine Gruppe CD^{I}D^{II}, in welchen D^{I} oder D^{II} jeweils ein Substituent mit einer Hammett-Konstante zwischen 0,4 und 2,0 oder beide Substituenten in der Summe eine Hammett-Konstante zwischen 0,4 und 2,0 aufweisen;
- D³ steht für eine Carbonylgruppe, ein Sauerstoff-, ein Schwefelatom, eine Gruppe NM⁴⁷, wenn D² nicht Sauerstoff ist, oder eine Gruppe C=S, eine Gruppe C=NR⁴⁸, eine Sulfinylgruppe, eine Sulfonylgruppe, wobei R⁴⁷ und R⁴⁸ unabhängig voneinander ein Wasserstoffatom oder einen C₁-C₄-Alkylrest bedeuten,

Hydroxypyrimidinderivate mit der Formel C18 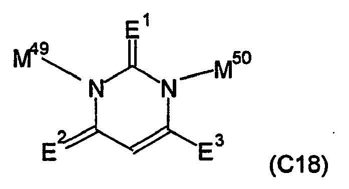 worin
- M⁴⁹ und M⁵⁰ unabhängig voneinander ein Wasserstoffatom oder eine substituierte oder unsubstituierte C₁-C₆-Alkylgruppe sind,
- E¹ für ein Sauerstoff, ein Schwefelatom oder eine Gruppe NH steht,
- E² für eine Gruppe NH oder ein Sauerstoffatom steht,
- E³ für eine Aminogruppe oder eine Hydroxygruppe steht,
mit der Maßgabe, daß
a) wenn E¹ und E² für ein Sauerstoffatom stehen, E³ keine Hydroxygruppe ist, und
b) wenn E¹ ein Schwefelatom und E² ein Sauerstoffatom ist, E³ keine Hydroxygruppe ist,
quatemierte Stickstoffverbindungen der Formel C19 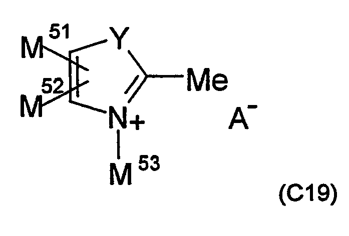 worin,
- M⁵¹ und M⁵² stehen unabhängig voneinander für ein Wasserstoffatom, ein Halogenatom, eine Hydroxygruppe, eine C₁-C₄-Hydroxyalkylgruppe, eine C₁-C₆-Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine Sulfonsäuregruppe, eine Carboxylgruppe, eine Formylgruppe, eine Nitrogruppe, eine Cyanogruppe oder eine Gruppe -NM⁵⁴M⁵⁵,
wobei M⁵⁴und M⁵⁵ stehen unabhängig voneinander für ein Wasserstoffatom, eine C₁-C₆-Alkylgruppe, eine C₂-C₆-Alkenylgruppe, eine Aryl-C₁-C₄-alkylgruppe oder eine C₁-C₄-Hydroxyalkylgruppe, wobei M⁵¹ und M⁵² zusammen einen ankondensierten 5- oder 6-gliedrigen, aliphatischen oder aromatischen bzw. heteroaromatischen Ring bilden können, welcher wiederum mit den Resten M⁵⁶ und M⁵⁷ substituiert ist, wobei M⁵⁶ und M⁵⁷ stehen unabhängig voneinander für die Reste, die unter M⁵¹ definiert sind,
- M⁵³ steht für ein Wasserstoffatom, eine C₁-C₄-Hydroxyalkylgruppe, eine C₁-C₆-Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine C₁-C₄-Sulfonylalkylgruppe, eine C₁-C₄-Carboxyalkylgruppe oder eine C₂-C₆-Polyhydroxyalkylgruppe,
- Y steht für ein Sauerstoffatom, ein Schwefelatom, eine gegebenenfalls substituierte Methylengruppe oder eine Gruppe NM⁶⁰, wobei M⁶⁰ für die gleichen Gruppen stehen kann, die unter M⁵⁵ definiert sind,
- A steht für ein Chlorid, Bromid, lodid, Hexafluorophosphat, Tetrachlorozinkat, Tetrafluoroborat, Trifluormethylsulfonat, Methylsulfonat oder p-Toluolsulfonat,

Oniumverbindungen der Formeln C20 und C21 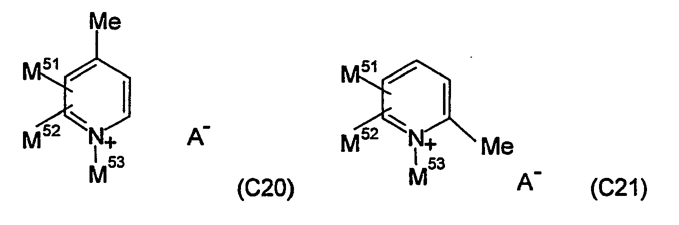 wobei M⁵¹, M⁵², M⁵³ und A⁻ aus den Gruppen ausgewählt werden, die unter Verbindung C19 definiert sind.

In einer besonders bevorzugten Ausführungsform sind die CH-aciden Verbindungen ausgewählt aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 1-Methyl-3-phenyl-pyrazolin-5-on, Indan-1,2-dion, Indan-1,3-dion, Indan-1-on, Benzoylacetonitril, 3-Dicyanmethylenindan-1-on, 1,3-Diiminoisoindolin, 2-Amino-4-imino-1,3-thiazolin-hydrochlorid, 5,5-Dimethylcyclohexan-1,3-dion, 2H-1,4-Benzoxazin-4H-3-on, , 3-Ethyl-2-methyl-benzoxazoliumiodid, 3-Ethyl-2-methylbenzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methylbenzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Färbungen mit noch erhöhter Brillanz und verbesserten Echtheitseigenschaften (Lichtechtheit, Waschechtheit, Reibechtheit) über einen weiten Nuancenbereich werden erzielt, wenn die erfindungsgemäß eingesetzten Verbindungen der Formeln I bzw II gemeinsam mit mindestens einer weiteren Komponente (im folgenden Komponente C genannt), ausgewählt aus (a) aromatischen oder heteroaromatischen Aldehyden oder Ketonen, (b) Aminosäuren, (c) aus 2 bis 9 Aminosäuren aufgebauten Oligopeptiden sowie (d) Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen und deren physiologisch verträglichen Salzen verwendet werden. Dies sind einerseits Verbindungen, die für sich alleine keratinhaltige Fasern nur schwach färben und erst gemeinsam mit den Verbindungen der Formeln I bzw. II brillante Färbungen ergeben.

Andererseits sind darunter aber auch Verbindungen, die bereits als Oxidationsfarbstoffvorprodukte eingesetzt werden.

In einer zweiten Ausführungsform ist es daher bevorzugt, dem Färbemittel zusätzlich eine Komponente C, enthaltend mindestens eine Verbindung ausgewählt aus (a) aromatischen oder heteroaromatischen Aldehyden oder Ketonen, (b) Aminosäuren, (c) aus 2 bis 9 Aminosäuren aufgebauten Oligopeptiden und (d) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, zu addieren.

Bevorzugt enthält die Komponente C mindestens eine Verbindung ausgewählt aus aromatischen oder heteroaromatischen Aldehyden oder Ketonen.

Bevorzugt eingesetzte aromatische oder heteroaromatische Aldehyde oder Ketone sind ausgewählt aus
- 5-(4-Dimethylaminophenyl)-penta-2,4-dienal, 5-(4-Diethylaminophenyl)-penta-2,4-dienal, 5-(4-Methoxyphenyl)-penta-2,4-dienal, 5-(3,4-Dimethoxyphenyl)-penta-2,4-dienal, 5-(2,4-Dimethoxyphenyl)-penta-2,4-dienal, 5-(4-Piperidinophenyl)-penta-2,4-dienal, 5-(4-Morpholinophenyl)-penta-2,4-dienal, 5-(4-Pyrrolidinophenyl)-penta-2,4-dienal, 6-(4-Dimethylaminophenyl)-hexa-3,5-dien-2-on, 6-(4-Diethylaminophenyl)-hexa-3,5-dien-2-on, 6-(4-Methoxyphenyl)-hexa-3,5-dien-2-on, 6-(3,4-Dimethoxyphenyl)-hexa-3,5-dien-2-on, 6-(2,4-Dimethoxyphenyl)-hexa-3,5-dien-2-on, 6-(4-Piperidinophenyl)-hexa-3,5-dien-2-on, 6-(4-Morpholinophenyl)-hexa-3,5-dien-2-on, 6-(4-Pyrrolidinophenyl)-hexa-3,5-dien-2-on, 5-(4-Dimethylaminonaphth-1-yl)-penta-2,4-dienal,
- 2-Nitropiperonal, 5-Nitropiperonal, 6-Nitropiperonal, 5-Hydroxy-2-nitropiperonal, 2-Hydroxy-5-nitropiperonal, 2-Chlor-6-nitropiperonal, 5-Chlor-2-nitropiperonal, 2,6-Dinitropiperonal,
- 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
- 4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4-Acetylphenyl)-trimethylammonium-, 4-(N,N-Diethyl)-N-methylammonio)-benzaldehyd-, N-(4-Benzoylphenyl)-trimethylammonium-, N-(4-Benzoylphenyl)-N,N-diethylmethylammonium-, N-(4-Formylphenyl)-N-methylpyrrolidinium-, N-(4-Formylphenyl)-N-methylpiperidinium-, N-(4-Formylphenyl)-N-methylmorpholinium-, N-(4-Acetylphenyl)-N-methylmorpholinium-, N-(4-Benzoylphenyl)-N-methylmorpholinium-, 3-Formyl-N-ethyl-N-methylcarbazolium-, 3-Formyl-9,9-dimethylcarbazolium-, 1-(4-Acetylphenyl)-3-methylimidazolium-, 1-(4-Acetylphenyl)-3-methyl-2-imidazolinium-, 1-(4-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, Iodide, Tetrachlorozinkate, Methylsulfate, Trifluormethansulfonate, Hexafluorophosphate, Tetrafluoroborate,
- 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2-Formylvinyl)-1-methylpyridinium-, 1,3-Dimethyl-2-(4-formylphenyl)-benzimidazolinium-, 1,3-Dimethyl-2-(4-formylphenyl)-imidazolinium-, 2-(4-Formylphenyl)-3-methylbenzothiazolium-, 2-(4-Acetylphenyl)-3-methylbenzothiazolium-, 2-(4-Formylphenyl)-3-methylbenzoxazolium-, 2-(5-Formyl-2-furyl)-3-methylbenzothiazolium-, 2-(5-Formyl-2-thienyl)-3-methylbenzothiazolium-, 2-(3-Formylphenyl)-3-methylbenzothiazolium-, 2-(4-Formylnaphth-1-yl)-3-methylbenzothiazolium-, 5-Chlor-2-(4-formylphenyl)-3-methylbenzothiazolium-, 2-(4-Formylphenyl)-3,5-dimethylbenzothiazolium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium, 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-formylphenyl)-ethenyl]-chinolinium, 1-Methyl-2-[2-(4-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzothiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-, 1-Methyl-5-oxo-indeno[1,2-b]pyridinium(4-methyl-4-azonio-9-fluorenon-), 1-Ethyl-5-oxo-indeno[1,2-b]pyridinium(4-Ethyl-4-azonio-9-fluorenon-), 1-Benzyl-5-oxo-indeno[1,2-b]pyridinium(-4-benzyl-4-azonio-9-fluorenon-), 2-Methyl-5-oxo-indeno[1,2-c]pyridinium-, 2-Methyl-9-oxo-indeno[2,1-c]pyridinium-, 1-Methyl-9-oxo-indeno[2, 1-b]pyridinium-salze, insbesondere Benzolsulfonat, p-Toluolsulfonat, Methansulfonat, Perchlorat, Sulfat, Chlorid, Bromid, Iodid, Tetrachlorozinkat, Methylsulfat, Trifluormethansulfonat, Tetrafluoroborat,
- Salicylaldehyd, Vanillin, 4-Hydroxy-3-methoxyzimtaldehyd (Coniferylaldehyd), 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylamino-zimtaldehyd, 4-(Dibutylamino)-benzaldehyd, 4-Diphenylaminobenzaldehyd, 2,3,6,7-Tetrahydro-1 H,5H-benzo[ij]chinolizin-9-carboxaldehyd, 4-Dimethylamino-2-methoxybenzaldehyd, 2,3,6,7-Tetrahydro-8-hydroxy-1 H,SH-benzo[ij]chinolizin-9-carboxaldehyd, 4-(1-Imidazolyl)-benzaldehyd, 2-Morpholinobenzaldehyd, Indol-3-carboxaldehyd, 1-Methylindol-3-carboxaldehyd, N-Ethylcarbazol-3-carboxaldehyd, 2-Formylmethylen-1,3,3-trimethylindolin (Tribasen Aldehyd)
- 1,3-Diacetylbenzol, 1,4-Diacetylbenzol, 1,3,5-Triacetylbenzol, 2-Benzoyl-acetophenon, 2-(4-Methoxybenzoyl)-acetophenon, 2-(2-Furoyl)-acetophenon, 2-(2-Pyridoyl)-acetophenon, 2-(3-Pyridoyl)-acetophenon,
- 1-Phenyl-1,2-propandion, 1-Phenyl-1,2-butandion, 1-Phenyl-3,3-dimethyl-1,2-butandion, Benzil, Anisil, Salicil, 5,5'-Dibromsalicil, 2,2'-Furil, 2,2'-Thienil, 2,2'-, 4,4'-Pyridil, 6,6'-Dimethyl-4,4'-pyridil, 4-Hydroxy-, 4-Methoxy-, 4-Chlor-, 4-Methyl-, 4-Dimethylamino-, 4,4'-Dihydroxy-, -Dimethyl-, -Dibrom-, -Dichlor-, -Bisdimethylamino-, 2,4-Dihydroxy-, 3,3'-Dimethoxy, 2'-Chlor-3,4-dimethoxy-, 3,4,5,3',4',5'-Hexamethoxybenzil,
- Isatinderivate, wie 5-Chlorisatin, 5-Methoxyisatin, 5-Nitroisatin, 6-Nitroisatin, 5-Sulfoisatin, lsatin-5-sulfonsäure, Isatin-4-carbonsäure und Isatin-5-carbonsäure,
- N-substituierte Isatin-Derivate, wie N-Methylisatin, N-(2-Hydroxyalkyl)-isatin, N-(2-Hydroxypropyl)-isatin, N-(3-Hydroxypropyl)-isatin, N-(2,3-Dihydroxypropyl)-isatin, N-(2-Sulfoethyl)-isatin, (3-Sulfopropyl)-isatin, N-Allylisatin, N-Vinylisatin, N-Benzylisatin, N-(4-Methoxybenzyl)-isatin, N-(4-Carboxybenzyl)-isatin, N-(4-Sulfobenzyl)-isatin, N-(2-Dimethylaminoethyl)-isatin, N-(2-Pyrrolidinoethyl)-isatin, N-(2-Piperidinoethyl)-isatin, (2-Morpholinoethyl)-isatin, N-(2-Furylmethyl)-isatin, N-(Thien-2-ylmethyl)-isatin, N-(Pyrid-2-ylmethyl)-isatin, N-(Pyrid-3-ylmethyl)-isatin, N-(Pyrid-4-ylmethyl)-isatin, N-Allylisatin-5-sulfonsäure, 5-Chlor-N-(2-hydroxyethyl)-isatin, 5-Methyl-N-(2-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2-hydroxyethyl)-aminomethyl)-isatin, N-(2-Hydroxyethylaminomethyl)-isatin, N-(Bis-(2-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-Imidazolyl)-methylisatin, N-Carboxymethylaminomethylisatin, N-(2-Carboxyethylaminomethyl)-isatin, N-(3-Carboxypropylaminomethyl)-isatin, N-(Bis-(2-hydroxyethyl)-aminomethyl)-5-methylisatin, N-Piperidinomethyl-5-chlorisatin, N-(2-Sulfoethylamino)-isatin, sowie die Alkali- und gegebenenfalls Ammoniumsalze der sauren Verbindungen, Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxy-acetophenon, 4-Amino-acetophenon, 4-Dimethylamino-acetophenon, 4-Morpholino-acetophenon, 4-Piperidinoacetophenon, 4-Imidazolino-acetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, Indanon, 9-Fluorenon, 3-Hydroxyfluorenon, Anthron, 1,8-Dihydroxyanthron,
- heterocyclische Carbonylverbindungen, wie 2-Indolaldehyd, 3-Indolaldehyd, 1-Methylindol-3-aldehyd,- 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, Imidazol-2-aldehyd,
- Indanon-Derivate, wie z. B. 1,2-Indandion, 2-Oximo-1-indanon, lndan-1,2,3-trion-2-oxim, 5-Methoxy-indan-1,2,3-trion-2-oxim, 2-Nitro-1,3-indandion
sowie physiologisch verträgliche Salze der voranstehenden Verbindungen.

Die primären und sekundären aromatischen Amine der Komponente C sind bevorzugt ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel III dargestellt sind 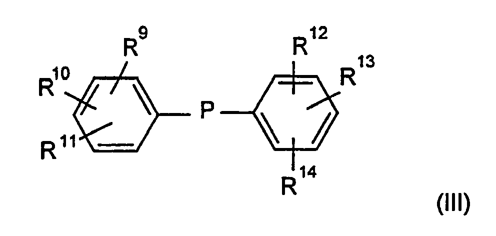 in der
- R⁹ für eine Hydroxy- oder eine Aminogruppe, die durch C₁₋₄-Alkyl-, C₁₋₄-Hydroxyalkyl, C₁₋₄-Alkoxy- oder C₁₋₄-Alkoxy-C₁₋₄-alkylgruppen substituiert sein kann, steht,
- R¹⁰, R¹¹, R¹², R¹³ und R¹⁴ unabhängig voneinander für ein Wasserstoffatom, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel IV

-Q'-(CH₂-Q-CH₂-Q")ₒ- (IV)

in der
- Q eine direkte Bindung, eine CH₂- oder CHOH-Gruppe bedeutet,
- Q' und Q" unabhängig voneinander für ein Sauerstoffatom, eine NR¹⁵-Gruppe, worin R¹⁵ ein Wasserstoffatom, eine C₁₋₄-Alkyl- oder eine Hydroxy-C₁₋₄-alkylgruppe, wobei auch beide Gruppen zusammen mit dem Restmolekül einen 5, 6- oder 7-Ring bilden können, bedeutet, die Gruppe O-(CH₂)ₚ-NH oder NH-(CH₂)ₚ'-O, worin p und p' 2 oder 3 sind, stehen und
- o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die stickstoffhaltigen heterocyclischen Verbindungen der Komponente C sind bevorzugt ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-aminopyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Die aromatischen Hydroxyverbindungen der Komponente C sind bevorzugt ausgewählt aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, - acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure und 3,6-Dihydroxy-2,7-naphthalinsulfonsäure.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Prolin, Lysin und Tryptophan. Aber auch andere Aminosäuren, wie z.B. 6-Aminocapronsäure und β-Alanin, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Die Verbindungen der Komponente C werden besonders bevorzugt ausgewählt aus 4-Formyl-1-ethylpyridinium-p-toluolsulfonat, 4-Formyl-1-methylchinolinium-p-toluoisulfonat, 2-Formyl-1-methylchinolinium-p-toluolsulfonat, Salicylaldehyd, Vanillin, 4-Hydroxy-3-methoxy-zimtaldehyd, 4-Dimethylaminobenzaldehyd, 4-Dimethylamino-2-hydroxybenzaldehyd, 4-Hydroxynaphthaldehyd, Indol-3-carboxaldehyd und Isatin, sowie jeweils aus den vorzugsweise mit anorganischen Säuren gebildeten physiologisch verträglichen Salzen dieser Verbindungen.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Die Verbindungen mit der Formel I bzw. II sowie die Verbindungen der Komponenten B bzw. C werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

In allen Färbemitteln können auch mehrere verschiedene Verbindungen mit der Formel 1 bzw. Formel II und verschiedene CH-acide Verbindungen gemeinsam zum Einsatz kommen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkail- und Erdalkalimetalle oder aus lodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze oder Metalloxide, die einen leichten Wechsel zwischen zwei Oxidationsstufen der Metallionen ermöglichen. Beispiele sind Salze oder Oxide von Eisen, Ruthenium, Mangan und Kupfer.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, Disperse Violet 1, Disperse Violet 4, Disperse Black 9, Basic Brown 16 und Basic Brown 17 bekannten Verbindungen sowie 6-Nitro-1,2,3,4-tetrahydrochinoxalin, 4-N-Ethyl-1,4-bis-(2-hydroxyethylamino)-2-nitrobenzolhydrochlorid und 1-Methyl-3-nitro-4-(2-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die Oxidationsfarbstoffvorprodukte oder die fakultativ enthaltenen direktziehenden Farbstoffe jeweils einheitliche Verbindungen darstellen.

Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, lsostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.
   Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.
   Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller. Th. Goldschmidt; diquatemäre Polydimethylsiloxane, Quatemium-80).
   Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.
   Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.
   Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".
   Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.
   Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.
   Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/lsobomylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalko= hol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.% und Verdickungsmittel in Konzentrationen von 0,1 bis 25 Gew.% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung einer Kombination aus
**A.** mindestens einer quaternierten heterozyklischen Verbindung gemäß Formel I, 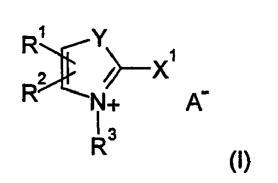 worin R¹, R², R³, X¹ und A wie oben definiert sind,
   mit der Maßgabe, daß wenn Y eine gegebenenfalls substituierte Vinylengruppe ist, die Verbindungen der Formel II 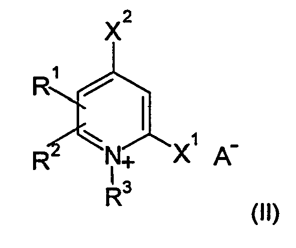 wobei R¹, R², R³, X¹, X² und A⁻ wie oben definiert sind,
   mitumfaßt sind,
   sowie entsprechenden inneren Salzen, wobei A⁻ entfällt, und
**B.** mindestens einer CH-aciden Verbindung gemäß Anspruch 1,
   sowie gegebenenfalls
**C.** mindestens einer Verbindung ausgewählt aus der Gruppe, die gebildet wird aus (a) aromatischen oder heteroaromatischen Aldehyden oder Ketonen, (b) Aminosäuren, (c) aus 2 bis 9 Aminosäuren aufgebauten Oligopeptiden und (d) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen
als färbende Komponenten in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel gemäß den Ansprüchen 1 bis 18 auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Kombination der Verbindungen gemäß Formel I bzw. 11 und die Verbindungen der Komponente B bzw. C können entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I bzw. II oder den Verbindungen der Komponente B zugesetzt werden. Zwischen dem Auftragen der einzelnen Komponenten in einem mehrstufigen Verfahren können bis zu 30 Minuten Zeitabstand liegen. Auch eine Vorbehandlung der Fasern mit der Salzlösung ist möglich.

In einer Variante dieser Ausführungsform des erfindungsgemäßen Verfahrens werden die Verbindungen gemäß Formel I bzw. II und die Verbindungen der Komponente B zuerst auf das Haar aufgetragen. Nach einem Zeitraum von bis zu 30 Minuten wird anschließend die Komponente C auf das Haar aufgebracht.

Die Verbindungen mit der Formel I bzw. II, die Verbindungen mit der Komponente B und die Verbindungen der Komponente C können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

### Beispiele

### 1. Herstellung der kationischen heterozyklischen Verbindungen (erfindungsgemäß)

4-Chlor-1-ethylchinolinium-tetrafluoroborat und 2-Chlor-1-ethylchinoliniumtetrafluoroborat werden gemäß Liebigs Ann. Chem., 1967, 708, 158-209,
3-Ethyl-2-methylmercapto-benzothiazolium-tetrafluoroborat wird gemäß *Liebigs* Ann. Chem., 1971, 752, 182-195 und
3-Ethyl-benzothiazolium-2-sulfonat wird gemäß Chem. Ber., 1968, 101, 1137-1139 und der Patentschrift CH 473121 synthetisiert.
4,7-Dichlor-1-ethylchinolinium-tetrafluoroborat wird gemäß Liebigs Ann. Chem., 1967, 708, 198-209 synthetisiert.
1-Ethylchinolinium-4-sulfonat wird gemäß GB-A-1102891 synthetisiert.

### 2. Herstellung einer Färbelösung

Es wurde eine Aufschlämmung bzw. Lösung der Komponente B (3 mmol) und 0,41 g Natriumacetat in 30 ml Wasser hergestellt. Unmittelbar vor der Anwendung auf dem Haar werden 3 mmol der Verbindung gemäß Formel I bzw. II zugemischt und der pH-Wert des resultierenden Färbemittels mit 10 %-iger wäßriger NaOH oder Salzsäure auf den pH-Wert gemäß Tabelle 1 eingestellt.

In diese Färbemischung wurde bei 30°C 30 Minuten lang eine Strähne Menschenhaar (naturweiß, Firma Kerling) eingebracht. Die Strähne wurde dann 30 Sek. mit lauwarmem Wasser gespült, mit warmer Luft (30°C bis 40°C) getrocknet und anschließend ausgekämmt.

Die jeweiligen Farbnuancen und Fluoreszenzstärke im UV-Licht sind in der nachfolgenden Tabelle 1 wiedergegeben. Die Stärke der Fluoreszenz wird mit folgender Skala bewertet:
(+) stark
(o) mittel
(-) schwach

**Tabelle 1:Färbebeispiele**

| **kationische heterozyklische Verbindung gemäß l bzw. II** | **Komponente B** | **pH** | **Farbe** | **Fluoreszenz (stärke)** |
|---|---|---|---|---|
| 4-Chlor-1-ethylchinolinium-tetrafluoroborat | 1,2,3,3-Tetramethyl-indolium-iodid | 9 | rot | (-) |
| 4-Chlor-1-ethylchinolinium-tetrafluoroborat | 2-Amino-4-imino-thiazolin-hydrochlorid | 9 | rot-violett | (-) |
| 4-Chlor-1-ethylchinolinium-tetrafluoroborat | 1-Ethyl-4-methyl-chinolinium-iodid | 9 | türkisblau | (-) |
| 4-Chlor-1-ethylchinolinium-tetrafluoroborat | 1,3-Diethylthiobarbitursäure | 9 | orange | (-) |
| 4-Chlor-1-ethylchinolinium-tetrafluoroborat | Benzoylacetonitril | 9 | gelb-orange | (-) |
| 3-Ethyl-2-methylmercaptobenzothiazolium-tetrafluoroborat | 1,2,3,3-Tetramethyl-indolium-iodid | 9 | gelb | gelb (o) |
| 3-Ethyl-2-methylmercaptobenzothiazolium-tetrafluoroborat | 3-Ethyl-2-methyl-benzothiazolium-iodid | 9 | hellgelb | hellgelb (+) |
| 3-Ethyl-2-methylmercaptobenzothiazolium-tetrafluoroborat | 1,3-Indandion | 9 | voilett-braun | (-) |
| 3-Ethyl-2-methylmercaptobenzothiazolium-tetrafluoroborat | 2-Amino-4-imino-thiazolin-hydrochlorid | 9 | orange | orange (+) |
| 3-Ethyl-2-methylmercaptobenzothiazolium-tetrafluoroborat | 1-Ethyl-4-methyl-chinolinium-iodid | 9 | orangerot | (-) |
| 3-Ethyl-benzothiazolium-2-sulfonat | 3-Ethyl-2-methyl-benzothiazolium-iodid | 6 | intensiv gelb | grüngelb (+) |
| 3-Ethyl-benzothiazolium-2-sulfonat | 3-Ethyl-2-methylbenzoxazolium-iodid | 6 | hellgelb | blau (+) |
| 3-Ethyl-benzothiazolium-2-sulfonat | 1,2,3,3-Tetramethyl-indolium-iodid | 6 | gelb | gelbgrün (+) |
| 3-Ethyl-benzothiazolium-2-sulfonat | 1,3-Indandion | 6 | orange | orange (+) |
| 2-Chlor-1-ethyl-chinolinium-tetrafluoroborat | 1-Ethyl-4-methyl-chinolinium-iodid | 6 | rot | (-) |
| 2-Chlor-1-ethyl-chinolinium-tetrafluoroborat | 1,2,3,3,-Tetramethyl-indolium-iodid | 6 | violett | (-) |
| 4,7-Dichlor-1-ethyl-chinolinium-tetrafluoroborat | 1,2,3,3-Tetramethyl-indolium-iodid | 9 | intensiv rotviolett | (-) |
| 4,7-Dichlor-1-ethyl-chinolinium-tetrafluoroborat | 1,3-Indandion | 9 | grünbraun | (-) |
| 4,7-Dichlor-1-ethyl-chinolinium-tetrafluoroborat | 2-Amino-4-imino-thiazolin-hydrochlorid | 9 | intensiv violett | (-) |
| 4,7-Dichlor 1-ethyl-chinolinium-tetrafluoroborat | 3-Ethyl-2-methyl-benzothiazolium-iodid | 9 | rot | (-) |
| 4,7-Dichlor-1-ethyl-chinolinium-tetrafluoroborat | 1-Ethyl-2-methyl-chinolinium-iodid | 9 | violett | (-) |
| 4,7-Dichlor 1-ethyl-chinolinium-tetrafluoroborat | 3-Ethyl-2-methyl-benzoxazolium-iodid | 9 | gelb | (-) |
| 4,7-Dichlor-1-ethyl-chinolinium-tetrafluoroborat | 1-Ethyl-4-methyl-chinolinium-iodid | 9 | türkisblau | (-) |
| 4,7-Dichlor-1 -ethyl-chinolinium-tetrafluoroborat | 1,3-Diethylthiobarbitursäure | 9 | intensiv orange | (-) |
| 4,7-Dichlor-1-ethyl-chinolinium-tetrafluoroborat | Benzoylacetonitril | 9 | intensiv gelb | (-) |
| 1-Ethylchinolinium-4-sulfonat | 1,2,3,3-Tetramethyl-indolium-iodid | 6 | intensiv rot | (-) |
| 1-Ethylchinolinium-4-sulfonat | 1,3-Indandion | 6 | rotbraun | (-) |
| 1-Ethylchinolinium-4-sulfonat | 2-Amino-4-imino-thiazolin-hydrochlorid | 6 | schwarz | (-) |
| 1-Ethylchinolinium-4-sulfonat | 3-Ethyl-2-methyl-benzothiazolium-iodid | 6 | intensiv rot | (-) |
| 1-Ethylchinolinium-4-sulfonat | 3-Ethyl-2-methyl-benzoxazolium-iodid | 6 | hellgelb | (-) |
| 1-Ethylchinolinium-4-sulfonat | 1-Ethyl-4-methyl-chinolinium-iodid | 6 | dunkelblau | (-) |
| 1-Ethylchinolinium-4-sulfonat | 1,3-Diethylthiobarbitursäure | 6 | gelb | (-) |
| 1-Ethylchinolinium-4-sulfonat | Benzoylacetonitril | 6 | intensiv gelborange | (-) |

## Patentansprüche

1. Mittel zum Färben von keratinhaltigen Fasern, insbesondere menschlichen Haaren, enthaltend
**A.** mindestens eine quatemierte heterozyklische Verbindung gemäß Formel 1, 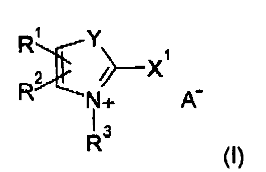 worin
• R¹ und R² stehen unabhängig voneinander für ein Wasserstoffatom, ein Halogenatom, eine Hydroxygruppe, eine C₁-C₄-Hydroxyalkylgruppe, eine C₁-C₆-Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine Sulfonsäuregruppe, eine Carboxylgruppe, eine Nitrogruppe, eine Cyanogruppe oder eine Gruppe -NR⁴R⁵, wobei R⁴ und R⁵ stehen unabhängig voneinander für ein Wasserstoffatom, eine C₁-C₆-Alkylgruppe, eine C₂-Cₐ-Alkenylgruppe, eine Aryl-C₁-C₄-alkylgruppe oder eine C₁-C₄-Hydroxyalkylgruppe, wobei R¹ und R² zusammen einen ankondensierten 5- oder 6-gliedrigen, aliphatischen oder aromatischen Ring bilden können, welcher wiederum mit den Resten R⁶ und R⁷ substituiert ist, wobei R⁶ und R⁷ stehen unabhängig voneinander für die Reste, die unter R¹ definiert sind,
• R³ steht für eine C₁-C₄-Hydroxyalkylgruppe, eine C₁-C₆-Aminoalkylgruppe, eine C₁-C₄-Dialkylamino-C₁-C₄-alkylgruppe, eine Di(C₁-C₄-hydroxyalkyl)amino-C₁-C₄-alkylgruppe, eine lineare oder verzweigte C₁-C₆-Alkylgruppe, eine C₂-C₆-Alkenylgruppe, eine gegebenenfalls substituierte Arylgruppe,
• A⁻ steht für ein Chlorid, Bromid, lodid, Hexafluorophosphat, Tetrachlorozinkat, Tetrafluoroborat, Trifluormethylsulfonat, Methylsulfonat oder p-Toluolsulfonat,
• Y steht für ein Sauerstoffatom, ein Schwefelatom, eine Gruppe -N=CH-, eine gegebenenfalls substituierte Methylen- oder eine gegebenenfalls substituierte Vinylengruppe oder eine Gruppe NR⁸, wobei R⁸ für die gleichen Gruppen stehen kann, die unter R⁵ definiert sind,
• X¹ steht für ein Halogenatom, eine C₁-C₄-Alkoxygruppe, eine C₁-C₄-Alkylmercaptogruppe, eine Sulfonsäuregruppe oder eine p-Toluolsulfonylgruppe,
mit der Maßgabe, daß wenn Y eine gegebenenfalls substituierte Vinylengruppe ist, die Verbindungen der Formel 11 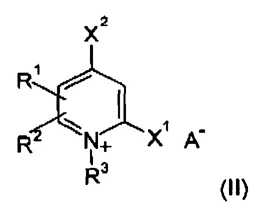 wobei
• R¹ R², R³ und A⁻ wie oben definiert sind,
• einer der Reste X¹ oder X² steht für ein Halogenatom, eine C₁-C₄-Alkoxygruppe, eine C₁-C₄-Alkylmercaptogruppe, eine Sulfonsäuregruppe oder eine p-Toluolsulfonylgruppe und der andere für ein Wasserstoffatom oder die Gruppen, die unter R¹ und R² definiert sind,
mitumfaßt sind,
sowie entsprechenden inneren Salzen, wobei A⁻ entfällt, und
**B.** mindestens eine CH-acide Verbindung, wobei die CH-acide Verbindung ausgewählt wird aus Verbindungen gemäß einer der Formeln C1 bis C21
M¹-CH₂-M² (C1)
worin
• M¹ eine Gruppe -COM³, COOM³, S(O)M³ oder SO₂M³ ist, worin M³ für ein Wasserstoffatom oder eine C₁-C₆-Alkylgruppe steht, oder eine Gruppe - C(M⁴)=C(C≡N)₂ bedeutet, worin M⁴ für ein Wasserstoffatom, eine C₁-C₄-Alkylgruppe oder eine Arylgruppe steht,
• M² die gleiche Bedeutung wie M¹ hat oder eine Cyanogruppe, eine substituierte oder unsubstituierte Arylgruppe oder Aryl-C₁-C₄-alkylgruppe, ein substituierter oder unsubstituierter, gesättigter oder ungesättigter Heterozyklus ist,
Verbindungen mit der Formel C2 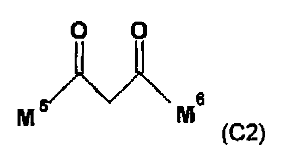 worin
• M⁶ eine Cyanogruppe, eine substituierte oder unsubstituierte Arylgruppe oder eine Aryl-C₁-C₄-alkylgruppe, ein substituierter oder unsubstituierter, gesättigter oder ungesättigter Heterozyklus oder eine Gruppe -COM⁷ oder COOM⁷ ist, worin M⁷ für ein Wasserstoffatom oder eine C₁-C₆-Alkylgruppe steht,
• M⁶ eine substituierte oder unsubstituierte C₁-C₆-Alkylgruppe, eine Acetyloxygruppe, eine C₃-C₆-Cycloalkylgruppe, eine substituierte oder unsubstituierte Arylgruppe oder Aryl-C₁-C₄-alkylgruppe, eine substituierte oder unsubstituierte Aminoarylgruppe, ein substituierter oder unsubstituierter, gesättigter oder ungesättigter Heterozyklus ist,
Verbindungen mit der Formel C3 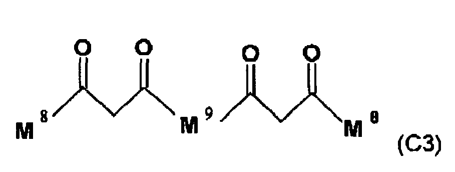 worin
• M⁸ eine Cyanogruppe, eine substituierte oder unsubstituierte Arylgruppe oder Aryl-C₁-C₄-alkylgruppe, ein substituierter oder unsubstituierter, gesättigter oder ungesättigter Heterozyklus oder eine Gruppe -COM¹⁰ oder COOM¹⁰ ist, worin M¹⁰ für ein Wasserstoffatom oder eine C₁-C₆-Alkylgruppe steht,
• M⁹ eine substituierte oder unsubstituierte Arylgruppe oder Aryl-C₁-C₄-alkylgruppe, eine substituierte oder unsubstituierte Aminoarylgruppe, ein substituierter oder unsubstituierter, gesättigter oder ungesättigter Heterozyklus ist,
Pyrazolderivate (a), die ausgewählt sein können aus den folgenden Formeln C4 und C5 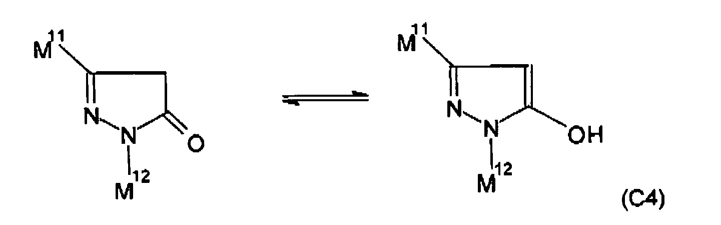 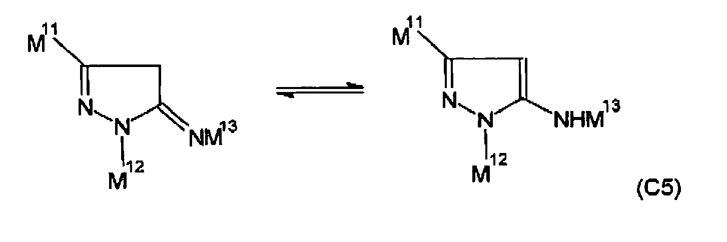 worin
• M¹¹ und M¹² unabhängig voneinander für eine substituierte oder unsubstituierte C₁-C₆-Alkylgruppe, eine Acetyloxygruppe, eine C₃-C₆-Cycloalkylgruppe, eine substituierte oder unsubstituierte Arylgruppe oder Aryl-C₁-C₄-alkylgruppe, eine substituierte oder unsubstituierte Aminoarylgruppe, einen substituierter oder unsubstituierter, gesättigten oder ungesättigten Heterozyklus stehen,
• M¹³ ein Wasserstoffatom oder eine substituierte oder unsubstituierte C₁-C₆-Alkylgruppe ist,
(b) zwei über M¹¹ oder M¹² gebundene Pyrazolringe mit der Formel C4 oder C5 Barbitursäurederivate mit der folgenden Formel C6 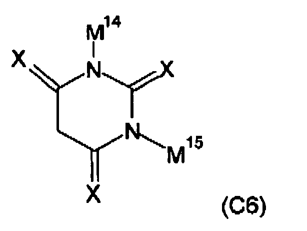
worin
• M¹⁴ und M¹⁵ stehen unabhängig voneinander für eine C₁-C₆-Alkylgruppe, eine C₂-C₆-AlKenylgruppe, eine C₃-C₆-Cycloalkylgruppe, eine Aryl-C₁-C₄-alkylgruppe, eine substituierte oder unsubstituierte Arylgruppe, oder einen über die Reste M¹⁴ oder M¹⁵ gebundenen Bicyclus,
• X steht für ein Sauerstoff- oder ein Schwefelatom,
Pyridinderivate mit der Formeln C7a und C7b 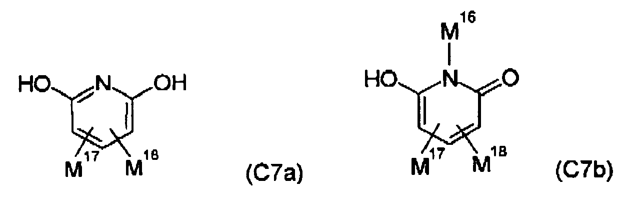 worin
• M¹⁶ eine substituierte oder unsubstituierte C₁-C₆-Alkylgruppe oder substituierte oder unsubstituierte Arylgruppe ist,
• M¹⁷ ein Wasserstoffatom, eine substituierte oder unsubstituierte C₁-C₆-Alkylgruppe oder eine substituierte oder unsubstituierte Arylgruppe ist,
• M¹⁸ ein Wasserstoffatom, eine Cyanogruppe, eine substituierte oder unsubstituierte C₁-C₆-Alkylgruppe, eine Gruppe COOM¹⁹. worin M¹⁹ ein Wasserstoffatom oder eine substituierte oder unsubstituierte C₁-C₆-Alkylgruppe bedeutet, ist,
Verbindungen mit der folgenden Formel C8 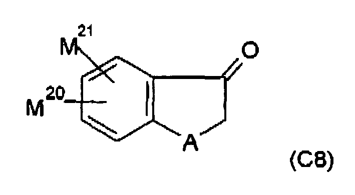 worin
• A steht für ein Sauerstoffatom, ein Schwefelatom, eine Sulfoxylgruppe, eine Sulfonylgruppe oder eine Gruppe NM^{20a}, worin M^{20a} ein Wasserstoffatom, eine substituierte oder unsubstituierte C₁-C₆-Alkylengruppe bedeutet,
• M²⁰ und M²¹ stehen unabhängig voneinander für ein Wasserstoff-, ein Chlor-, ein Bromatom, eine Hydroxygruppe, eine Nitrogruppe, eine C₁-C₆-Alkylgruppe, eine C₁-C₆-Alkoxy-, eine Carboxamid-, eine Sulfonamid-, eine Carboxyl-, eine C₁-C₄-Acyl, eine Cyanogruppe oder eine Aminogruppe - NM²²M²³, in der M²² und M²³ unabhängig voneinander stehen für ein Wasserstoffatom oder eine C₁-C₆-Alkylgruppe,
Verbindungen mit den Formeln C9 und C10 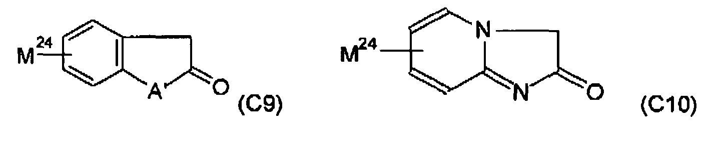
worin
• A' steht für ein Sauerstoffatom, ein Schwefelatom oder eine Gruppe NM²⁵, worin M²⁵ ein Wasserstoffatom oder eine substituierte oder unsubstituierte C₁-C₆-Alkylgruppe bedeutet,
• M²⁴ steht für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM²⁶M²⁷, in der M²⁶ und M²⁷ unabhängig voneinander stehen für Wasserstoff und eine C₁-C₆-Alkylgruppe
Verbindungen mit der Formel C11 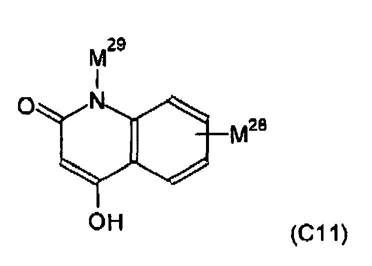 worin
• M²⁸ ein Wasserstoffatom, eine Hydroxygruppe, eine substituierte oder unsubstituierte C₁-C₆-Alkylgruppe oder eine substituierte oder unsubstituierte Aryl- oder C₁-C₆-Alkylarylgruppe ist;
• M²⁹ steht für ein Wasserstoffatom oder eine C₁-C₄-Alkylgruppe
Indandionderivate mit der Formel C12 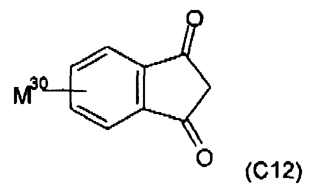 worin
• M³⁰ ein Wasserstoff-, ein Chlor-, ein Bromatom, eine Nitro-, eine C₁-C₆-Alkyl, eine C₁-C₆-Alkoxy-, eine Carboxamid-, eine Sulfonamid- oder eine Cyanogruppe ist,
Verbindungen mit der Formel C13 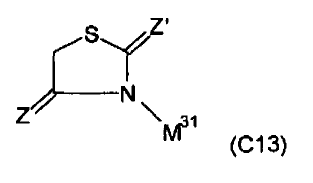 worin
• Z steht für ein Sauerstoffatom oder eine Gruppe NM³², worin M³² ein Wasserstoffatom oder eine C₁-C₆-Alkylgruppe bedeutet,
• Z' steht für ein Schwefelatom oder eine Gruppe NM³³, worin M³³ ein Wasserstoffatom oder eine C₁-C₆-Alkylgruppe bedeutet,
• M³¹ steht für ein Wasserstoffatom, eine C₁-C₆-Alkylgruppe oder eine C₁-C₄-Carboxyalkylgruppe,
Dioxopyrazolverbindungen mit der Formel C14 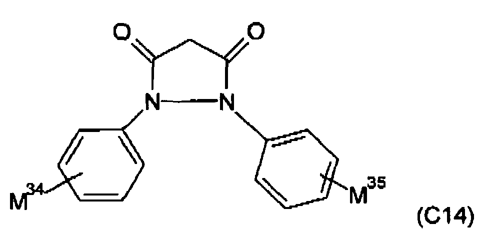 worin
• M³⁴und M³⁵ stehen unabhängig voneinander für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM³⁶M³⁷, in der M³⁶ und M³⁷ unabhängig voneinander stehen für Wasserstoff oder eine C₁-C₆-Alkylgruppe,
5-Oxoimidazolderivate mit der Formel C15 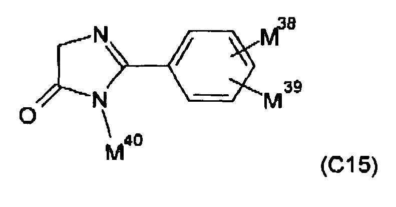 worin
• M³⁸ und M³⁹ stehen unabhängig voneinander für ein Wasserstoff-, ein Chlor-, ein Bromatom, eine Hydroxygruppe, eine Nitrogruppe, eine C₁-C₆-Alkyl-, eine C₁-C₆-Alkoxy-, eine Carboxamid-, eine Sulfonamid-, eine Carboxyl-, eine C₁-C₄-Acyl, eine Cyanogruppe oder eine Aminogruppe -NM⁴¹M⁴². in der M⁴¹ und M⁴² unabhängig voneinander stehen für ein Wasserstoffatom oder eine C₁-C₆-Alkylgruppe,
• M⁴⁰ steht für ein Waserstoffatom oder eine C₁-C₆-Alkylgruppe,
Derivate von Dehydrobutyrolacton mit der Formel C16 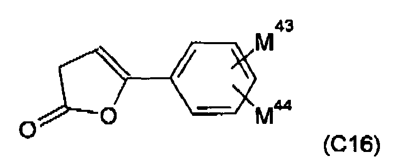
worin
• M⁴³ und M⁴⁴ unabhängig voneinander stehen für ein Wasserstoff-, ein Chlor-, ein Bromatom, eine Hydroxygruppe, eine Nitrogruppe, eine C₁-C₆-Alkyl-, eine C₁-C₈-Alkoxy-, eine Carboxamid-, eine Sulfonamid-, eine Carboxyl-, eine C₁-C₄-Acyl, eine Cyanogruppe oder eine Aminogruppe -NM⁴⁵M⁴⁸, in der M⁴⁵ und M⁴⁶ unabhängig voneinander stehen für ein Wasserstoffatom oder eine C₁-C₆-Alkylgruppe
Verbindungen mit der Formel C17 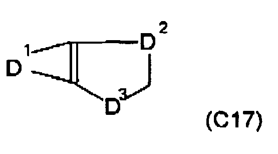
worin
• D¹ ist ein ankondensierter aromatischer oder heteroaromatischer Ring,
• D² ist eine Carbonylgruppe, eine Gruppe C=CD'D" oder eine Gruppe CD'D", in welchen D^{I} oder D^{II} jeweils ein Substituent mit einer Hammett-Konstante zwischen 0,4 und 2,0 oder beide Substituenten in der Summe eine Hammett-Konstante zwischen 0,4 und 2,0 aufweisen;
• D³ steht für eine Carbonylgruppe, ein Sauerstoff-, ein Schwefelatom, eine Gruppe NM⁴⁷, wenn D² nicht Sauerstoff ist, oder eine Gruppe C=S, eine Gruppe C=NR⁴⁸, eine Sulfinylgruppe, eine Sulfonylgruppe, wobei R⁴⁷ und R⁴⁸ unabhängig voneinander ein Wasserstoffatom oder einen C₁-C₄-Alkylrest bedeuten,
Hydroxypyrimidinderivate mit der Formel C18 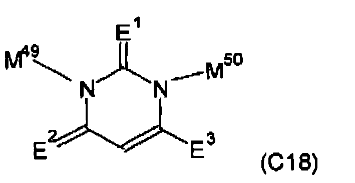
worin
• M⁴⁹ und M⁵⁰ unabhängig voneinander ein Wasserstoffatom oder eine substituierte oder unsubstituierte C₁-C₆-Alkylgruppe sind,
• E¹ für ein Sauerstoff, ein Schwefelatom oder eine Gruppe NH steht,
• E² für eine Gruppe NH oder ein Sauerstoffatom steht,
• E³ für eine Aminogruppe oder eine Hydroxygruppe steht,
mit der Maßgabe, daß
a) wenn E¹ und E² für ein Sauerstoffatom stehen, E³ keine Hydroxygruppe ist, und
b) wenn E¹ ein Schwefelatom und E² ein Sauerstoffatom ist, E³ keine Hydroxygruppe ist,
quatemierte Stickstoffverbindungen der Formel C19 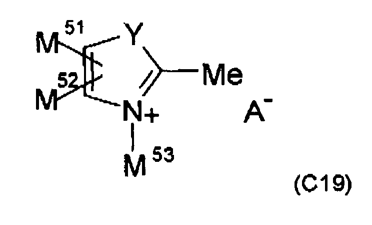 worin,
• M⁵¹ und M⁵² stehen unabhängig voneinander für ein Wasserstoffatom, ein Halogenatom, eine Hydroxygruppe, eine C₁-C₄-Hydroxyalkylgruppe, eine C₁-Cs-Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine Sulfonsäuregruppe, eine Carboxylgruppe, eine Formylgruppe, eine Nitrogruppe, eine Cyanogruppe oder eine Gruppe -NM⁵⁴M⁵⁵, wobei M⁵⁴und M⁵⁵ stehen unabhängig voneinander für ein Wasserstoffatom, eine C₁-C₆-Alkylgruppe, eine C₂-C₆-Alkenylgruppe, eine Aryl-C₁-C₄-alkylgruppe oder eine C₁-C₄-Hydroxyalkylgruppe, wobei M⁵¹ und M⁵² zusammen einen ankondensierten 5- oder 6-gliedrigen, aliphatischen oder aromatischen bzw. heteroaromatischen Ring bilden können, welcher wiederum mit den Resten M⁵⁶ und M⁵⁷ substituiert ist, wobei M⁵⁶ und M⁵⁷ stehen unabhängig voneinander für die Reste, die unter M⁵¹ definiert sind,
• M⁵³ steht für ein Wasserstoffatom, eine C₁-C₄-Hydroxyalkylgruppe, eine C₁-C₆-Aminoalkylgruppe, eine C₁-C₄-Dialkylamino-C₁-C₄-alkylgruppe, eine lineare oder verzweigte C₁-C₆-Alkylgruppe, eine C₂-C₆-Alkenylgruppe, eine gegebenenfalls substituierte Arylgruppe, eine C₁-C₄-Sulfonylalkylgruppe, eine C₁-C₄-Carboxyalkylgruppe oder eine C₂-C₆-Polyhydroxyalkylgruppe,
• Y steht für ein Sauerstoffatom, ein Schwefelatom, eine gegebenenfalls substituierte Methylengruppe oder eine Gruppe NM⁶⁰, wobei M⁶⁰ für die gleichen Gruppen stehen kann, die unter M⁵⁵ definiert sind,
• A⁻ steht für ein Chlorid, Bromid, Iodid, Hexafluorophosphat, Tetrachlorozinkat, Tetrafluoroborat, Trifluormethylsulfonat, Methylsulfonat oder p-Toluolsulfonat,
Oniumverbindungen der Formeln C20 und C21 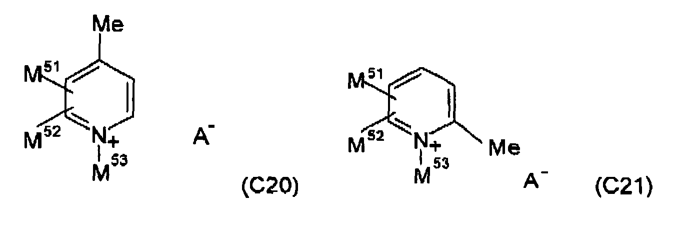
wobei M⁵¹, M⁵², M⁵³ und A⁻ aus den Gruppen ausgewählt werden, die unter Verbindung C19 definiert sind.

2. Mittel gemäß Anspruch 1, **dadurch gekennzeichnet, daß** die Verbindungen mit der Formel I bzw. Formel II ausgewählt sind aus Substanzen, in denen das Kation ausgewählt ist aus 2-Chlor-1-ethyl-chinolinium, 4-Chlor-1-ethyl-chinolinium, 2-Chlor-1-methyl-pyridinium, 4-Chlor-1-methyl-pyridinium, 3-Ethyl-2-methylmercaptobenzothiazolium, 3-Ethyl-2-methylmercapto-benzoxazolium, 2-Chlor-1,3-diethylbenzimidazolium, 2-Chlor-3-ethyl-benzoxazolium, 2-Chlor-3-ethyl-benzothiazolium und das Gegenion A' ausgewählt ist aus Chlorid, Bromid, Iodid, Hexafluorophosphat, Tetrachlorozinkat, Tetrafluoroborat, Trifluormethylsulfonat, Methylsulfonat oder p-Toluolsulfonat und 3-Ethyl-benzothiazolium-2-sulfonat, 1-Ethyl-chinolinium-4-sulfonat und 1-Ethyl-chinolinium-2-sulfonat.

3. Mittel nach einem der Ansprüche 1 oder 2, **dadurch gekennzeichnet, daß** die CH-acide Verbindung ausgewählt ist aus 1,2,3,3-Tetramethyl-3H-indoliumiodid, 1,2,3,3-Tetramethyl-3H-indolium-p-toluolsulfonat, 1,2,3,3-Tetramethyl-3H-indoliummethansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 1-Methyl-3-phenyl-pyrazolin-5-on, Indan-1,2-dion, Indan-1,3-dion, Indan-1-on, Benzoylacetonitril, 3-Dicyanmethylenindan-1-on, 1,3-Diiminoisoindolin, 2-Amino-4-imino-'1,3-thiazolin-hydrochlorid, 5,5-Dimethylcyclohexan-1,3-dion, 2H-1,4-8enzoxazin-4H-3-on, 3-Ethyl-2-methylbenzoxazoliumlodid, 3-Ethyl-2-methyl-benzothiazoliumiodid, 1-Ethyl-4-methylchinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1 -Ethyl-2-methylchinolinium-p-toluolsulfonat, und 1,2,3-Trimethylchinoxalinium-p-toluolsutfonat.

4. Mittel nach einem der Ansprüche 1 bis 3, **dadurch gekennzeichnet, daß** es zusätzlich eine Komponente C, enthaltend mindestens eine Verbindung ausgewählt aus (a) aromatischen oder heteroaromatischen Aldehyden oder Ketonen, (b) Aminosäuren, (c) aus 2 bis 9 Aminosäuren aufgebauten Oligopeptiden und (d) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, enthält.

5. Mittel gemäß Anspruch 4, **dadurch gekennzeichnet, daß** es als Komponente C mindestens eine Verbindung ausgewählt aus aromatischen oder heteroaromatischen Aldehyden oder Ketonen enthält.

6. Mittel nach einem der Anspruch 5, **dadurch gekennzeichnet, daß** die in Komponente C enthaltenen aromatischen und/oder heteroaromatischen Aldehyde und/oder Ketone ausgewählt sind aus
- 5-(4-Dimethylaminophenyl)-penta-2,4-dienal, 5-(4-Diethylaminophenyl)-penta-2,4-dienal, 5-(4-Methoxyphenyl)-penta-2,4-dienal, 5-(3,4-Dimethoxyphenyl)-penta-2,4-dienal, 5-(2,4-Dimethoxyphenyl)-penta-2,4-dienal, 5-(4-Piperidinophenyl)-penta-2,4-dienal, 5-(4-Morpholinophenyl)-penta-2,4-dienal, 5-(4-Pyrrolidinophenyl)-penta-2,4-dienal, 6-(4-Dimethylaminophenyl)-hexa-3,5-dien-2-on, 6-(4-Diethylaminophenyl)-hexa-3,5-dien-2-on, 6-(4-Methoxyphenyl)-hexa-3,5-dien-2-on, 6-(3,4-Dimethoxyphenyl)-hexa-3,5-dien-2-on, 6-(2,4-Dimethoxyphenyl)-hexa-3,5-dien-2-on, 6-(4-Piperidinophenyl)-hexa-3,5-dien-2-on, 6-(4-Morpholinophenyl)-hexa-3,5-dien-2-on, 6-(4-Pyrrolidinophenyl)-hexa-3,5-dien-2-on, 5-(4-Dimethylaminonaphth-1-yl)-penta-2,4-dienal,
- 2-Nitropiperonal, 5-Nitropiperonal, 6-Nitropiperonal, 5-Hydroxy-2-nitropiperonal, 2-Hydroxy-5-nitropiperonal, 2-Chlor-6-nitropiperonal, 5-Chlor-2-nitropiperonal, 2,6-Dinitropiperonal,
- 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
- 4-Trimethylammoniobenzaldehyd-, 4-Benryldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4-Acetylphenyl)-trimethylammonium-, 4-(N,N-Diethyl)-N-methylammonio)-benzaldehyd-, N-(4-Benzoylphenyl)-trimethylammonium-, N-(4-Benzoylphenyl)-N,N-diethylmethylammonium-, N-(4-Formylphenyl)-N-methylpyrrolidinium-, N-(4-Formylphenyl)-N-methylpiperidinium-, N-(4-Formylphenyl)-N-methylmorpholinium-, N-(4-Acetylphenyl)-N-methylmorpholinium-, N-(4-Benzoylphenyl)-N-methylmorpholinium-, 3-Formyl-N-ethyl-N-methylcarbazolium-, 3-Formyl-9,9-dimethylcarbazolium-, 1-(4-Acetylphenyl)-3-methylimidazolium-, 1-(4-Acetylphenyl)-3-methyl-2-imidazolinium-, 1-(4-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, lodide, Tetrachlorozinkate, Methylsulfate, Trifluormethansulfonate, Hexafluorophosphate, Tetrafluoroborate,
- 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-FormyM ,2-dimethytpyridinium-, 4-Formyl-1,3-dimethylpyridinium-, 4-Formyl-1-methylchinolinium-, 2-Formyl-1-methylchinolinium-, 4-Acetyi-1-methylpyridinium-, 2-Acetyl-1-methylpyridinium, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium. 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7 -Acetyl-1-methylchinolinium-, 8-Acetyl-1-methylchinolinium, 5-Acetyl-1-ethylchinolinium-, 6-Acetyl-1-ethylchinolinium-, 7-Acety!-1 -ethytchinotinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2-Formylvinyl)-1-methylpyridinium-, 1,3-Dimethyl-2-(4-formylphenyl)-benzimidazolinium-, 1,3-Dimethyl-2-(4-formylphenyl)-imidazolinium-, 2-(4-Formylphenyl)-3-methylbenzothiazolium-, 2-(4-Acetylphenyl)-3-methylbenzothiazolium-, 2-(4-Formylphenyl)-3-methylbenzoxazolium-, 2-(5-Formyl-2-furyl)-3-methylbenzothiazolium-, 2-(5-Formyl-2-thienyl)-3-methylbenzothiazolium-, 2-(3-Formylphenyl)-3-methylbenzothiazolium-, 2-(4-Formylnaphth-1-yl)-3-methylbenzothiazolium-, 5-Chlor-2-(4-formylphenyl)-3-methylbenzothiazolium-, 2-(4-Formylphenyl)-3,5-dimethylbenzothiazolium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium, 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-formylphenyl)-ethenyl]-chinolinium, 1-Methyl-2-[2-(4-formylphenyl)-ethenyl]-chinolinium-, 1-Methyl-2-(2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzothiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-, 1-Methyl-5-oxo-indeno[1,2-b]pyridinium(4-methyl-4-azonio-8-fluorenon-), 1-Ethyl-5-oxo-indeno[1,2-b]pyridinium(4-Ethyl-4-azonio-9-fluorenon-), 1-Benzyl-5-oxo-indeno[1,2-b]pyridinium(-4-benzyl-4-azonio-9-fluorenon-), 2-Methyl-5-oxo-indeno[1,2-c]pyridinium-, 2-Methyl-9-oxoindeno[2.1-c]pyridinium-, 1-Methyl-9-oxo-indeno[2,1-b]pyridinium-salze, insbesondere Benzolsulfonat, p-Tofuolsulfonat, Methansulfonat, Perchlorat, Sulfat, Chlorid, Bromid, lodid, Tetrachlorozinkat, Methylsulfat, Trifluormethansulfonat, Tetrafluoroborat,
- Salicylaldehyd, Vanillin, 4-Hydroxy-3-methoxyzimtaldehyd (Coniferylaldehyd), 2,4-Dihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylaminozimtaldehyd, 4-(Dibutylamino)-benzaldehyd, 4-Diphenylaminobenzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-Imidazolyl)-benzaldehyd, 2-Morpholinobenzaldehyd, Indol-3-carboxaldehyd, 1-Methylindol-3-carboxaldehyd, N-Ethylcarbazol-3-carboxaldehyd, 2-Formylmethylen-1,3,3-trimethylindolin (Tribasen Aldehyd)
- 1,3-Diacetylbenzol, 1,4-Diacetylbenzol, 1,3,5-Triacetylbenzol, 2-Benzoylacetophenon, 2-(4-Methoxybenzoyl)-acetophenon, 2-(2-Furoyl)-acetophenon, 2-(2-Pyridoyl)-acetophenon, 2-(3-Pyridoyl)-acetophenon,
- 1-Phenyl-1,2-propandion, 1-Phenyl-1,2-butandion, 1-Phenyl-3,3-dimethyl-1,2-butandion, Benzil, Anisil, Salicil, 5,5'-Dibromsalicil, 2,2'-Furil, 2,2'-Thienil, 2,2'-, 4,4'-Pyridil, 6,6'-Dimethyl-4,4'-pyridil, 4-Hydroxy-, 4-Methoxy-, 4-Chlor-, 4-Methyl-, 4-Dimethylamino-, 4,4'-Dihydroxy-, -Dimethyl-, -Dibrom-, -Dichlor-, - Bis-dimethylamino-, 2,4-Dihydroxy-, 3,3'-Dimethoxy, 2'-Chlor-3,4-dimethoxy-, 3,4,5,3',4',5'-Hexamethoxybenzil,
- Isatinderivate, wie 5-Chlorisatin, 5-Methoxyisatin, 5-Nitroisatin, 6-Nitroisatin, 5-Sulfoisatin, Isatin-5-sulfonsäure, Isatin-4-carbonsäure und Isatin-5-carbonsäure,
- N-substituierte Isatin-Derivate, wie N-Methylisatin, N-(2-Hydroxyalkyl)-isatin, N-(2-Hydroxyprapyl)-isatin, N-(3-Hydroxypropyl)-isatin, N-(2,3-Dihydroxypropyl)-isatin, N-(2-Sulfoethyl)-isatin, (3-Sulfopropyl)-isatin, N-Allylisatin, N-Vinylisatin, N-Benzylisatin, N-(4-Methoxybenzyl)-isatin, N-(4-Carboxybenzyl)-isatin, N-(4-Sulfobenzyl)-isatin, N-(2-Dimethylaminoethyl)-isatin, N-(Z-Pyrrolidinoethyl)-isatin, N-(2-Piperidinoethyl)-isatin, (2-Morpholinoethyl)-isatin, N-(2-Furylmethyl)-isatin, N-(Thien-2-ylmethyl)-isatin, N-(Pyrid-2-ylmethyl)-isatin, N-(Pyrid-3-ylmethyl)-isatin, N-(Pyrid-4-ylmethyl)-isatin, N-Allylisatin-5-sulfonsäure, 5-Chlor-N-(2-hydroxyethyl)-isatin, 5-Methyl-N-(2-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2-hydroxyethyl)-aminomethyl)-isatin, N-(2-Hydroxyethylaminomethyl)-isatin, N-(Bis-(2-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-Imidazolyl)-methylisatin, N-Carboxymethylaminomethylisatin, N-(2-Carboxyethylaminomethyl)-isatin, N-(3-Carboxypropylaminomethyl)-isatin, N-(Bis-(2'-hydroxyethyl)-aminomethyl)-5-methylisatin, N-Piperidinomethyl-5-chlorisatin, N-(2-Sulfoethylamino)-isatin, sowie die Alkali- und gegebenenfalls Ammoniumsalze der sauren Verbindungen, Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxyacetophenon, 3,5-Dimethoxy-4-hydroxy-acetophenon, 4-Amino-acetophenon, 4-Dimethylamino-acetophenon, 4-Morpholino-acetophenon, 4-Piperidinoacetophenon, 4-Imidazolino-acetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroacy-2-acetonaphthon, Chromon, Chromon-2-carbonsäure, Flavon, 3-Hydroxyffavon, 3,5,7-Trihydroxyflavon, 4',5,7-Trihydroxyflavon, 5,6,7-Trihydroxyflavon, Quercetin, Indanon, 9-Fluorenon, 3-Hydroxyfluorenon, Anthron, 1,8-Dihydroxyanthron,
- heterocyclische Carbonylverbindungen, wie 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, Imidazol-2-aldehyd,
- Indanon-Derivate, wie z. B. 1,2-Indandion, 2-Oximo-1-indanon, Indan-1,2,3-trion-2-oxim, 5-Methoxy-indan-1,2,3-trion-2-oxim, 2-Nitro-1,3-indandion
sowie physiologisch verträglichen Salzen der voranstehenden Verbindungen.

7. Mittel gemäß einem der Ansprüche 4 bis 6. **dadurch gekennzeichnet, daß** die primären und sekundären aromatischen Amine der Komponente C ausgewählt sind aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methy-benzol, 1-Hydroxy-2-amino-6-methyl-benzol, 2-Amino-5-acetamido-phenol, 1,3-Dimethyl-2,5-diaminobenzol, 5-(3-Hydraxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallal, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[biS-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3'.5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitra-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, 4,4'-Diaminostilben und dessen Hydrochlorid, 4,4'-Diaminostilben-2,2'-disulfonsäure-mono- oder -di-Na-Salz, 4-Amino-4'-dimethylaminostitben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

8. Mittel gemäß einem der Ansprüche 4 bis 7, **dadurch gekennzeichnet, daß** die stickstoffhaltigen heterocyclischen Verbindungen der Komponente C ausgewählt sind aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxypyridin, 2,6-Dimethoxy-3,5-diamino-pyridin. 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Wydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-mvrpholino-anifin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin, 4-Hydroxyindolin und HydroxypyrimidinDerivate und die physiologisch verträglichen Salze der vorgenannten Verbindungen.

9. Mittel gemäß einem der Ansprüche 4 bis 8, **dadurch gekennzeichnet, daß** die aromatischen Hydroxyverbindungen der Komponente C ausgewählt sind aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure und 3,6-Dihydroxy-2,7-naphthalinsulfonsäure

10. Mittel gemäß einem der Ansprüche 4 bis 9, **dadurch gekennzeichnet, daß** die Aminosäuren der Komponente C ausgewählt sind aus Arginin, Histidin, Tyrosin, Phenylalanin, DOPA (Dihydroxyphenylalanin), Ornithin, Prolin, Lysin, Tryptophan, 6-Aminocapronsäure und β-Alanin.

11. Mittel gemäß einem der Ansprüche 4 bis 10, **dadurch gekennzeichnet, daß** die Oligopeptide der Komponente C ausgewählt sind aus Glutathion oder den in den Hydrolysaten von Kollagen, Keratin, Casein, Elastin, Sojaprotein, Weizengluten oder Mandelprotein enthaltenen Oligopeptide.

12. Mittel gemäß einem der Ansprüche 4 bis 11, **dadurch gekennzeichnet, daß** die in Komponente C enthaltenen Verbindungen ausgewählt sind aus 4-Formyl-1-ethylpyridinium-p-tofuolsulfonat, 4-Formyl-1-methylchinolinium-p-toluolsulfonat, 2-Formyl-1-methylchinolinium-p-toluolsulfonat, Salicylaldehyd, Vanillin, 4-Hydroxy-3-methoxy-zimtaldehyd, 4-Dimethylaminobenzaldehyd, 4-Dimethylamino-2-hydroxybenzaldehyd, 4-Hydroxynaphthaldehyd, Indol-3-carboxaldehyd und Isatin, sowie jeweils aus den vorzugsweise mit anorganischen Säuren gebildeten physiologisch verträglichen Salzen dieser Verbindungen.

13. Mittel gemäß einem der Ansprüche 1 bis 12, **dadurch gekennzeichnet, daß** die Verbindungen der Formel I in einer Menge von 0,03 bis 65 mmol, insbesondere von 1 bis 40 mmol, bezogen auf 100 g des gesamten Färbemittels, enthalten sind.

14. Mittel nach einem der Ansprüche 1 bis 13, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

15. Mittel nach einem der Ansprüche 1 bis 14, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

16. Mittel nach einem der Ansprüche 1 bis 15, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

17. Mittel nach einem der Ansprüche 1 bis 16, **dadurch gekennzeichnet, daß** es Oxidationsmittel, insbesondere H₂O₂, in einer Menge von 0,01 bis 6 Gew.-%, bezogen auf die Anwendungslösung, enthält.

18. Mittel nach einem der Ansprüche 1 bis 17, **dadurch gekennzeichnet, daß** es anionische, zwitterionische oder nichtionische Tenside enthält.

19. Verwendung einer Kombination aus
A. mindestens einer quaternierten heterozyklischen Verbindung gemäß Formel 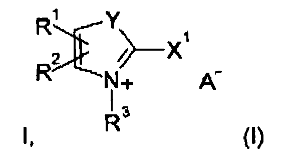 worin R¹, R², R³, X¹ und A⁻wie oben definiert sind,
mit der Maßgabe, daß wenn Y eine gegebenenfalls substituierte Vinylengruppe ist, die Verbindungen der Formel II 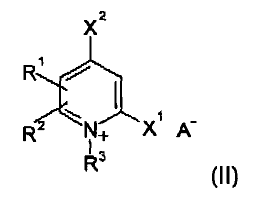 wobei R¹, R², R³, X¹, X² und A' wie oben definiert sind,
mitumfaßt sind,
sowie entsprechenden inneren Salzen, wobei A⁻ entfällt, und
B. mindestens einer CH-aciden Verbindung gemäß Anspruch 1,
sowie gegebenenfalls
C. mindestens einer Verbindung ausgewählt aus der Gruppe, die gebildet wird aus (a) aromatischen oder heteroaromatischen Aldehyden oder Ketonen, (b) Aminosäuren, (c) aus 2 bis 9 Aminosäuren aufgebauten Oligopeptiden (e) quartären Ammoniumverbindungen und (f) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen
als eine färbende Komponente in Oxidationshaartärbemitteln.

20. Verfahren zum Färben von keratinhaltigen Fasern, insbesondere menschlichen Haaren, worin ein Färbemittel gemäß den Ansprüchen 1 bis 18 auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for colouring fibres containing keratin, in particular human hair, comprising
**A.** at least one quaternized heterocyclic compound according to formula 1, 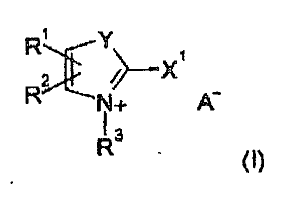 in which
• R¹ and R², independently of one another, are a hydrogen atom, a halogen atom, a hydroxy group, a C₁-C₄-hydroxyalkyl group, a C₁-C₆-aminoalkyl group, a C₁-C₄-dialkylamino-C₁-C₄-alkyl group, a linear or branched C₁-C₆-alkyl group, a C₂-C₆-alkenyl group, an optionally substituted aryl group, a sulphonic acid group, a carboxyl group, a nitro group, a cyano group or a group -NR⁴R⁵, where R⁴ and R⁵, independently of one another, are a hydrogen atom, a C₁-C₆-alkyl group, a C₂-C₆-alkenyl group, an aryl-C₁-C₄-alkyl group or a C₁-C₄-hydroxyalkyl group, where R¹ and R² can together form a fused-on 5- or 6-membered, aliphatic or aromatic ring, which in turn is substituted by the radicals R⁶ and R⁷, where R⁶ and R⁷, independently of one another, are the radicals which are defined under R¹,
• R³ is a C₁-C₄-hydroxyalkyl group, a C₁-C₆-aminoalkyl group, a C₁-C₄-dialkylamino-C₁-C₄-alkyl group, a di (C₁-C₄-hydroxyalkyl) amino-C₁-C₄-alkyl group, a linear or branched C₁-C₆-alkyl group, a C₂-C₆-alkenyl group, an optionally substituted aryl group,
• A- is a chloride, bromide, iodide, hexafluorophosphate, tetrachlorozincate, tetrafluoroborate, trifluoromethylsulphonate, methylsulphonate or p-toluenesulphonate,
• Y is an oxygen atom, a sulphur atom, a group -N=CH-, an optionally substituted methylene or an optionally substituted vinylene group or a group NR⁸, where R⁸ can be the same groups which are defined under R⁵
• X¹ is a halogen atom, a C₁-C₄-alkoxy group, a C₁-C₄-alkylmercapto group, a sulphonic acid group or a p-toluenesulphonyl group,
with the proviso that when Y is an optionally substituted vinylene group, the compounds of the formula II 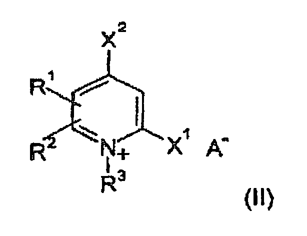 where
• R¹, R², R³ and A⁻ are as defined above,
• one of the radicals X¹ or X² is a halogen atom, a C₁-C₄-alkoxy group, a C₁-C₄-alkylmercapto group, a sulphonic acid group or a p-toluenesulphonyl group and the other is a hydrogen atom or the groups which are defined under R¹ and R²
are included,
and also corresponding internal salts, where A ⁻ is not present, and
**B.** at least one CH-acidic compound, where the CH-acidic compound is selected from compounds according to one of the formulae C1 to C21
**M¹-CH₂-M²** **(C1)**
in which
• M¹ is a group -COM³, COOM³, S (O) M³ or SO₂M³, in which M³ is a hydrogen atom or a C₁-C₆-alkyl group, or is a group -C(M⁴)=C(C≡N)₂, in which M⁴ is a hydrogen atom, a C₁-C₄-alkyl group or an aryl group,
• M² has the same meaning as M¹ or is a cyano group, a substituted or unsubstituted aryl group or aryl-C₁-C₄-alkyl group, a substituted or unsubstituted, saturated or unsaturated heterocycle,
compounds with the formula C2 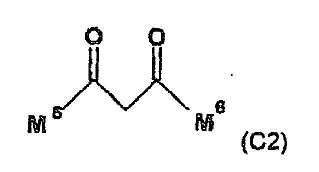 in which
• M⁵ is a cyano group, a substituted or unsubstituted aryl group or an aryl-C₁-C₄₋ alkyl group, a substituted or unsubstituted, saturated or unsaturated heterocycle or a group -COM⁷ or COOM⁷, in which M⁷ is a hydrogen atom or a C₁-C₆-alkyl group,
• M⁶ is a substituted or unsubstituted C₁-C₆-alkyl group, an acetyloxy group, a C₃-C₆-cycloalkyl group, a substituted or unsubstituted aryl group or aryl-C₁-C₉-alkyl group, a substituted or unsubstituted aminoaryl group, a substituted or unsubstituted, saturated or unsaturated heterocycle,
compounds with the formula C3 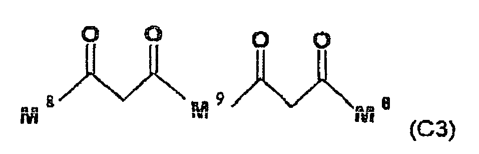 in which
• m⁸ is a cyano group, a substituted or unsubstituted aryl group or aryl-C₁-C₄-alkyl group, a substituted or unsubstituted, saturated or unsaturated heterocycle or a group -COM¹⁰ or COOM¹⁰, in which M¹⁰ is a hydrogen atom or a C₁-C₆-alkyl group,
• M⁹ is a substituted or unsubstituted aryl group or aryl-C₁-C₄-alkyl group, a substituted or unsubstituted aminoaryl group, a substituted or unsubstituted, saturated or unsaturated heterocycle,
pyrazole derivatives (a) which can be selected from the following the formulae C4 and C5 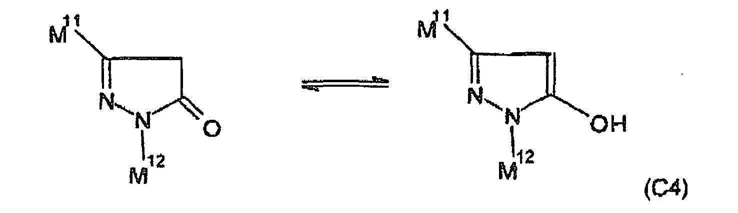 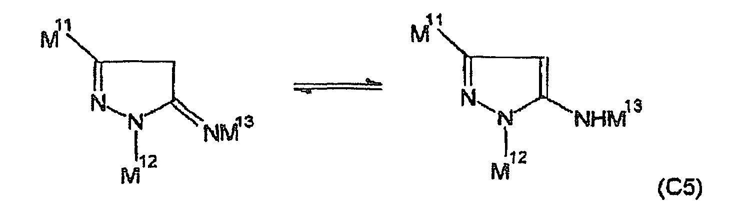 in which
• M¹¹ and M¹², independently of one another, are a substituted or unsubstituted C₁-C₆-alkyl group, an acetyloxy group, a C₃-C₆-cycloalkyl group, a substituted or unsubstituted aryl group or aryl-C₁-C₄-alkyl group, a substituted or unsubstituted aminoaryl group, a substituted or unsubstituted, saturated or unsaturated heterocycle,
• M¹³ is a hydrogen atom or a substituted or unsubstituted C₁-C₆-alkyl group,
(b) two pyrazole rings bonded via M¹¹ or _{M}¹² with the formula C4 or C5
barbituric acid derivatives with the following formula C6 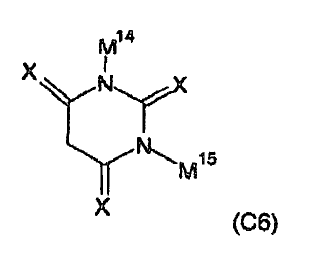
in which
• M¹⁴ and M¹⁵, independently of one another, are a C₁-C₆-alkyl group, a C₂-C₆-alkenyl group, a C₃-C₆-cycloalkyl group, an aryl-C₁-C₄-alkyl group, a substituted or unsubstituted aryl group, or a bicycle bonded via the radicals M¹⁴ or M¹⁵,
• X is an oxygen atom or a sulphur atom,
pyridine derivatives with the formulae C7a and C7b 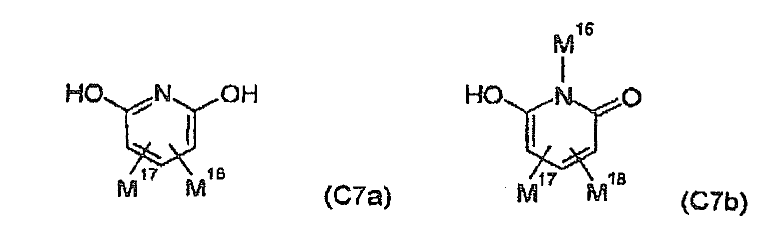 in which
• M¹⁶ is a substituted or unsubstituted C₁-C₆-alkyl group or substituted or unsubstituted aryl group,
• M¹⁷ is a hydrogen atom, a substituted or unsubstituted C₁-C₈-alkyl group or a substituted or unsubstituted aryl group,
• M¹⁸ is a hydrogen atom, a cyano group, a substituted or unsubstituted C₁-C₆-alkyl group, a group COOM¹⁹, in which M¹⁹ is a hydrogen atom or a substituted or unsubstituted C₁-C₆-alkyl group,
compounds with the following formula C8 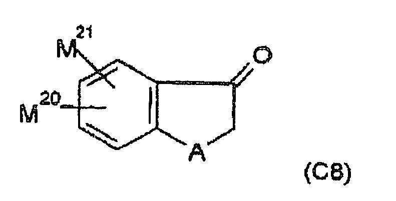 in which
• A is an oxygen atom, a sulphur atom, a sulphoxyl group, a sulphonyl group or a group NM^{20a}, in which M^{20a} is a hydrogen atom, a substituted or unsubstituted C₁-C₆-alkylene group,
• M²⁰ and M²¹, independently of one another, are a hydrogen atom, a chlorine atom, a bromine atom, a hydroxy group, a nitro group, a C₁-C₈-alkyl group, a C₁-C₆-alkoxy group, a carboxamide group, a sulphonamide group, a carboxyl group, a C₁-C₄-acyl, a cyano group or an amino group - NM²²M²³, in which M²² and M²³, independently of one another, are a hydrogen atom or a C₁-C₆-alkyl group,
compounds with the formulae C9 and C10 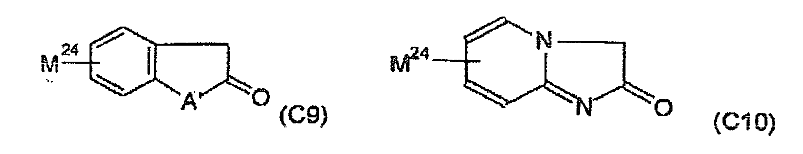 in which
• A' is an oxygen atom, a sulphur atom or a group NM²⁵, in which M²⁵ is a hydrogen atom or a substituted or unsubstituted C₁-C₆-alkyl group,
• M²⁴ is a hydrogen atom, a chlorine atom, a bromine atom, a hydroxy group, a nitro group, a C₁-C₆-alkyl group, a C₁-C₆-alkoxy group, a carboxamide group, a sulphonamide group, a carboxyl group, a C₁-C₄-acyl, a cyano group or an amino group - NM²⁶M²⁷, in which M²⁶ and M²⁷, independently of one another, are hydrogen and a C₁-C₆-alkyl group,
compounds with the formula C11 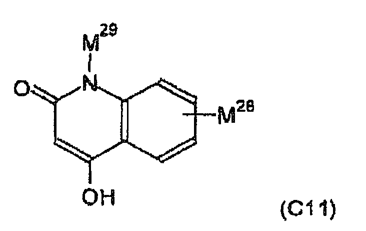
in which
• M²⁸ is a hydrogen atom, a hydroxy group, a substituted or unsubstituted C₁-C₈-alkyl group or a substituted or unsubstituted aryl or C₁-C₆-alkylaryl group;
• M²⁹ is a hydrogen atom or a C₁-C₄-alkyl group
indandione derivatives with the formula C12 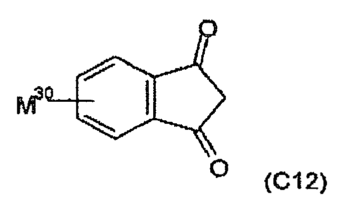 in which
• M³⁰ is a hydrogen atom, a chlorine atom, a bromine atom, a nitro group, a C₁-C₆-alkyl group, a C₁-C₆-alkoxy group, a carboxamide group, a sulphonamide group or a cyano group,
compounds with the formula C13 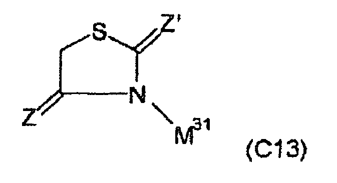 in which
• Z is an oxygen atom or a group NM³², in which M³² is a hydrogen atom or a C₁-C₆-alkyl group,
• Z' is a sulphur atom or a group NM³³, in which M³³ is a hydrogen atom or a C₁-C₆-alkyl group,
• M³¹ is a hydrogen atom, a C₁-C₆-alkyl group or a C₁-C₄-carboxyalkyl group,
dioxopyrazole compounds with the formula C14 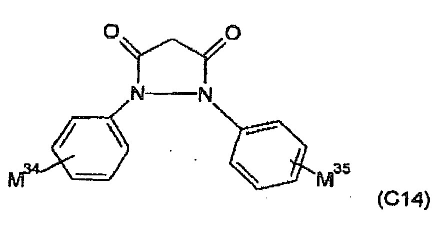
in which
• M³⁴ and M³⁵ independently of one another, are a hydrogen atom, a chlorine atom, a bromine atom, a hydroxy group, a nitro group, a C₁-C₆-alkyl group, a C₁-C₈-alkoxy group, a carboxamide group, a sulphonamide group, a carboxyl group, a C₁-C₄-acyl, a cyano group or an amino group -NM³⁶M³⁷, in which M³⁶ and M³⁷, independently of one another, are hydrogen or a C₁-C₈-alkyl group,
5-oxoimidazole derivatives with the formula C15 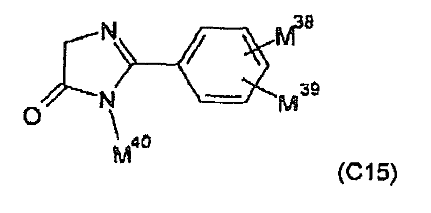 in which
• M³⁸ and M³⁹, independently of one another, are a hydrogen atom, a chlorine atom, a bromine atom, a hydroxy group, a nitro group, a C₁-C₆-alkyl group, a C₁-C₆-alkoxy group, a carboxamide group, a sulphonamide group, a carboxyl group, a C₁-C₄-acyl, a cyano group or an amino group - NM⁴¹M⁴², in which M⁴¹ and M⁴², independently of one another, are a hydrogen atom or a C₁-C₆-alkyl group,
• M⁴⁰ is a hydrogen atom or a C₁-C₆-alkyl group,
derivatives of dehydrobutyrolactone with the formula C16 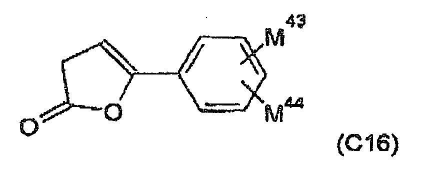 in which
• M⁴³ and M⁴⁴, independently of one another, are a hydrogen atom, a chlorine atom, a bromine atom, a hydroxy group, a nitro group, a C₁-C₆-alkyl group, a C₁-C₆-alkoxy group, a carboxamide group, a sulphonamide group, a carboxyl group, a C₁-C₄-acyl, a cyano group or an amino group - NM⁴⁵M⁴⁶, in which M⁴⁵ and M⁴⁶, independently of one another, are a hydrogen atom or a C₁-C₆-alkyl group,
compounds with the formula C17 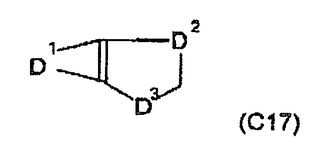
in which
• D¹ is a fused-on aromatic or heteroaromatic ring,
• D² is a carbonyl group, a group C=CD^{I}D^{II} or a group CD^{I}D^{II}, in which in D^{I} or D^{II} each have a substituent with a Hammett constant between 0.4 and 2.0 or both substituents in total have a Hammett constant between 0.4 and 2.0;
• D³ is a carbonyl group, an oxygen atom, a sulphur atom, a group NM⁴⁷, when D² is not oxygen, or a group C=S, a group C=NR⁴⁸, a sulphinyl group, a sulphonyl group, where R⁴⁷ and R⁴⁸, independently of one another, are a hydrogen atom or a C₁-C₄-alkyl radical,
hydroxypyrimidine derivatives with the formula C18 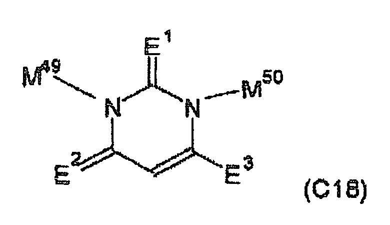
in which
• M⁴⁹ and M⁵⁰, independently of one another, are a hydrogen atom or a substituted or unsubstituted C₁-C₆-alkyl group,
• E¹ is an oxygen, a sulphur atom or a group NH,
• E² is a group NH or an oxygen atom,
• E³ is an amino group or a hydroxy group,
with the proviso that
a) when E¹ and E² are an oxygen atom, E³ is not a hydroxy group, and
b) when E¹ is a sulphur atom and E² is an oxygen atom, E³ is not a hydroxy group,
quaternized nitrogen compounds of the formula C19 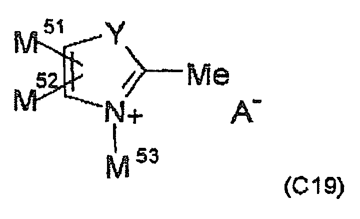 in which
• M⁵¹ and M⁵², independently of one another, are a hydrogen atom, a halogen atom, a hydroxy group, a C₁-C₄-hydroxyalkyl group, a C₁-C₆-aminoalkyl group, a C₁-C₄-dialkylamino-C₁-C₄-alkyl group, a linear or branched C₁-C₆-alkyl group, a C₂-C₆-alkenyl group, an optionally substituted aryl group, a sulphonic acid group, a carboxyl group, a formyl group, a nitro group, a cyano group, or a group -NM⁵⁴M⁵⁵, where M⁵⁴ and M⁵⁵, independently of one another, are a hydrogen atom, a C₁-C₆-alkyl group, a C₂-C₈-alkenyl group, an aryl-C₁-C₄-alkyl group or a C₁-C₄-hydroxyalkyl group, where M⁵¹ and M⁵² can together form a fused-on 5- or 6-membered, aliphatic or aromatic or heteroaromatic ring, which in turn is substituted by the radicals M⁵⁶ and M⁵⁷, where M⁵⁶ and M⁵⁷, independently of one another, are the radicals which are defined under M⁵¹,
• M⁵³ is a hydrogen atom, a C₁-C₄-hydroxyalkyl group, a C₁-C₆-aminoalkyl group, a C₁-C₄-dialkylamino-C₁-C₄-alkyl group, a linear or branched C₁-C₆-alkyl group, a C₂-C₆-alkenyl group, an optionally substituted aryl group, a C₁-C₄-sulphonylalkyl group, a C₁-C₄-carboxyalkyl group or a C₂-C₆-polyhydroxyalkyl group,
• Y is an oxygen atom, a sulphur atom, an optionally substituted methylene group or a group NM⁶⁰, where M⁶⁰ can be the same groups which are defined under M⁵⁵,
• A⁻ is a chloride, bromide, iodide, hexafluorophosphate, tetrachlorozincate, tetrafluoroborate, trifluoromethylsulphonate, methylsulphonate or p-toluenesulphonate,
onium compounds of the formulae C20 and C21 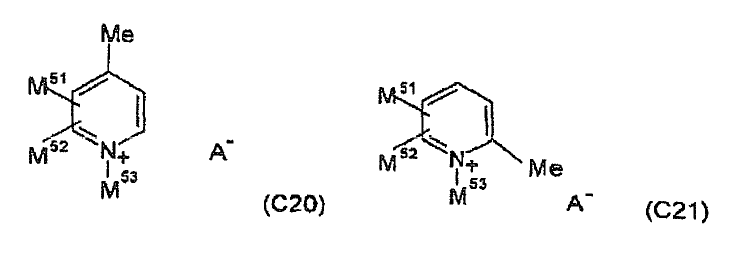
where M⁵¹, M⁵², M⁵³ and A⁻ are selected from the groups which are defined under compound C19.

2. Agent according to Claim 1, **characterized in that** the compounds with the formula I or formula II are selected from substances in which the cation is selected from 2-chloro-1-ethylquinolinium, 4-chloro-1-ethylquinolinium, 2-chloro-1-methylpyridinium, 4-chloro-1-methylpyridinium, 3-ethyl-2-methylmercaptobenzothiazolium, 3-ethyl-2-methylmercaptobenzoxazolium, 2-chloro-1,3-diethylbenzimidazolium, 2-chloro-3-ethylbenzoxazolium, 2-chloro-3-ethylbenzothiazolium and the counterion A⁻ is selected from chloride, bromide, iodide, hexafluorophosphate, tetrachlorozincate, tetrafluoroborate, trifluoromethylsulphonate, methylsulphonate or p-toluenesulphonate and 3-ethyl-benzothiazolium-2-sulphonate, 1-ethylquinolinium-4-sulphonate and 1-ethylquinolinium-2-sulphonate.

3. Agent according to one of Claims 1 or 2, **characterized in that** the CH-acidic compound is selected from 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline (Fischer's base), 2,3-dimethyl-benzothiazolium iodide, 2,3-dimethyl-benzothiazolium p-toluenesulphonate, 2,3-dimethylnaphtho[1,2-d]thiazolium p-toluenesulphonate, 3-ethyl-2-methylnaphtho[1,2-d]thiazolium p-toluenesulphonate, rhodanine, rhodanine-3-acetic acid, 1,4-dimethylquinolinium iodide, 1,2-dimethylquinolinium iodide, barbituric acid, thiobarbituric acid, 1,3-dimethylthiobarbituric acid, 1,3-diethylthiobarbituric acid, 1,3-diethylbarbituric acid, oxindole, 3-indoxyl acetate, 2-coumaranone, 5-hydroxy-2-coumaranone, 6-hydroxy-2-coumaranone, 1-methyl-3-phenylpyrazolin-5-one, indane-1,2-dione, indane-1,3-dione, indan-1-one, benzoylacetonitrile, 3-dicyanmethyleneindan-1-one, 1,3-diiminoisoindoline, 2-amino-4-imino-1,3-thiazoline hydrochloride, 5,5-dimethylcyclohexane-1,3-dione, 2H-1,4-benzoxazin-4H-3-one, 3-ethyl-2-methylbenzoxazolium iodide, 3-ethyl-2-methylbenzothiazolium iodide, 1-ethyl-4-methylquinolinium iodide, 1-ethyl-2-methylquinolinium iodide, 1,2,3-trimethylquinoxalinium iodide, 3-ethyl-2-methylbenzoxazolium p-toluenesulphonate, 3-ethyl-2-methylbenzothiazolium p-toluenesulphonate, 1-ethyl-4-methylquinolinium p-toluenesulphonate, 1-ethyl-2-methylquinolinium p-toluenesulphonate, and 1,2,3-trimethylquinoxalinium p-toluenesulphonate.

4. Agent according to one of Claims 1 to 3, **characterized in that** it additionally comprises a component C comprising at least one compound selected from (a) aromatic or heteroaromatic aldehydes or ketones, (b) amino acids, (c) oligopeptides composed of 2 to 9 amino acids and (d) compounds with a primary or secondary amino or hydroxy group selected from aromatic hydroxy compounds, primary or secondary aromatic amines and nitrogen-containing heterocyclic compounds.

5. Agent according to Claim 4, **characterized in that** it comprises, as component C, at least one compound selected from aromatic or heteroaromatic aldehydes or ketones.

6. Agent according to Claim 5, **characterized in that** the aromatic and/or heteroaromatic aldehydes and/or ketones present in component C are selected from
- 5-(4-dimethylaminophenyl)penta-2,4,dienal, 5-(4-diethylaminophenyl)penta-2,4-dienal, 5-(4-methoxyphenyl)penta-2,4,-dienal, 5-(3,4-dimethoxyphenyl)penta-2,4-dienal, 5-(2,4-dimethoxyphenyl)penta-2,4-dienal, 5-(4-piperidinophenyl)penta-2,4-dienal, 5-(4-morpholinophenyl)penta-2,4-dienal, 5-(4-pyrrolidinophenyl)penta-2,4-dienal, 6-(4-dimethylaminophenyl)hexa-3,5-dien-2-one, 6-(4-diethylaminophenyl)hexa-3,5-dien-2-one, 6-(4-methoxyphenyl)hexa-3,5-dien-2-one, 6-(3,4-dimethoxyphenyl)hexa-3,5-dien-2-one, 6-(2,4-dimethoxyphenyl)hexa-3,5-dien-2-one, 6-(4-piperidinophenyl)hexa-3,5-dien-2-one, 6-(4-morpholinophenyl)hexa-3,5-dien-2-one, 6-(4-pyrrolidinophenyl)hexa-3,5-dien-2-one, 5-(4-dimethylaminonaphth-1-yl)penta-2,4,dienal,
- 2-nitropiperonal, 5-nitropiperonal, 6-nitropiperonal, 5-hydroxy-2-nitropiperonal, 2-hydroxy-5-nitropiperonal, 2-chloro-6-nitropiperonal, 5-chloro-2-nitropiperonal, 2,6-dinitropiperonal,
- 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 4-hydroxy-3-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-dinitrobenzaldehyde, 2,6-dinitrobenzaldehyde, 2-hydroxy-3-methoxy-5-nitrobenzaldehyde, 4,5-dimethoxy-2-nitrobenzaldehyde, 5-nitrovanillin, 3,5-dinitrosalicylaldehyde, 5-bromo-3-nitrosalicylaldehyde, 3-nitro-4-formylbenzenesulphonic acid, 4-nitro-1-naphthaldehyde, 2-nitrocinnamaldehyde, 3-nitrocinnamaldehyde, 4-nitrocinnamaldehyde,
- carbazolealdehydes or carbazole ketones, in particular 9-methyl-3-carazolealdehyde, 9-ethyl-3-carbazolealdehyde, 3-acetylcarbazole, 3,6-diacetyl-9-ethylcarbazole, 3-acetyl-9-methylcarbazole, 1,4-dimethyl-3-carbazolealdehyde, 1,4,9-trimethyl-3-carbazolealdehyde,
- 4-trimethylammoniobenzaldehyde, 4-benzyldimethylammoniobenzaldehyde, 4-trimethylammoniocinnamaldehyde, 4-trimethylammonionaphthaldehyde, 2-methoxy-4-trimethylammoniobenzaldehyde, N-(4-acetylphenyl)trimethylammonium, 4-(N,N-diethyl)-N-methylammonio)benzaldehyde, N-(4-benzoylphenyl)trimethylammonium, N-(4-benzoylphenyl)-N,N-diethylmethylammonium, N-(4-formylphenyl)-N-methylpyrrolidinium, N-(4-formylphenyl)-N-methylpiperidinium, N-(4-formylphenyl)-N-methylmorpholinium, N-(4-acetylphenyl)-N-methylmorpholinium, N-(4-benzoylphenyl)-N-methylmorpholinium, 3-formyl-N-ethyl-N-methylcarbazolium, 3-formyl-9,9-dimethylcarbazolium, 1-(4-acetylphenyl)-3-methylimidazolium, 1-(4-acetylphenyl)-3-methyl-2-imidazolinium, 1-(4-benzoylphenyl)-3-methylimidazolium, 5-acetyl-1,3-diethyl-2-methylbenzimidazolium, 5-trimethylammonio-1-indanone salts, in particular the benzenesulphonates, p-toluenesulphonates, methanesulphonates, ethanesulphonates, propanesulphonates, perchlorate, sulphates, chlorides, bromides, iodides, tetrachlorozincates, methyl sulphates, trifluoromethanesulphonates, hexafluorophosphates, tetrafluoroborates,
- 4-formyl-1-methylpyridinium, 2-formyl-1-methylpyridinium, 4-formyl-1-ethylpyridinium, 2-formyl-1-ethylpyridinium, 4-formyl-1-benzylpyridinium, 2-formyl-1-benzylpyridinium, 4-formyl-1,2-dimethylpyridinium, 4-formyl-1,3-dimethylpyridinium, 4-formyl-1-methylquinolinium, 2-formyl-1-methylquinolinium, 4-acetyl-1-methylpyridinium, 2-acetyl-1-methylpyridinium, 4-acetyl-1-methylquinolinium, 5-formyl-1-methylquinolinium, 6-formyl-1-methylquinolinium, 7-formyl-1-methylquinolinium, 8-formyl-1-methylquiholinium, 5-formyl-1-ethylquinolinium, 6-formyl-1-ethylquinolinium, 7-formyl-1-ethylquinolinium, 8-formyl-1-ethylquinolinium, 5-formyl-1-benzylquinolinium, 6-formyl-1-benzylquinolinium, 7-formyl-1-benzylquinolinium, 8-formyl-1-benzylquinolinium, 6-formyl-1-allylquinolinium, 6-formyl-1-allylquinolinium, 7-formyl-1-allylquinolinium, and 8-formyl-1-allylquinolinium, 5-acetyl-1-methylquinolinium, 6-acetyl-1-methylquinolinium, 7-acetyl-1-methylquinolinium, 8-acetyl-1-methylquinolinium, 5-acetyl-1-ethylquinolinium, 6-acetyl-1-ethylquinolinium, 7-acetyl-1-ethylquinolinium, 8-acetyl-1-ethylquinolinium, 5-acetyl-1-benzylquinolinium, 6-acetyl-1-benzylquinolinium, 7-acetyl-1-benzylquinolinium, 8-acetyl-1-benzylquinolinium, 5-acetyl-1-allylquinolinium, 6-acetyl-1-allylquinolinium, 7-acetyl-1-allylquinolinium and 8-acetyl-1-allylquinolinium, 9-formyl-10-methylacridinium, 4-(2-formylvinyl)-1-methylpyridinium, 1,3-dimethyl-2-(4-formylphenyl)benzimidazolinium, 1,3-dimethyl-2-(4-formylphenyl)imidazolinium, 2-(4-formylphenyl)-3-methylbenzothiazolium, 2-(4-acetylphenyl)-3-methylbenzothiazolium, 2-(4-formylphenyl)-3-methylbenzoxazolium, 2-(5-formyl-2-furyl)-3-methylbenzothiazolium, 2-(5-formyl-2-thienyl)-3-methylbenzothiazolium, 2-(3-formylphenyl)-3-methylbenzothiazolium, 2-(4-formylnaphth-1-yl)-3-methylbenzothiazolium, 5-chloro-2-(4-formylphenyl)-3-methylbenzothiazolium, 2-(4-formylphenyl)-3,5-dimethylbenzothiazolium, 1-methyl-2-[2-(4-formylphenyl)ethenyl]pyridinium, 1-methyl-4-[2-(4-acetylphenyl)ethenyl]pyridinium, 1-benzyl-4-[2-(4-formylphenyl)-ethenyl]pyridinium, 1-methyl-4-[2-(4-formylphenyl)ethenyl]pyridinium, 1-methyl-2-[2-(4-formylphenyl)ethenyl]pyridinium, 1-methyl-4-[2-(4-formylphenyl)ethenyl]quinolinium, 1-methyl-2-[2-(4-formylphenyl)ethenyl]quinolinium, 1-methyl-2-[2-(5-formyl-2-furyl)-ethenyl]quinolinium, 1-methyl-2-[2-(5-formyl-2-thienyl)ethenyl]quinolinium, 1-methyl-2-[2-(4-formylphenyl)ethenyl]benzothiazolinium, 1,3-dimethyl-2[2-(4-formylphenyl)-ethenyl]benzimidazolinium, 1,3-dimethyl-2-[2-(4-formylphenyl)ethenyl]imidazolinium, 1-methyl-5-oxoindeno[1,2-b]pyridinium(4-methyl-4-azonio-9-fluorenone), 1-ethyl-5-oxoindeno[1,2-b]pyridinium(4-ethyl-4-azonio-9-fluorenone), 1-benzyl-5-oxoindeno[1,2-b]pyridinium(-4-benzyl-4-azonio-9-fluorenone), 2-methyl-5-oxoindeno[1,2-c]pyridinium, 2-methyl-9-oxoindeno[2,1-c]pyridinium, 1-methyl-9-oxoindeno[2,1-b]pyridinium, salts, in particular benzenesulphonate, p-toluenesulphonate, methanesulphonate, perchlorate, sulphate, chloride, bromide, iodide, tetrachlorozincate, methyl sulphate, trifluoromethanesulphonate, tetrafluoroborate,
- salicylaldehyde, vanillin, 4-hydroxy-3-methoxycinnamaldehyde (coniferylaldehyde), 2,4-dihydroxybenzaldehyde, 4-dimethylaminobenzaldehyde, 4-diethylaminobenzaldehyde, 4-dimethylamino-2-hydroxybenzaldehyde, 4-pyrrolidinobenzaldehyde, 4-morpholinobenzaldehyde, 4-piperidinobenzaldehyde, 4-dimethylaminoacetophenone, 4-hydroxynaphthaldehyde, 4-dimethylaminonaphthaldehyde, 4-dimethylaminobenzylideneacetone, 4-dimethylaminocinnamaldehyde, 2-dimethylaminobenzaldehyde, 2-chloro-4-dimethylaminobenzaldehyde, 4-dimethylamino-2-methylbenzaldehyde, trans-4-diethylaminocinnamaldehyde, 4-(dibutylamino)-benzaldehyde, 4-diphenylaminobenzaldehyde, 2,3,6,7-tetrahydro-1H,5H-benzo[ij]quinolizine-9-carboxaldehyde, 4-dimethylamino-2-methoxybenzaldehyde, 2,3,6,7-tetrahydro-8-hydroxy-1H,5H-benzo[ij]quinolizine-9-carboxaldehyde, 4-(1-imidazolyl)benzaldehyde, 2-morpholinobenzaldehyde, indole-3-carboxaldehyde, 1-methylindole-3-carboxaldehyde, N-ethylcarbazole-3-carboxaldehyde, 2-formylmethylene-1,3,3-trimethylindoline (tribase aldehyde)
- 1,3-diacetylbenzene, 1,4-diacetylbenzene, 1,3,5-triacetylbenzene, 2-benzoylacetophenone, 2-(4-methoxybenzoyl)acetophenone, 2-(2-furoyl)-acetophenone, 2-(2-pyridoyl)acetophenone, 2-(3-pyridoyl)acetophenone,
- 1-phenyl-1,2-propanedione, 1-phenyl-1,2-butanedione, 1-phenyl-3,3-dimethyl-1,2-butanedione, benzil, anisil, salicil, 5,5'-dibromosalicil, 2,2'-furil, 2,2'-thienil, 2,2'-4,4'-pyridil, 6,6'-dimethyl-4,4'-pyridil, 4-hydroxy-, 4-methoxy-, 4-chloro-, 4-methyl-, 4-dimethylamino-, 4,4'-dihydroxy-, -dimethyl-, -dibromo-, -dichloro-, -bisdimethylamino-, 2,4-dihydroxy-, 3,3'-dimethoxy, 2'-chloro-3,4-dimethoxy-, 3,4,5,3',4',5'-hexamethoxybenzil,
- isatin derivatives, such as 5-chloroisatin, 5-methoxyisatin, 5-nitroisatin, 6-nitroisatin, 5-sulphoisatin, isatin-5-sulphonic acid, isatin-4-carboxylic acid and isatin-5-carboxylic acid,
- N-substituted isatin derivatives, such as N-methylisatin, N-(2-hydroxyalkyl)isatin, N-(2-hydroxypropyl)isatin, N-(3-hydroxypropyl)isatin, N-(2,3-dihydroxypropyl)isatin, N-(2-sulphoethyl)isatin, (3-sulphopropyl)isatin, N-allylisatin, N-vinylisatin, N-benzylisatin, N-(4-methoxybenzyl)isatin, N-(4-carboxybenzyl)isatin, N-(4-sulphobenzyl)isatin, N-(2-dimethylaminoethyl)isatin, N-(2-pyrrolidinoethyl)isatin, N-(2-piperidinoethyl)isatin, (2-morpholinoethyl)isatin, N-(2-furylmethyl)isatin, N-(thien-2-ylmethyl)isatin, N-(pyrid-2-ylmethyl)isatin, N-(pyrid-3-yl-methyl)isatin, N-(pyrid-4-ylmethyl)isatin, N-allylisatin-5-sulphonic acid, 5-chloro-N-(2-hydroxyethyl)isatin, 5-methyl-N-(2-hydroxyethyl)isatin, 5,7-dichloro-N-allylisatin, 5-nitro-N-allylisatin, N-hydroxymethylisatin, N-hydroxymethyl-5-methylisatin, N-hydroxymethyl-5-chloroisatin, N-hydroxymethyl-5-sulphoisatin, N-hydroxymethyl-5-carboxyisatin, N-hydroxymethyl-5-nitroisatin, N-hydroxymethyl-5-bromoisatin, N-hydroxymethyl-5-methoxyisatin, N-hydroxymethyl-5,7-dichloroisatin, N-dimethylaminomethylisatin, N-diethylaminomethylisatin, N-(bis(2-hydroxyethyl)aminomethyl)isatin, N-(2-hydroxyethylaminomethyl)isatin, N-(bis(2-hydroxypropyl)aminomethyl)isatin, N-pyrrolidinomethylisatin, N-piperidinomethylisatin, N-morpholinomethylisatin, N-(1,2,4-triazolyl)methylisatin, N-(1-imidazolyl)methylisatin, N-carboxymethylaminomethylisatin, N-(2-carboxyethylaminomethyl)isatin, N-(3-carboxypropylaminomethyl)isatin, N-bis(2'-hydroxyethyl)aminomethyl)-5-methylisatin, N-piperidinomethyl-5-chloroisatin, N-(2-sulphoethylamino)isatin, and also the alkali metal and optionally ammonium salts of the acidic compounds, quinisatin and derivatives thereof, such as N-methylquinisatin,
- acetophenone, propiophenone, 2-hydroxyacetophenone, 3-hydroxyacetophenone, 4-hydroxyacetophenone, 2-hydroxypropiophenone, 3-hydroxypropiophenone, 4-hydroxypropiophenone, 2-hydroxybutyrophenone, 3-hydroxybutyrophenone, 4-hydroxybutyrophenone, 2,4-dihydroxyacetophenone, 2,5-dihydroxyacetophenone, 2,6-dihydroxyacetophenone, 2,3,4-trihydroxyacetophenone, 3,4,5-trihydroxyacetophenone, 2,4,6-trihydroxyacetophenone, 2,4,6-trimethoxyacetophenone, 3,4,5-trimethoxyacetophenone, 3,4,5-trimethoxyacetophenone diethyl ketal, 4-hydroxy-3-methoxyacetophenone, 3,5-dimethoxy-4-hydroxyacetophenone, 4-aminoacetophenone, 4-dimethylaminoacetophenone, 4-morpholinoacetophenone, 4-piperidinoacetophenone, 4-imidazolinoacetophenone, 2-hydroxy-5-bromoacetophenone, 4-hydroxy-3-nitroacetophenone, acetophenone-2-carboxylic acid, acetophenone-4-carboxylic acid, benzophenone, 4-hydroxybenzophenone, 2-aminobenzophenone, 4,4'-dihydroxybenzophenone, 2,4-dihydroxybenzophenone, 2,4,4'-trihydroxybenzophenone, 2,3,4-trihydroxybenzophenone, 2-hydroxy-1-acetonaphthone, 1-hydroxy-2-acetonaphthone, chromone, chromone-2-carboxylic acid, flavone, 3-hydroxyflavone, 3,5,7-trihydroxyflavone, 4',5,7-trihydroxyflavone, 5,6,7-trihydroxyflavone, quercetin, indanone, 9-fluorenone, 3-hydroxyfluorenone, anthrone, 1,8-dihydroxyanthrone,
- heterocyclic carbonyl compounds, such as 2-indolealdehyde, 3-indolealdehyde, 1-methylindole-3-aldehyde, 2-methylindole-3-aldehyde, 1-acetylindole-3-aldehyde, 3-acetylindole, 1-methyl-3-acetylindole, 2-(1,3,3-trimethyl-2-indolinylidene)acetaldehyde, 1-methylpyrrole-2-aldehyde, 1-methyl-2-acetylpyrrole, 1-pyridinealdehyde, 2-pyridinealdehyde, 3-pyridinealdehyde, 4-acetylpyridine, 2-acetylpyridine, 3-acetylpyridine, pyridoxale, quinoline-3-aldehyde, quinoline-4-aldehyde, antipyrine-4-aldehyde, furfural, 5-nitrofurfural, 2-thenoyltrifluoroacetone, chromone-3-aldehyde, 3-(5-nitro-2-furyl)acrolein, 3-(2-furyl)acrolein, imidazole-2-aldehyde,
- indanone derivatives, such as, for example, 1,2-indanedione, 2-oximo-1-indanone, indane-1,2,3-trione 2-oxime, 5-methoxyindane-1,2,3-trione 2-oxime, 2-nitro-1,3-indanedione
and physiologically compatible salts of the above compounds.

7. Agent according to one of Claims 4 to 6, **characterized in that** the primary and secondary aromatic amines of component C are selected from N,N-dimethyl-p-phenylenediamine, N,N-diethyl-p-phenylenediamine, N-(2-hydroxyethyl)-N-ethyl-p-phenylenediamine, N,N-bis(2-hydroxyethyl)-p-phenylenediamine, N-(2-methoxyethyl)-p-phenylenediamine, 2,3-dichloro-p-phenylenediamine, 2,4-dichloro-p-phenylenediamine, 2,5-dichloro-p-phenylenediamine, 2-chloro-p-phenylenediamine, 2,5-dihydroxy-4-morpholinoaniline, 2-aminophenol, 3-aminophenol, 4-aminophenol, 2-aminomethyl-4-aminophenol, 2-hydroxymethyl-4-aminophenol, o-phenylenediamine, m-phenylenediamine, p-phenylenediamine, 2,5-diaminotoluene, 2,5-diaminophenol, 2,5-diaminoanisole, 2,5-diaminophenetol, 4-amino-3-methylphenol, 2-(2,5-diaminophenyl)-ethanol, 2,4-diaminophenoxyethanol, 2-(2,5-diaminophenoxy)ethanol, 3-amino-4-(2-hydroxyethyloxy)phenol, 3,4-methylenedioxyphenol, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)phenol, 3-amino-2-chloro-6-methylphenol, 2-methyl-5-amino-4-chlorophenol, 5-(2-hydroxyethylamino)-4-methoxy-2-methylphenol, 4-amino-2-hydroxymethylphenol, 2-(diethylaminomethyl)-4-aminophenol, 4-amino-1-hydroxy-2-(2-hydroxyethylaminomethyl)benzene, 1-hydroxy-2-amino-5-methylbenzene, 1-hydroxy-2-amino-6-methylbenzene, 2-amino-5-acetamidophenol, 1,3-dimethyl-2,5-diaminobenzene, 5-(3-hydroxypropylamino)-2-methylphenol, 5-amino-4-methoxy-2-methylphenol, N,N-dimethyl-3-aminophenol, N-cyclopentyl-3-aminophenol, 5-amino-4-fluoro-2-methylphenol, 2,4-diamino-5-fluorotoluene, 2,4-diamino-5-(2-hydroxyethoxy)toluene, 2,4-diamino-5-methylphenetol, 3,5-diamino-2-methoxy-1-methylbenzene, 2-amino-4-(2-hydroxyethylamino)anisole, 2,6-bis(2-hydroxyethylamino)-1-methylbenzene, 1,3-diamino-2,4-dimethoxybenzene, 3,5-diamino-2-methoxytoluene, 2-aminobenzoic acid, 3-aminobenzoic acid, 4-aminobenzoic acid, 2-aminophenylacetic acid, 3-aminophenylacetic acid, 4-aminophenylacetic acid, 2,3-diaminobenzoic acid, 2,4-diaminobenzoic acid, 2,5-diaminobenzoic acid, 3,4-diaminobenzoic acid, 3,5-diaminobenzoic acid, 4-aminosalicylic acid, 5-aminosalicylic acid, 3-amino-4-hydroxybenzoic acid, 4-amino-3-hydroxybenzoic acid, 2-aminobenzenesulphonic acid, 3-aminobenzenesulphonic acid, 4-aminobenzenesulphonic acid, 3-amino-4-hydroxybenzenesulphonic acid, 4-amino-3-hydroxynaphthalene-1-sulphonic acid, 6-amino-7-hydroxynaphthalene-2-sulphonic acid, 7-amino-4-hydroxynapthalene-2-sulphonic acid, 4-amino-5-hydroxynapthalene-2,7-disulphonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechin, 4,6-diaminopyrogallol, 1-(2-hydroxy-5-aminobenzyl)-2-imidazolidinone, 4-amino-2-((4-[(5-amino-2-hydroxyphenyl)methyl]piperazinyl)-methyl)phenol, 3,5-diamino-4-hydroxypyrocatechin, 1,4-bis(4-aminophenyl)-1,4-diazacycloheptane, aromatic nitriles, such as 2-amino-4-hydroxybenzonitrile, 4-amino-2-hydroxybenzonitrile, 4-aminobenzonitrile, 2,4-diaminobenzonitrile, amino compounds containing nitro groups, such as 3-amino-6-methylamino-2-nitropyridine, picramic acid, [8-[(4-amino-2-nitrophenyl)azo]-7-hydroxynaphth-2-yl]trimethylammonium chloride, [8-((4-amino-3-nitrophenyl)azo)-7-hydroxynaphth-2-yl]trimethylammonium chloride (Basic Brown 17), 1-hydroxy-2-amino-4,6-dinitrobenzene, 1-amino-2-nitro-4-[bis(2-hydroxyethyl)amino]benzene, 1-amino-2-[(2-hydroxyethyl)amino]-5-nitrobenzene (HC Yellow No. 5), 1-amino-2-nitro-4-[(2-hydroxyethyl)amino]benzene (HC Red No. 7), 2-chloro-5-nitro-N-2-hydroxyethyl-1,4-phenylenediamine, 1-[(2-hydroxyethyl)amino]-2-nitro-4-aminobenzene (HC Red No. 3), 4-amino-3-nitrophenol, 4-amino-2-nitrophenol 6-nitro-o-toluidine, 1-amino-3-methyl-4-[(2-hydroxyethyl)amino]-6-nitrobenzene (HC Violet No. 1), 1-amino-2-nitro-4-[(2,3-dihydroxypropyl)amino]-5-chlorobenzene (HC Red No. 10), 2-(4-amino-2-nitroanilino)benzoic acid, 6-nitro-2,5-diaminopyridine, 2-amino-6-chloro-4-nitrophenol, 1-amino-2-(3-nitrophenylazo)-7-phenylazo-8-naphthol-3,6-disulphonic acid disodium salt (Acid blue No. 29), 1-amino-2-(2-hydroxy-4-nitrophenylazo)-8-naphthol-3,6-disulphonic acid disodium salt (Palatine chrome green), 1-amino-2-(3-chloro-2-hydroxy-5-nitrophenylazo)-8-naphthol-3,6-disulphonic acid disodium salt (Gallion), 4-amino-4'-nitrostilbene-2,2'-disuphonic acid disodium salt, 2,4-diamino-3',5'-dinitro-2'-hydroxy-5-methylazobenzene (Mordant brown 4), 4'-amino-4-nitrodiphenylamine-2-sulphonic acid, 4'-amino-3'-nitrobenzophenone-2-carboxylic acid, 1-amino-4-nitro-2-(2-nitrobenzylideneamino)benzene, 2-[2-diethylamino)ethylamino]-5-nitroaniline, 3-amino-4-hydroxy-5-nitrobenzenesulphonic acid, 3-amino-3'-nitrobiphenyl, 3-amino-4-nitroacenaphthene, 2-amino-1-nitronaphthalene, 5-amino-6-nitrobenzo-1,3-dioxole, anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)benzene, 2-nitro-1-amino-4-[bis(2-hydroxyethyl)amino]benzene, 4-amino-2-nitrodiphenylamine-2'-carboxylic acid, 1-amino-5-chloro-4-(2-hydroxyethylamino)-2-nitrobenzene, 4,4'-diaminostilbene and its hydrochloride, 4,4'-diaminostilbene-2,2'-disulphonic acid mono- or disodium salt, 4-amino-4'-dimethylaminostilbene and its hydrochloride, 4,4'-diaminodiphenylmethane, 4,4'-diaminodiphenyl sulphide, 4,4'-diaminodiphenyl sulphoxide, 4,4'-diaminodiphenylamine, 4,4'-diaminodiphenylamine-2-sulphonic acid, 4,4'-diaminobenzophenone, 4,4'-diaminodiphenyl ether, 3,3',4,4'-tetraaminodiphenyl, 3,3',4,4'-tetraaminobenzophenone, 1,3-bis(2,4-diaminophenoxy)propane, 1-8-bis(2-5-diaminophenoxy)-3,6-dioxaoctane, 1,3-bis(4-aminophenylamino)propane, 1,3-bis(4-aminophenylamino)-2-propanol, 1,3-bis[N-(4-aminophenyl)-2-hydroxyethylamino]-2-propanol, N,N-bis[2-(4-aminophenoxy)ethyl]methylamine, N-phenyl-1,4-phenylenediamine and bis(5-amino-2-hydroxyphenyl)methane.

8. Agent according to one of Claims 4 to 7, **characterized in that** the nitrogen-containing heterocyclic compounds of component C are selected from the group consisting of 2-aminopyridine, 3-aminopyridine, 4-aminopyridine, 2-amino-3-hydroxypyridine, 2,6-diaminopyridine, 2,5-diaminopyridine, 2-(aminoethylamino)-5-aminopyridine, 2,3-diaminopyridine, 2-dimethylamino-5-aminopyridine, 2-methylamino-3-amino-6-methoxypyridine, 2,3-diamino-6-methoxypyridine, 2,6-dimethoxy-3,5-diaminopyridine, 2,4,5-triaminopyridine, 2,6-dihydroxy-3,4-dimethylpyridine, N-[2-(2,4-diaminophenyl)aminoethyl]-N-(5-amino-2-pyridyl)amine, N-[2-(4-aminophenyl)aminoethyl]-N-(5-amino-2-pyridyl)amine, 2,4-dihydroxy-5,6-diaminopyrimidine, 4,5,6-triaminopyrimidine, 4-hydroxy-2,5,6-triaminopyrimidine, 2-hydroxy-4,5,6-triaminopyrimidine, 2,4,5,6-tetraaminopyrimidine, 2-methylamino-4,5,6-triaminopyrimidine, 2,4-diaminopyrimidine, 4,5-diaminopyrimidine, 2-amino-4-methoxy-6-methylpyrimidine, 3,5-diaminopyrazole, 3,5-diamino-1,2,4-triazole, 3-aminopyrazole, 3-amino-5-hydroxypyrazole, 1-phenyl-4,5-diaminopyrazole, 1-(2-hydroxyethyl)-4,5-diaminopyrazole, 1-phenyl-3-methyl-4,5-diaminopyrazole, 4-amino-2,3-dimethyl-1-phenyl-3-pyrazolin-5-one (4-aminoantipyrine), 1-phenyl-3-methylpyrazol-5-one, 2-aminoquinoline, 3-aminoquinoline, 8-aminoquinoline, 4-aminoquinaldine, 2-aminonicotinic acid, 6-aminonicotinic acid, 5-aminoisoquinoline, 5-aminoindazole, 6-aminoindazole, 5-aminobenzimidazole, 7-aminobenzimidazole, 5-aminobenzothiazole, 7-aminobenzothiazole, 2,5-dihydroxy-4-morpholinoaniline, and indole and indoline derivatives, such as 4-aminoindole, 5-aminoindole, 6-aminoindole, 7-aminoindole, 5,6-dihydroxyindole, 5,6-dihydroxyindoline, 4-hydroxyindoline and hydroxypyrimidine derivatives and the physiologically compatible salts of the above compounds.

9. Agent according to one of Claims 4 to 8, **characterized in that** the aromatic hydroxy compounds of component C are selected from 2-, 4-, 5-methylresorcinol, 2,5-dimethylresorcinol, resorcinol, 3-methoxyphenol, pyrocatechin, hydroquinone, pyrogallol, phloroglucine, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulphonic acid and 3,6-dihydroxy-2,7-naphthalenesulphonic acid.

10. Agent according to one of Claims 4 to 9, **characterized in that** the amino acids of component C are selected from arginine, histidine, tyrosine, phenylalanine, DOPA (dihydroxyphenylalanine), ornithine, proline, lysine, tryptophane, 6-aminocapronic acid and β-alanine.

11. Agent according to one of Claims 4 to 10, **characterized in that** the oligopeptides of component C are selected from glutathione or the oligopeptides present in the hydrolysates of collagen, keratin, casein, elastin, soy protein, wheat gluten or almond protein.

12. Agent according to one of Claims 4 to 11, **characterized in that** the compounds present in component C are selected from 4-formyl-1-ethylpyridinium p-toluenesulphonate, 4-formyl-1-methylquinolinium p-toluenesulphonate, 2-formyl-1-methylquinolinium p-toluenesulphonate, salicylaldehyde, vanillin, 4-hydroxy-3-methoxycinnamaldehyde, 4-diemthylaminobenzaldehyde, 4-dimethylamino-2-hydroxybenzaldehyde, 4-hydroxynaphthaldehyde, indole-3-carboxaldehyde and isatin, and in each case from the physiologically compatible salts of these compounds formed preferably with inorganic acids.

13. Agent according to one of Claims 1 to 12, **characterized in that** the compounds of the formula I are present in an amount of from 0.03 to 65 mmol, in particular from 1 to 40 mmol, based on 100 g of the total colorant.

14. Agent according to one of Claims 1 to 13, **characterized in that** it comprises colour boosters selected from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

15. Agent according to one of Claims 1 to 14, **characterized in that** it comprises direct dyes from the group of nitrophenylenediamines, nitroaminophenols, anthraquinone or indophenols, preferably in an amount of from 0.01 to 20% by weight, based on the total colorant.

16. Agent according to one of Claims 1 to 15, **characterized in that** ammonium or metal salts selected from the group of formates, carbonates, halides, sulphates, butyrates, valeriates, capronates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

17. Agent according to one of Claims 1 to 16, **characterized in that** it comprises oxidizing agents, in particular H₂O_{2,} in an amount of from 0.01 to 6% by weight, based on the application solution.

18. Agent according to one of Claims 1 to 17, **characterized in that** it comprises anionic, zwitterionic or nonionic surfactants.

19. Use of a combination of
**A.** at least one quaternized heterocyclic compound according to formula I 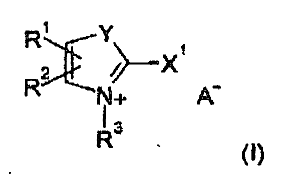 in which R¹, R², R³, X¹ and A⁻ are as defined above
with the proviso that when Y is an optionally substituted vinylene group, the compounds of the formula II 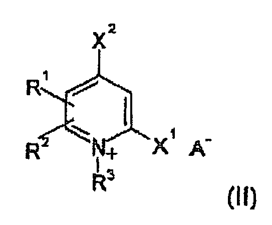 where R¹, R², R³, X¹, X² and A⁻ are as defined above,
are included
and corresponding internal salts, where A⁻ is not present, and
**B.** at least one CH-acidic compound according to Claim 1,
and optionally
**C.** at least one compound selected from the group which is formed from (a) aromatic or heteroaromatic aldehydes or ketones, (b) amino acids, (c) oligopeptides composed of 2 to 9 amino acids, (e) quaternary ammonium compounds and (f) compounds with primary or secondary amino or hydroxy group selected from aromatic hydroxy compounds, primary or secondary aromatic amines and nitrogen-containing heterocyclic compounds
as a colouring component in oxidation hair colorants.

20. Method of colouring fibres containing keratin, in particular human hair, in which a colorant according to Claims 1 to 18 is applied to the fibres containing keratin, left on the fibres for a certain amount of time, usually about 30 minutes, and then rinsed out again or washed out using a shampoo.

## Revendications

1. Agent pour la teinture de fibres kératiniques, en particulier de cheveux humains, contenant
A. au moins un composé hétérocyclique quaternisé répondant à la formule I : 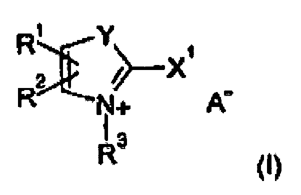 dans laquelle
- R¹ et R² représentent, indépendamment l'un de l'autre, un atome d'hydrogène, un atome d'halogène, un groupe hydroxyle, un groupe hydroxyalkyle en C₁-C₄, un groupe aminoalkyle en C₁-C₆, un groupe dialkyl(en C₁-C₄)aminoalkyle en C₁-C₄, un groupe alkyle en C₁-C₆ linéaire ou ramifié, un groupe alcényle en C₂-C₆, un groupe aryle éventuellement substitué, un groupe d'acide sulfonique, un groupe carboxyle, un groupe nitro, un groupe cyano ou un groupe -NR⁴R⁵, R⁴ et R⁵ représentant, indépendamment l'un de l'autre, un atome d'hydrogène, un groupe alkyle en Cₗ-C₆, un groupe alcényle en C₂-C₆, un groupe arylalkyle en C₁-C₄ ou un groupe hydroxyalkyle en C₁-C₄, R¹ et R² pouvant former ensemble un noyau aliphatique ou aromatique condensé à 5 ou 6 membres, qui est à son tour substitué avec les radicaux R⁶ et R⁷, R⁶ et R⁷ représentant, indépendamment l'un de l'autre, les radicaux qui ont été définis sous R¹ ;
- R³ représente un groupe hydroxyalkyle en C₁-C₄, un groupe aminoalkyle en C₁-C₆, un groupe dialkyl(en C₁-C₄)aminoalkyle en C₁-C₄, un groupe di(hydroxyalkyl en C₁-C₄)aminoalkyle en C₁-C₄, un groupe alkyle en C₁-C₆ linéaire ou ramifié, un groupe alcényle en C₂-C₆, un groupe aryle éventuellement substitué ;
- A- représente un groupe chlorure, un groupe bromure, un groupe iodure, un groupe hexafluorophosphate, un groupe tétrachlorozincate, un groupe tétrafluoroborate, un groupe trifluorométhylsulfonate, un groupe méthylsulfonate ou un groupe p-toluènesulfonate ;
- Y représente un atome d'oxygène, un atome de soufre, un groupe -N=CH-, un groupe méthylène éventuellement substitué ou un groupe vinylène éventuellement substitué ou encore un groupe NR⁸ où R⁸ peut représenter les mêmes groupes que ceux qui ont été définis sous R⁵ ;
- X¹ représente un atome d'halogène, un groupe alcoxy en C₁-C₄, un groupe alkyl(en C₁-C₄)mercapto, un groupe d'acide sulfonique ou un groupe p-toluènesulfonyle ;
avec cette mesure que, lorsque Y représente un groupe vinylène éventuellement substitué, les composés répondant à la formule II 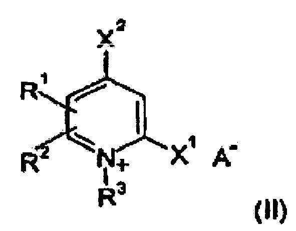 dans laquelle
- R¹, R², R³ et A⁻ sont tels qu'ils ont été définis ci-dessus ;
- un des radicaux X¹ ou X² représente un atome d'halogène, un groupe alcoxy en C₁-C₄, un groupe alkyl(en C₁-C₄)mercapto, un groupe d'acide sulfonique ou un groupe p-toluènesulfonyle, et l'autre représente un atome d'hydrogène ou bien les groupes qui ont été définis sous R¹ et R² ;
sont également repris,
ainsi que des sels internes correspondants dans lesquels A⁻ est absent ; et
B. au moins un composé acide à liaison CH, le composé acide à liaison CH étant choisi parmi des composés répondant à une des formules C1 à C21
**M¹-CH₂-M²** **(C1)**
dans laquelle
- M¹ représente un groupe -COM³, un groupe COOM³, un groupe S(O)M³ ou un groupe SO₂M³, où M³ représente un atome d'hydrogène ou un groupe alkyle en C₁-C₆, ou représente un groupe C(M⁴)=C(C≡N)2, où M⁴ représente un atome d'hydrogène, un groupe alkyle en C₁-C₄ ou un groupe aryle;
- M² a la même signification que celle de M¹ ou représente un groupe cyano, un groupe aryle ou arylalkyle en C₁-C₄ substitué ou non substitué, un groupe hétérocyclique substitué ou non substitué, saturé ou insaturé ;
des composés répondant à la formule C2 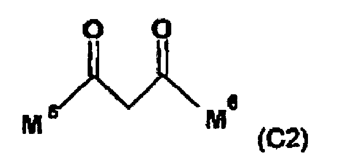 dans laquelle
- M⁵ représente un groupe cyano, un groupe aryle substitué ou non substitué ou un groupe arylalkyle en C₁-C₄, un groupe hétérocyclique substitué ou non substitué, saturé ou insaturé ou un groupe -COM⁷ ou un groupe COOM⁷ où M⁷ représente un atome d'hydrogène ou un groupe alkyle en C₁-C₆ ;
- M⁶ représente un groupe alkyle en C₁-C₆ substitué ou non substitué, un groupe acétyloxy, un groupe cycloalkyle en C₃-C₆, un groupe aryle ou arylalkyle en C₁-C₄ substitué ou non substitué, un groupe aminoaryle substitué ou non substitué, un groupe hétérocyclique substitué ou non substitué, saturé ou insaturé ;
des composés répondant à la formule C3 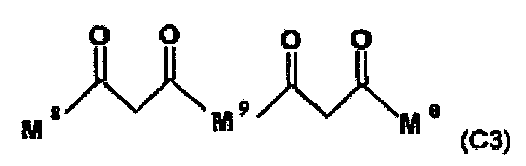
dans laquelle
- M⁸ représente un groupe cyano, un groupe aryle ou un groupe arylalkyle en C₁-C₄ substitué ou non substitué, un groupe hétérocyclique substitué ou non substitué, saturé ou insaturé ou un groupe -COM¹⁰ ou un groupe COOM¹⁰, où M¹⁰ représente un atome d'hydrogène ou un groupe alkyle en C₁-C₆;
- M⁹ représente un groupe aryle ou arylalkyle en C₁-C₄ substitué ou non substitué, un groupe aminoaryle substitué
ou non substitué, un groupe hétérocyclique substitué ou non substitué, saturé ou insaturé ;
des dérivés de pyrazole (a) qui peuvent être choisis à partir des formules suivantes C4 et C5 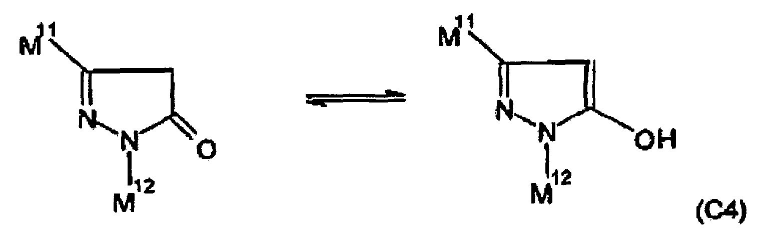 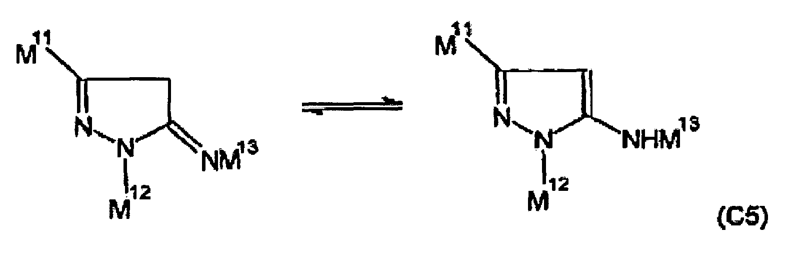 dans lesquelles
- M¹¹ et M¹² représentent, indépendamment l'un de l'autre, un groupe alkyle en C₁-C₆ substitué ou non substitué, un groupe acétyloxy, un groupe cycloalkyle en C₃-C₆, un groupe aryle ou arylalkyle en C₁-C₄ substitué ou non substitué, un groupe aminosryle substitué ou non substitué, un groupe hétérocyclique substitué ou non substitué, saturé ou insaturé ;
- M¹³ représente un atome d'hydrogène ou un groupe alkyle en C₁-C₆ substitué ou non substitué ;
(b) deux noyaux pyrazole liés via M¹¹ ou M¹², répondant aux formules C4 ou C5
des dérivés de l'acide barbiturique répondant à la formule suivante C6 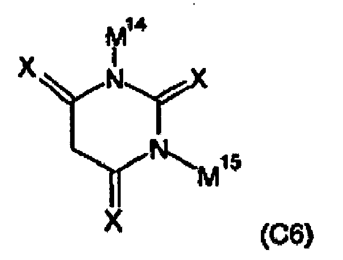
dans laquelle
- M¹⁴ et M¹⁵ représentent, indépendamment l'un de l'autre, un groupe alkyle en C₁-C₆, un groupe alcényle en C₂-C₆, un groupe cycloalkyle en C₃-C₆, un groupe arylalkyle en C₁-C₄, un groupe aryle substitué ou non substitué, ou un groupe bicyclique lié via les radicaux M¹⁴ ou M¹⁵ ;
- X représente un atome d'oxygène ou un atome de soufre ;
des dérivés de pyridine répondant aux formules C7a et C7b 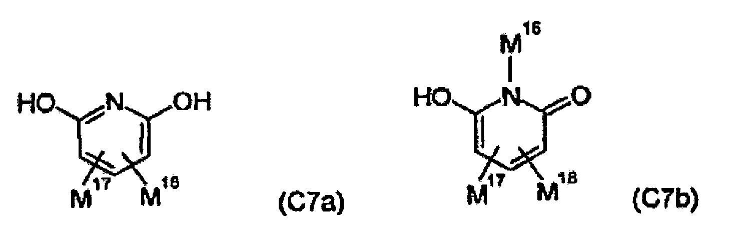
dans lesquelles
- M¹⁶ représente un groupe alkyle en C₁-C₆ substitué ou non substitué ou un groupe aryle substitué ou non substitué ;
- M¹⁷ représente un atome d'hydrogène, un groupe alkyle en C₁-C₆ substitué ou non substitué ou un groupe aryle substitué ou non substitué ;
- M¹⁸ représente un atome d'hydrogène, un groupe cyano, un groupe alkyle en C₁-C₆ substitué ou non substitué, un groupe COOM¹⁹, où M¹⁹ représente un atome d'hydrogène
ou un groupe alkyle en C₁-C₆ substitué ou non substitué ;
des composés répondant à la formule suivante C8 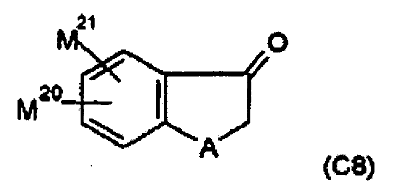 dans laquelle
- A représente un atome d'oxygène, un atome de soufre, un groupe sulfoxyle, un groupe sulfonyle ou un groupe NM^{20a} où M^{20a} représente un atome d'hydrogène, un groupe alkylène en C₁-C₆ substitué ou non substitué ;
- M²⁰ et M²¹ représentent, indépendamment l'un de l'autre, un atome d'hydrogène, un atome de chlore, un atome de brome, un groupe hydroxyle, un groupe nitro, un groupe alkyle en C₁-C₆, un groupe alcoxy en C₁-C₆, un groupe carboxamide, un groupe sulfonamide, un groupe carboxyle, un groupe acyle en C₁-C₄, un groupe cyano ou un groupe amino -NM²²M²³ dans lequel M²² et M²³ représentent, indépendamment l'un de l'autre, un atome d'hydrogène ou un groupe alkyle en C₁-C₆ ;
des composés répondant aux formules C9 et C10 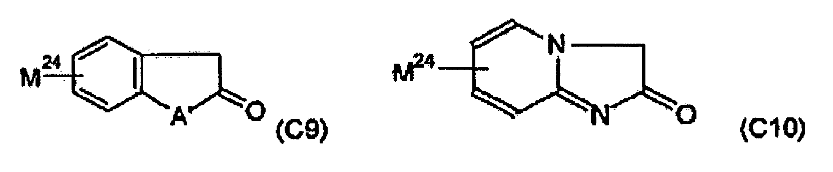
dans lesquelles
- A' représente un atome d'oxygène, un atome de soufre ou un groupe NM²⁵ où M²⁵ représente un atome d'hydrogène ou un groupe alkyle en C₁-C₆ substitué ou non substitué ;
- M²⁴ représente un atome d'hydrogène, un atome de chlore, un atome de brome, un groupe hydroxyle, un groupe nitro, un groupe alkyle en C₁-C₆, un groupe alcoxy en C₁-C₆, un groupe carboxamide, un groupe sulfonamide, un groupe carboxyle, un groupe acyle en C₁-C₄, un groupe cyano ou un groupe amino, un groupe -NM²⁶M²⁷ dans lequel M²⁶ et M²⁷ représentent, indépendamment l'un de l'autre, un atome d'hydrogène et un groupe alkyle en C₁-C₆;
des composés répondant à la formule C11 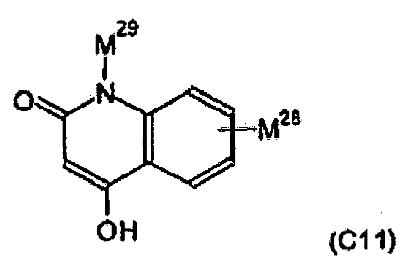
dans laquelle
- M²⁸ représente un atome d'hydrogène, un groupe hydroxyle, un groupe alkyle en C₁-C₆ substitué ou non substitué ou un groupe aryle ou alkyl(en C₁-C₆)aryle substitué ou non substitué ;
- M²⁹ représente un atome d'hydrogène ou un groupe alkyle en C₁-C₄;
des dérivés d'indanedione répondant à la formule C12 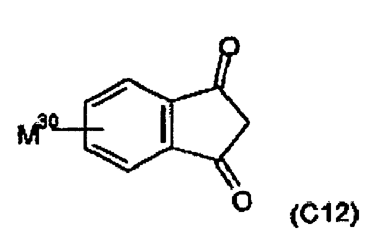 dans laquelle
- M³⁰ représente un atome d'hydrogène, un atome de chlore, un atome de brome, un groupe nitro, un groupe alkyle en C₁-C₆, un groupe alcoxy en C₁-C₆, un groupe carboxamide, un groupe sulfonamide ou un groupe cyano ;
des composés répondant à la formule C13 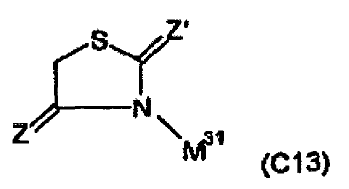
dans laquelle
- Z représente un atome d'oxygène ou un groupe NM³² où M³² représente un atome d'hydrogène ou un groupe alkyle en C₁-C₆;
- Z' représente un atome de soufre ou un groupe NM³³, où M³³ représente un atome d'hydrogène ou un groupe alkyle en C₁-C₆ ;
- M³¹ représente un atome d'hydrogène, un groupe alkyle en C₁-C₆ ou un groupe carboxyalkyle en C₁-C₄ ;
des composés de dioxopyrazole répondant à la formule C14 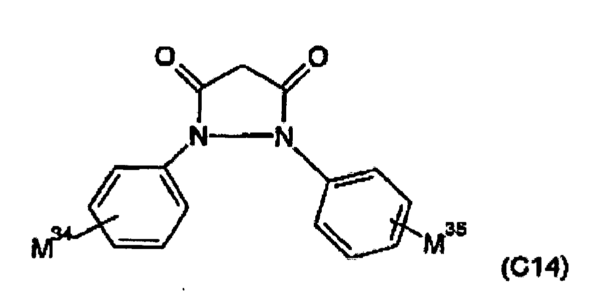
dans laquelle
- M³⁴ et M³⁵ représentent, indépendamment l'un de l'autre, un atome d'hydrogène, un atome de chlore, un atome de brome, un groupe hydroxyle, un groupe nitro, un groupe alkyle en C₁-C₆, un groupe alcoxy en C₁-C₆, un groupe carboxamide, un groupe sulfonamide, un groupe carboxyle, un groupe acyle en C₁-C₄, un groupe cyano ou un groupe amino -NM³⁶M³⁷ dans lequel M³⁶ et M³⁷ représentent, indépendamment l'un de l'autre, un atome d'hydrogène ou un groupe alkyle en C₁-C₆ ;
des dérivés de 5-oxoimidazole répondant à la formule C15 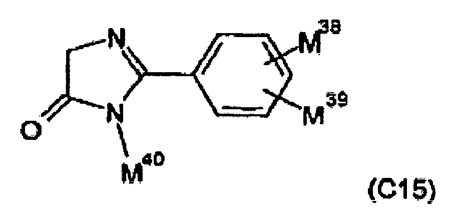
dans laquelle
- M³⁸ et M³⁹ représentent, indépendamment l'un de l'autre, un atome d'hydrogène, un atome de chlore, un atome de brome, un groupe hydroxyle, un groupe nitro, un groupe alkyle en C₁-C₆, un groupe alcoxy en C₁-C₆, un groupe carboxamide, un groupe sulfonamide, un groupe carboxyle, un groupe acyle en C₁-C₄, un groupe cyano ou un groupe amino -NM⁴¹M⁴² dans lequel M⁴¹ et M⁴² représentent, indépendamment l'un de l'autre, un atome d'hydrogène ou un groupe alkyle en C₁-C₆ ;
- M⁴⁰ représente un atome d'hydrogène ou un groupe alkyle en C₁-C₆ ;
des dérivés de déshydrobutyrolactone répondant à la formule C16 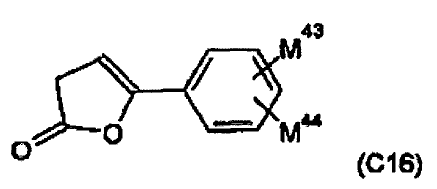
dans laquelle
- M⁴³ et M⁴⁴ représentent, indépendamment l'un de l'autre, un atome d'hydrogène, un atome de chlore, un atome de brome, un groupe hydroxyle, un groupe nitro, un groupe alkyle en C₁-C₆, un groupe alcoxy en C₁-C₆, un groupe carboxamide, un groupe sulfonamide, un groupe carboxyle, un groupe acyle en C₁-C₄, un groupe cyano ou un groupe amino -NM⁴⁵M⁴⁶ dans lequel M⁴⁵ et M⁴⁶ représentent, indépendamment l'un de l'autre, un atome d'hydrogène ou un groupe alkyle en C₁-C₆ ;
des composés répondant à la formule C17 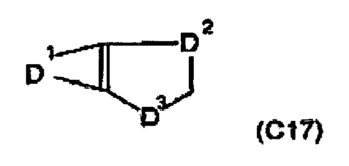
dans laquelle
- D¹ représente un noyau aromatique ou hétéroaromatique condensé;
- D² représente un groupe carbonyle, un groupe C=CD^{I}D^{II} ou un groupe CD'D" dans lequel D^{I} ou D^{II} représente respectivement un substituant possédant une constante de Hammett entre 0,4 et 2,0 ou deux substituants avec au total une constante de Hammett entre 0,4 et 2,0;
- D³ représente un groupe carbonyle, un atome d'oxygène, un atome de soufre, un groupe NM⁴⁷, lorsque D² ne représente pas un atome d'oxygène, ou bien un groupe C=S, un groupe C=NR⁴⁸, un groupe sulfinyle, un groupe sulfonyle, R⁴⁷ et R⁴⁸ représentant indépendamment l'un de l'autre un atome d'hydrogène ou un radical alkyle en C₁-C₄ ;
des dérivés d'hydroxypyrimidine répondant à la formule C18 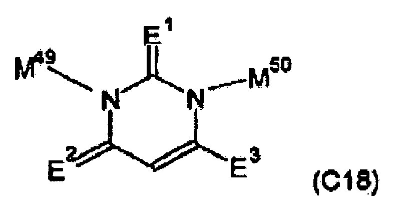
dans laquelle
- M⁴⁹ et M⁵⁰ représentent, indépendamment l'un de l'autre, un atome d'hydrogène ou un groupe alkyle en C₁-C₆ substitué
ou non substitué ;
- E¹ représente un atome d'oxygène, un atome de soufre ou un groupe NH ;
- E² représente un groupe NH ou un atome d'oxygène ;
- E³ représente un groupe amino ou un groupe hydroxyle ;
avec cette mesure que
a) lorsque E¹ et E² représentent un atome d'oxygène, E³ ne représente pas un groupe hydroxyle ; et
b) lorsque E¹ représente un atome de soufre et E² représente un atome d'oxygène, E³ ne représente pas un groupe hydroxyle ;
des composés azotés quaternisés répondant à la formule C19 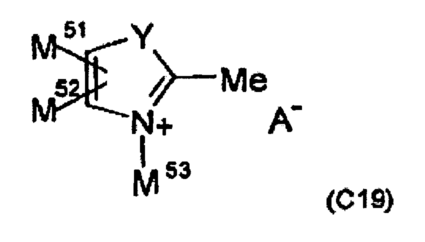
dans laquelle,
- M⁵¹ et M⁵² représentent, indépendamment l'un de l'autre, un atome d'hydrogène, un atome d'halogène, un groupe hydroxyle, un groupe hydroxyalkyle en C₁-C₄, un groupe aminoalkyle en C₁-C₆, un groupe dialkyl(en C₁-C₄)aminoalkyle en C₁-C₄, un groupe alkyle en C₁-C₆ linéaire
ou ramifié, un groupe alcényle en C₂-C₆, un groupe aryle éventuellement substitué, un groupe d'acide sulfonique, un groupe carboxyle, un groupe formyle, un groupe nitro, un groupe cyano ou un groupe -NM⁵⁴M⁵⁵, M⁵⁴ et M⁵⁵ représentant, indépendamment l'un de l'autre, un atome d'hydrogène, un groupe alkyle en C₁-C₆, un groupe alcényle en C₂-C₆, un groupe arylalkyle en C₁-C₄ ou un groupe hydroxyalkyle en C₁-C₄, M⁵¹ et M⁵² pouvant former ensemble un noyau aliphatique ou aromatique, respectivement hétéroaromatique condensé à 5 ou 6 membres, qui est à son tour substitué avec les radicaux M⁵⁶ et M⁵⁷, M⁵⁶ et M⁵⁷ représentant, indépendamment l'un de l'autre, les radicaux qui ont été définis sous M⁵¹ ;
- M⁵³ représente un atome d'hydrogène, un groupe hydroxyalkyle en C₁-C₄, un groupe aminoalkyle en C₁-C₆, un groupe dialkyl(en C₁-C₄)aminoalkyle en C₁-C₄, un groupe alkyle en C₁-C₆ linéaire ou ramifié, un groupe alcényle en C₂-C₆, un groupe aryle éventuellement substitué, un groupe sulfonylalkyle en C₁-C₄, un groupe carboxyalkyle en C₁-C₄ ou un groupe polyhydroxyalkyle en C₂-C₆,
- Y représente un atome d'oxygène, un atome de soufre, un groupe méthylène éventuellement substitué ou un groupe NM⁶⁰, M⁶⁰ pouvant représenter les mêmes groupes qui ont été définis sous M⁵⁵ ;
- A⁻ représente un groupe chlorure, un groupe bromure, un groupe iodure, un groupe hexafluorophosphate, un groupe tétrachlorozincate, un groupe tétrafluoroborate, un groupe trifluorométhylsulfonate, un groupe méthylsulfonate ou un groupe p-toluènesulfonate ;
des composés à radical onium répondant aux formules C20 et C21 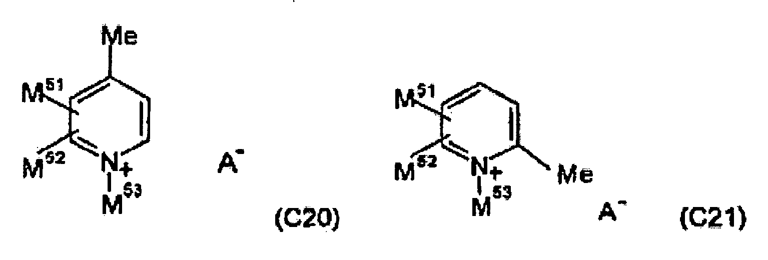
dans lesquelles M⁵¹, M⁵², M⁵³ et A⁻ sont choisis parmi les groupes qui ont été définis dans le cadre du composé C19,

2. Agent selon la revendication 1, **caractérisé en ce que** les composés répondant à la formule 1, respectivement Il sont choisis parmi des substances dans lequel le cation est choisi parmi le 2-chloro-1-éthylquinolinium, le 4-chloro-1-éthylquinolinium, le 2-chloro-1-méthylpyridinium, le 4-chloro-1-méthylpyridinium, le 3-éthyl-2-méthylmercaptobenzothiazolium, le 3-éthyl-2-méthylmercaptobenzoxazolium, le 2-chloro-1,3-diéthylbenzimidazolium, le 2-chloro-3-éthylbenzoxazolium, le 2-chloro-3-éthylbenzothiazolium et l'ion antagoniste A⁻ est choisi parmi un chlorure, un bromure, un iodure, un hexafluorophosphate, un tétrachlorozincate, un tétrafluoroborate, un trifluorométhylsulfonate, un méthylsulfonate ou un p-toluènesulfonate et le 3-éthyl-benzothiazolium-2-sulfonate, le 1-éthyl-quinolinium-4-sulfonate et le 1-éthyl-quinolinium-2-sulfonate.

3. Agent selon l'une quelconque des revendications 1 ou 2, **caractérisé en ce que** le composé acide à liaison CH est choisi parmi l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline (base de Fischer), l'iodure de 2,3-diméthyl-benzothiazolium, le p-toluènesulfonate de 2,3-diméthyl-benzothiazolium, le p-toluènesulfonate de 2,3-diméthylnaphto[1,2-d]thiazolium, le p-toluènesulfonate de 3-éthyl-2-méthylnaphto[1,2-d]thiazolium, la rhodanine, l'acide rhodanine-3-acétique, l'iodure de 1,4-diméthylquinolinium, l'iodure de 1,2-diméthylquinolinium, l'acide barbiturique, l'acide thiobarbiturique, l'acide 1,3-diméthylthiobarbiturique, l'acide 1,3-diéthylthiobarbiturique, l'acide 1,3-diéthylbarbiturique, l'oxindole, l'acétate de 3-indoxyle, la 2-coumaranone, la 5-hydroxy-2-coumaranone, la 6-hydroxy-2-coumaranone, la 3-méthyl-1-phényl-pyrazolin-5-one, l'indane-1,2-dione, l'indane-1,3-dione, l'indane-1-one, le benzoylacétonitrile, la 3-dicyanométhylène-indan-1-one, la 1,3-diiminoisoindoline, le chlorhydrate de 2-amino-4-imino-1,3-thiazoline, la 5,5-diméthylcyclohexane-1,3-dione, la 2H-1,4-benzoxazin-4H-3-one, l'iodure de 3-éthyl-2-méthyl-benzoxazolium, l'iodure de 3-éthyl-2-méthylbenzothiazolium, l'iodure de 1-éthyl-4-méthyl-quinolinium, l'iodure de 1-éthyl-2-méthylquinolinium, l'iodure de 1,2,3-triméthylquinoxalinium, le p-toluènesulfonate de 3-éthyl-2-méthyl-benzoxazolium-, le p-toluènesulfonate de 3-éthyl-2-méthyl-benzothiazolium, le p-toluènesulfonate de 1-éthyl-4-méthyl-quinolinium, le p-toluènesulfonate de 1-éthyl-2-méthylquinolinium et le p-toluènesulfonate de 1,2,3-triméthylquinoxalinium.

4. Agent selon l'une quelconque des revendications 1 à 3, **caractérisé en ce qu'**il contient en outre un composant C contenant au moins un composé choisi parmi (a) des aldéhydes ou des cétones aromatiques ou hétéroaromatiques, (b) des acides aminés, (c) des oligopeptides constitués par de 2 à 9 acides aminés et (d) des composés comprenant des groupes amino primaires ou secondaires ou des groupes hydroxyle choisis parmi des composés hydroxylés aromatiques, des amines aromatiques primaires ou secondaires et des composés hétérocycliques azotés.

5. Agent selon la revendication 4, **caractérisé en ce qu'**il contient, à titre de composant C, au moins un composé choisi parmi des aldéhydes ou des cétones aromatiques ou hétéroaromatiques.

6. Agent selon la revendication 5, **caractérisé en ce que** les aldéhydes et/ou les cétones aromatiques et/ou hétéroaromatiques que contient le composant C sont choisis parmi
- le 5-(4-diméthylaminophényl)penta-2,4-diénal, le 5-(4-diéthylaminophényl)penta-2,4-diénal, le 5-(4-méthoxyphényl)penta-2,4-diénal, le 5-(3,4-diméthoxyphényl)penta-2,4-diénal, le 5-(2,4-diméthoxyphényl)penta-2,4-diénal, le 5-(4-pipéridinophényl)-penta-2,4-diénal, le 5-(4-morpholinophényl)-penta-2,4-diénal, le 5-(4-pyrrolidinophényl)-penta-2,4-diénal, la 6-(4-diméthylaminophényl)hexa-3,5-dién-2-one, la 6-(4-diéthylaminophényl)hexa-3,5-dién-2-one, la 6-(4-méthoxyphényl)hexa-3,5-dién-2-one, la 6-(3,4-diméthoxyphényl)hexa-3,5-dién-2-one, la 6-(2,4-diméthoxyphényl)hexa-3,5-dién-2-one, la 6-(4-pipéridinophényl) hexa-3,5-dién-2-one, la 6-(4-morpholinophényl)hexa-3,5-dién-2-one, la 6-(4-pyrrolidinophényl)hexa-3,5-dién-2-one, le 5-(4-diméthylamino-napht-1-yl)penta-2,4-diénal,
- le 2-nitropipéronal, le 5-nitropipéronal, le 6-nitropipéronal, le 5-hydroxy-2-nitropipéronal, le 2-hydroxy-5-nitropipéronal, le 2-chloro-6-nitropipéronal, le 5-chloro-2-nitropipéronal, le 2,6-dinitropipéronal,
- le 2-nitrobenzaldéhyde, le 3-nitrobenzaldéhyde, le 4-nitrobenzaldéhyde, le 4-méthyl-3-nitrobenzaldéhyde, le 3-hydroxy-4-nitrobenzaldéhyde, le 4-hydroxy-3-nitrobenzaldéhyde, le 5-hydroxy-2-nitrobenzaldéhyde, le 2-hydroxy-5-nitrobenzaldéhyde, le 2-hydroxy-3-nitrobenzaldéhyde, le 2-fluoro-3-nitrobenzaldéhyde, le 3-méthoxy-2-nitrobenzaldéhyde, le 4-chloro-3-nitrobenzaldéhyde, le 2-chloro-6-nitrobenzaldéhyde, le 5-chloro-2-nitrobenzaldéhyde, le 4-chloro-2-nitrobenzaldéhyde, le 2,4-dinitrobenzaldéhyde, le 2,6-dinitrobenzaldéhyde, le 2-hydroxy-3-méthoxy-5-nitrobenzaldéhyde, le 4,5-diméthoxy-2-nitrobenzaldéhyde, la 5-nitrovanilline, le 3,5-dinitrosalicylaldéhyde, le 5-bromo-3-nitrosalicylaldéhyde, l'acide 3-nitro-4-formylbenzènesulfonique, le 4-nitro-1-naphtaldéhyde, le 2-nitrocinnamaldéhyde, le 3-nitrocinnamaldéhyde, le 4-nitrocinnamaldéhyde,
- des carbazolaldéhydes ou des carbazolcétones, en particulier le 9-méthyl-3-carbazolaldéhyde, le 9-éthyl-3-carbazolaldéhyde, le 3-acétylcarbazole, le 3,6-diacétyl-9-éthylcarbazole, le 3-acétyl-9-méthylcarbazole, le 1,4-diméthyl-3-carbazolaldéhyde, le 1,4,9-triméthyl-3-carbazolaldéhyde,
- des sels de 4-triméthylammoniobenzaldéhyde, de 4-benzyldiméthylammoniobenzaldéhyde, de 4-triméthylammoniocinnamaldéhyde, de 4-triméthylammonionaphtaldéhyde, de 2-méthoxy-4-triméthylammoniobenzaldéhyde, de N-(4-acétylphényl)-triméthylammonium, de 4-(N,N-diéthyl)-N-méthylammonio)-benzaldéhyde, de N-(4-benzoylphényl)-triméthylammonium, de N-(4-benzoylphényl)-N,N-diéthylméthylammonium, de N-(4-formylphényl)-N-méthylpyrrolidinium, de N-(4-formylphényl)-N-méthylpipéridinium, de N-(4-formylphényl)-N-méthylmorpholinium, de N-(4-acétylphényl)-N-méthylmorpholinium, de N-(4-benzoylphényl)-N-méthylmorpholinium, de 3-formyl-N-éthyl-N-méthylcarbazolium, de 3-formyl-9,9-diméthylcarbazolium, de 1-(4-acétylphényl)-3-méthylimidazolium, de 1-(4-acétylphényl)-3-méthyl-2-imidazolinium, de 1-(4-benzoylphényl)-3-méthylimidazolium, de 5-acétyl-1,3-diéthyl-2-méthylbenzimidazolium, de 5-triméthyl-ammonio-1-indanone, en particulier les benzènesulfonates, les p-toluènesulfonates, les méthanesulfonates, les éthanesulfonates, les propanesulfonates les perchlorates, les sulfates, les chlorures, les bromures, les iodures, les tétrachlorozincates, les méthylsulfates, les trifluorométhanesulfonates, les hexafluorophosphates, les tétrafluoroborates,
- des sels de 4-formyl-1-méthylpyridinium, de 2-formyl-1-méthylpyridinium, de 4-formyl-1-éthylpyridinium, de 2-formyl-1-éthylpyridinium, de 4-formyl-1-benzylpyridinium, de 2-formyl-1-benzylpyridinium, de 4-formyl-1,2-diméthylpyridinium, de 4-formyl-1,3-diméthylpyridinium, de 4-formyl-1-méthylquinolinium, de 2-formyl-1-méthylquinolinium, de 4-acétyl-1-méthylpyridinium, de 2-acétyl-1-méthylpyridinium, de 4-acétyl-1-méthylquinolinium, de 5-formyl-1-méthylquinolinium, de 6-formyl-1-méthylquinolinium, de 7-formyl-1-méthylquinolinium, de 8-formyl-1-méthylquinolinium, de 5-formyl-1-éthylquinolinium, de 6-formyl-1-éthylquinolinium, de 7-formyl-1-éthylquinolinium, de 8-formyl-1-éthylquinolinium, de 5-formyl-1-benzylquinolinium, de 6-formyl-1-benzylquinolinium, de 7-formyl-1-benzylquinolinium, de 8-formyl-1-benzylquinolinium, de 5-formyl-1-allylquinolinium, de 6-formyl-1-allylquinolinium, de 7-formyl-1-allylquinolinium et de 8-formyl-1-allylquinolinium, de 5-acétyl-1-méthylquinolinium, de 6-acétyl-1-méthylquinolinium, de 7-acétyl-1-méthylquinolinium, de 8-acétyl-1-méthylquinolinium, de 5-acétyl-1-éthylquinolinium, de 6-acétyl-1-éthylquinolinium, de 7-acétyl-1-éthylquinolinium, de 8-acétyl-1-éthylquinolinium, de 5-acétyl-1-benzylquinolinium, de 6-acétyl-1-benzylquinolinium, de 7-acétyl-1-benzylquinolinium, de 8-acétyl-1-benzylquinolinium, de 5-acétyl-1-allylquinolinium, de 6-acétyl-1-allylquinolinium, de 7-acétyl-1-allylquinolinium. et de 8-acétyl-1-allylquinolinium, de 9-formyl-10-méthylacridinium, de 4-(2-formylvinyl)-1-méthylpyridinium, de 1,3-diméthyl-2-(4-formylphényl)-benzimidazolium, de 1,3-diméthyl-2-(4-formylphényl)-imidazolium, de 2-(4-formylphényl)-3-méthylbenzothiazolium, de 2-(4-acétylphényl)-3-méthylbenzothiazolium, de 2-(4-formylphényl)-3-méthylbenzoxazolium, de 2-(5-formyl-2-furyl)-3-méthylbenzothiazolium, de 2-(5-formyl-2-thiényl)-3-méthylbenzothiazolium, de 2-(3-formylphényl)-3-méthylbenzothiazolium, de 2-(4-formylnapht-1-yl)-3-méthylbenzothiazolium, de 5-chloro-2-(4-formylphényl)-3-méthylbenzothiazolium, de 2-(4-formylphényl)-3,5-diméthylbenzothiazolium, de 1-méthyl-2-[2-(4-formylphényl)-éthényl]-pyridinium, de 1-méthyl-4-[2-(4-acétylphényl)-éthényl]-pyridinium, de 1-benzyl-4-[2-(4-formylphényl)-éthényl]-pyridinium, de 1-méthyl-4-[2-(4-formylphényl)-éthényl]-pyridinium, de 1-méthyl-2-[2-(4-formylphényl)-éthényl]-pyridinium, de 1-méthyl-4-(2-(4-formylphényl)-éthényl]-quinolinium, de 1-méthyl-2-(2-(4-formylphényl)-éthényl]-quinolinium, de 1-méthyl-2-[2-(5-formyl-2-furyl)-éthényl]-quinolinium, de 1-méthyl-2-[2-(5-formyl-2-thiényl)-éthényl]-quinolinium, de 1-méthyl-2-[2-(4-formylphényl)-éthényl]-benzothiazolinium, de 1,3-diméthyl-2-[2-(4-formylphényl)-éthényl]-benzimidazolinium, de 1,3-diméthyl-2-[2-(4-formylphényl)-éthényl]-imidazolinium, de 1-méthyl-5-oxo-indéno[1,2-b]pyridinium(4-méthyl-4-azonio-9-fluorénone), de 1-éthyl-5-oxo-indéno[1,2-b]pyridinium(4-éthyl-4-azonio-9-fluorénone), de 1-benzyl-5-oxoindéno[1,2-b]pyridinium(4-benzyl-4-azonio-9-fluorénone), de 2-méthyl-5-oxo-indéno[1,2-c]pyridinium, de 2-méthyl-9-oxoindéno[2,1-c]pyridinium, de 1-méthyl-9-oxo-indéno[2,1-b]pyridinium, en particulier les benzènesulfonates, les p-toluènesulfonates, les méthanesulfonates, les perchlorates, les sulfates, les chlorures, les bromures, les iodures, les tétrachlorozincates, les méthylsulfates, les trifluorométhanesulfonates, les tétrafluoroborates,
- le salicylaldéhyde, la vanilline, le 4-hydroxy-3-méthoxycinnamaldéhyde (coniférylaldéhyde), le 2,4-dihydroxybenzaldéhyde, le 4-diméthylaminobenzaldéhyde, le 4-diéthylaminobenzaldéhyde, le 4-diméthylamino-2-hydroxybenzaldéhyde, le 4-pyrrolidinobenzaldéhyde, le 4-morpholinobenzaldéhyde, le 4-pipéridinobenzaldéhyde, la 4-diméthylaminoacétophénone, le 4-hydroxynaphtaldéhyde, le 4-diméthylaminonaphtaldéhyde, la 4-diméthylaminobenzylidènacétone, le 4-diméthylaminocinnamaldéhyde, le 2-diméthylaminobenzaldéhyde, le 2-chloro-4-diméthylaminobenzaldéhyde, le 4-diméthylamino-2-méthylbenzaldéhyde, le trans-4-diéthylaminocinnamaldéhyde, le 4-(dibutylamino)-benzaldéhyde, le 4-diphénylaminobenzaldéhyde, le 2,3,6,7-tétrahydro-1H,5H-benzo[ij]quinolizin-9-carboxaldéhyde, le 4-diméthylamino-2-méthoxybenzaldéhyde, le 2,3,6,7-tétrahydro-8-hydroxy-1 H,5H-benzo[ij]quinolizin-9-carboxaldéhyde, le 4-(1-imidazolyl)-benzaldéhyde, le 2-morpholinobenzaldéhyde, l'indol-3-carboxaldéhyde, le 1-méthylindol-3-carboxaldéhyde, le N-éthylcarbazol-3-carboxaldéhyde, la 2-formylméthylène-1,3,3-triméthylindoline (aldéhyde tribasique),
- le 1,3-diacétylbenzène, le 1,4-diacétylbenzène, le 1,3,5-triacétylbenzène, la 2-benzoyl-acétophénone, la 2-(4-méthoxybenzoyl)-acétophénone, la 2-(2-furoyl)-acétophénone, la 2-(2-pyridoyl)-acétophénone, la 2-(3-pyridoyl)-acétophénone, la 1-phényl-1,2-propanedione, la 1-phényl-1,2-butanedione, la 1-phényl-3,3-diméthyl-1,2-butanedione, le benzile, l'anisile, le salicile, le 5,5'-dibromosalicile, le 2,2'-furile, le 2,2'-thiénile, le 2,2'-, le 4,4'-pyridile, le 6,6'-diméthyl-4,4'-pyridile, le 4-hydroxy-, le 4-méthoxy-, le 4-chloro-, le 4-méthyl-, le 4-diméthylamino-, le 4,4'-dihydroxy-, -diméthyl-, -dibromo-, -dichloro-, -bis-diméthylamino-, le 2,4-dihydroxy-, le 3,3'-diméthoxy-, le 2'-chloro-3,4-diméthoxy-, le 3,4,5,3',4',5'-hexaméthoxy-benzile,
- des dérivés de l'isatine, tels que la 5-chloroisatine, la 5-méthoxyisatine, la 5-nitroisatine, la 6-nitroisatine, la 5-sulfoisatine, l'acide isatine-5-sulfonique, l'acide isatine-4-carboxylique et l'acide isatine-5-carboxylique,
- des dérivés de l'isatine substitués sur l'atome d'azote tels que la N-méthylisatine, la N-(2-hydroxyalkyl)-isatine, la N-(2-hydroxypropyl)-isatine, la N-(3-hydroxypropyl)-isatine, la N-(2,3-dihydroxypropyl)-isatine, la N-(2-sulfoéthyl)-isatine, la (3-sulfopropyl)-isatine, la N-allylisatine, la N-vinylisatine, la N-benzylisatine, la N-(4-méthoxybenzyl)-isatine, la N-(4-carboxybenzyl)-isatine, la N-(4-sulfobenzyl)-isatine, la N-(2-diméthylaminoéthyl)-isatine, la N-(2-pyrrolidinoéthyl)-isatine, la N-(2-pipéridinoéthyl)-isatine, la (2-morpholinoéthyl)-isatine, la N-(2-furylméthyl)-isatine, la N-(thién-2-ylméthyl)-isatine, la N-(pyrid-2-ylméthyl)-isatine, la N-(pyrid-3-ylméthyl)-isatine, la N-(pyrid-4-ylméthyl)-isatine, l'acide N-allylisatine-5-sulfonique, la 5-chloro-N-(2-hydroxyéthyl)-isatine, la 5-méthyl-N-(2-hydroxyéthyl)-isatine, la 5,7-dichloro-N-allylisatine, la 5-nitro-N-allylisatine, la N-hydroxyméthylisatine, la N-hydroxyméthyl-5-méthylisatine, la N-hydroxyméthyl-5-chloroisatine, la N-hydroxyméthyl-5-sulfoisatine, la N-hydroxyméthyl-5-carboxyisatine, la N-hydroxyméthyl-5-nitroisatine, la N-hydroxyméthyl-5-bromoisatine, la N-hydroxyméthyl-5-méthoxyisatine, la N-hydroxyméthyl-5,7-dichloroisatine, la N-diméthylaminométhylisatine, la N-diéthylaminométhylisatine, la N-(bis-(2-hydroxyéthyl)-aminométhyl)-isatine, la N-(2-hydroxyéthylaminométhyl)-isatine, la N-(bis-(2-hydroxypropyl)-aminométhyl)-isatine, la N-pyrrolidinométhylisatine, la N-pipéridinométhylisatine, la N-morpholinométhylisatine, la N-(1,2,4-triazolyl)-méthylisatine, la N-(1-imidazolyl)-méthylisatine, la N-carboxyméthylaminométhylisatine, la N-(2-carboxyéthylaminométhyl)-isatine, la N-(3-carboxypropylaminométhyl)-isatine, la N-(bis-(2'-hydroxyéthyl)-aminométhyl)-5-méthylisatine, la N-pipéridinométhyl-5-chloroisatine, la N-(2-sulfoéthylamino)-isatine, ainsi que les sels de métaux alcalins et éventuellement les sels d'ammonium des composés acides, la quinisatine et ses dérivés tels que la N-méthylquinisatine,
- l'acétophénone, la propiophénone, la 2-hydroxyacétophénone, la 3-hydroxyacétophénone, la 4-hydroxyacétophénone, la 2-hydroxypropiophénone, la 3-hydroxypropiophénone, la 4-hydroxypropiophénone, la 2-hydroxybutyrophénone, la 3-hydroxybutyrophénone, la 4-hydroxybutyrophénone, la 2,4-dihydroxyacétophénone, la 2,5-dihydroxyacétophénone, la 2,6-dihydroxyacétophénone, la 2,3,4-trihydroxyacétophénone, la 3,4,5-trihydroxyacétophénone, la 2,4,6-trihydroxyacétophénone, la 2,4,6-triméthoxyacétophénone, la 3,4,5-triméthoxyacétophénone, le 3,4,5-triméthoxy-acétophénone-diéthylcétal, la 4-hydroxy-3-méthoxy-acétophénone, la 3,5-diméthoxy-4-hydroxyacétophénone, la 4-amino-acétophénone, la 4-diméthylaminoacétophénone, la 4-morpholino-acétophénone, la 4-pipéridinoacétophénone, la 4-imidazolino-acétophénone, la 2-hydroxy-5-bromo-acétophénone, la 4-hydroxy-3-nitroacétophénone, l'acide acétophénone-2-carboxylique, l'acide acétophénone-4-carboxylique, la benzophénone, la 4-hydroxy-benzophénone, la 2-amino-benzophénone, la 4,4'-dihydroxy-benzophénone, la 2,4-dihydroxy-benzophénone, la 2,4,4'-trihydroxybenzophénone, la 2,3,4-trihydroxybenzophénone, la 2-hydroxy-1-acétonaphtone, la 1-hydroxy-2-acétonaphtone, la chromone, l'acide chromone-2-carboxylique, la flavone, la 3-hydroxyflavone, la 3,5,7-trihydroxyflavone, la 4',5,7-trihydroxyflavone, la 5,6,7-trihydroxyflavone, la quercétine, l'indanone, la 9-fluorénone, la 3-hydroxyfluorénone, l'anthrone, la 1,8-dihydroxyanthrone,
- des composés carbonylés hétérocycliques tels que le 2-indolaldéhyde, le 3-indolaldéhyde, le 1-méthylindol-3-aldéhyde, le 2-méthylindol-3-aldéhyde, le 1-acétylindol-3-aldéhyde, le 3-acétylindole, le 1-méthyl-3-acétylindole, le 2-(1,3,3-triméthyl-2-indolinylidène)acétaldéhyde, le 1-méthylpyrrol-2-aldéhyde, le 1-méthyl-2-acétylpyrrole, le 1-pyridinaldéhyde, le 2-pyridinaldéhyde, le 3-pyridinaldéhyde, la 4-acétylpyridine, la 2-acétylpyridine, la 3-acétylpyridine, le pyridoxal, le quinoléin-3-aldéhyde, le quinoléin-4-aldéhyde, l'antipyrine-4-aldéhyde, le furfural, le 5-nitrofurfural, la 2-thénoyl-trifluoro-acétone, le chromone-3-aldéhyde, la 3-(5-nitro-2-furyl)-acroléine, la 3-(2-furyl)-acroléine, l'imidazol-2-aldéhyde,
- des dérivés de l'indanone comme par exemple la 1,2-indanedione, la 2-oximo-1-indanone, l'indan-1,2,3-trione-2-oxime, le 5-méthoxyindane-1,2,3-trione-2-oxime, la 2-nitro-1,3-indanedione,
ainsi que des sels physiologiquement acceptables des composés mentionnés ci-dessus.

7. Agent selon l'une quelconque des revendications 4 à 6, **caractérisé en ce que** les amines aromatiques primaires et secondaires du composant C sont choisies parmi la N,N-diméthyl-p-phénylènediamine, la N,N-diéthyl-p-phénylènediamine, la N-(2-hydroxyéthyl)-N-éthyl-p-phénylènediamine, la N,N-bis-(2-hydroxyéthyl)-p-phénylènediamine, la N-(2-méthoxyéthyl)-p-phénylènediamine, la 2,3-dichloro-p-phénylènediamine, la 2,4-dichloro-p-phénylènediamine, la 2,5-dichloro-p-phénylènediamine, la 2-chloro-p-phénylènediamine, la 2,5-dihydroxy-4-morpholinoaniline, le 2-aminophénol, le 3-aminophénol, le 4-aminophénol, le 2-aminométhyl-4-aminophénol, le 2-hydroxyméthyl-4-aminophénol, la o-phénylènediamine, la m-phénylènediamine, la p-phénylènediamine, le 2,5-diaminotoluène, le 2,5-diaminophénol, le 2,5-diaminoanisole, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, le 3-amino-4-(2-hydroxyéthyloxy)phénol, le 3,4-méthylènedioxyphénol, le 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 3-amino-2-chloro-6-méthylphénol, le 2-méthyl-5-amino-4-chlorophénol, le 5-(2-hydroxyéthylamino)-4-méthoxy-2-méthylphénol, le 4-amino-2-hydroxyméthylphénol, le 2-(diéthylaminométhyl)-4-aminophénol, le 4-amino-1-hydroxy-2-(2-hydroxyéthylaminométhyl)-benzène, le 1-hydroxy-2-amino-5-méthyl-benzène, le 1-hydroxy-2-amino-6-méthyl-benzène, le 2-amino-5-acétamido-phénol, le 1,3-diméthyl-2,5-diaminobenzène, le 5-(3-hydroxypropylamino-)2-méthylphénol, le 5-amino-4-méthoxy-2-méthylphénol, le N,N-diméthyl-3-aminophénol, le N-cyclopentyl-3-aminophénol, 5-amino-4-fluoro-2-méthylphénol, le 2,4-diamino-5-fluorotoluène, le 2,4-diamino-5-(2-hydroxyéthoxy)-toluène, le 2,4-diamino-5-méthylphénétol, le 3,5-diamino-2-méthoxy-1-méthylbenzène, le 2-amino-4-(2-hydroxyéthylamino)-anisole, le 2,6-bis-(2-hydroxyéthylamino)-1-méthylbenzène, le 1,3-diamino-2,4-diméthoxybenzène, le 3,5-diamino-2-méthoxy-toluène, l'acide 2-aminobenzoïque, l'acide 3-aminobenzoïque, l'acide 4-aminobenzoïque, l'acide 2-aminophénylacétique, l'acide 3-aminophénylacétique, l'acide 4-aminophénylacétique, l'acide 2,3-diaminobenzoïque, l'acide 2,4-diaminobenzoïque, l'acide 2,5-diaminobenzoïque, l'acide 3,4-diaminobenzoïque, l'acide 3,5-diaminobenzoïque, l'acide 4-aminosalicylique, l'acide 5-aminosalicylique, l'acide 3-amino-4-hydroxybenzoïque, l'acide 4-amino-3-hydroxybenzoïque, l'acide 2-aminobenzènesulfonique, l'acide 3-aminobenzènesulfonique, l'acide 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diamino-pyrocatéchol, le 4,6-diaminopyrogallol, la 1-(2-hydroxy-5-aminobenzyl)-2-imidazolidinone, le 4-amino-2-((4-[(5-amino-2-hydroxyphényl)méthyl]-pipérazinyl)méthyl)phénol, le 3,5-diamino-4-hydroxypyrocatéchol, le 1,4-bis-(4-aminophényl)-1,4-diazacycloheptane, des nitriles aromatiques tels que le 2-amino-4-hydroxybenzonitrile, le 4-amino-2-hydroxybenzonitrile, le 4-aminobenzonitrile, le 2,4-diaminobenzonitrile, des composés amino contenant des groupes nitro, tels que la 3-amino-6-méthylamino-2-nitro-pyridine, l'acide picramique, le chlorure de [8-[(4-amino-2-nitrophényl)-azo]-7-hydroxynapht-2-yl]-triméthylammonium, le chlorure de [8-[(4-amino-3-nitrophényl)-azo]-7-hydroxynapht-2-yl]-triméthylammonium (Basic Brown 17), le 1-hydroxy-2-amino-4,6-dinitrobenzène, le 1-amino-2-nitro-4-[bis-(2-hydroxyéthyl)amino]-benzène, le 1-amino-2-[(2-hydroxyéthyl)amino]-5-nitrobenzène (HC Yellow n° 5), le 1-amino-2-nitro-4-[(2-hydroxyéthyl)amino]-benzène (HC Red n° 7), la 2-chloro-5-nitro-N-(2-hydroxyéthyl)-1,4-phénylènediamine, le 1-[(2-hydroxyéthyl)amino]-2-nitro-4-amino-benzène (HC Red n° 3), le 4-amino-3-nitrophénol, le 4-amino-2-nitrophénol, la 6-nitro-o-toluidine, le 1-amino-3-méthyl-4-[(2-hydroxyéthyl)amino]-6-nitrobenzène (HC violet n° 1), le 1-amino-2-nitro-4-[(2,3-dihydroxypropyl)amino)-5-chloro-benzène (HC Red n° 10), l'acide 2-(4-amino-2-nitroanilino)-benzoïque, la 6-nitro-2,5-diaminopyridine, le 2-amino-6-chloro-4-nitrophénol, le sel disodique de l'acide 1-amino-2-(3-nitrophénylazo)-7-phénylazo-8-naphtol-3,6-disulfonique (Acid blue n° 29), le sel disodique de l'acide 1-amino-2-(2-hydroxy-4-nitrophénylazo)-8-naphtol-3,6-disulfonique (Palatinchrome green), le sel disodique de l'acide 1-amino-2-(3-chloro-2-hydroxy-5-nitrophénylazo)-8-naphtol-3,6-disulfonique (gallion), le sel disodique de l'acide 4-amino-4'-nitrostilbène-2,2'-disulfonique, le 2,4-diamino-3',5'-dinitro-2'-hydroxy-5-méthylazobenzène (Mordant brown 4), l'acide 4'-amino-4-nitrodiphénylamine-2-sulfonique, l'acide 4'-amino-3'-nitro-benzophénone-2-carboxylique, le 1-amino-4-nitro-2-(2-nitro-benzylidènamino)benzène, la 2-[2-(diéthylamino)-éthylamino]-5-nitroaniline, l'acide 3-amino-4-hydroxy-5-nitrobenzènesulfonique, le 3-amino-3'-nitrobiphényle, le 3-amino-4-nitro-acénaphtène, le 2-amino-1-nitronaphtalène, le 5-amino-6-nitrobenzo-1,3-dioxol, des anilines, en particulier des anilines contenant des groupes nitro, telles que la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)benzène, le 2-nitro-1-amino-4-[bis-(2-hydroxyéthyl)amino]benzène, l'acide 4-amino-2-nitrodiphénylamine-2'-carboxylique, le 1-amino-5-chloro-4-(2-hydroxyéthylamino)-2-nitrobenzène, le 4,4'-diaminostilbène et son chlorhydrate, le sel monosodique ou disodique de l'acide 4,4'-diaminostilbène-2,2'-disulfonique, le 4-amino-4'-diméthylaminostilbène et son chlorhydrate, le 4,4'-diaminodiphénylméthane, le 4,4'-diaminodiphénylsulfure, le 4,4'-diaminodiphénylsulfoxyde, la 4,4'-diaminodiphénylamine, l'acide 4,4'-diaminodiphénylamin-2-sulfonique, la 4,4'-diaminobenzophénone, l'éther 4,4'-diaminodiphénylique, le 3,3',4,4'-tétraaminodiphényle, la 3,3',4,4'-tétraaminobenzophénone, le 1,3-bis-(2,4-diaminophénoxy)propane, le 1,8-bis-(2,5-diaminophénoxy)-3,6-dioxaoctane, le 1,3-bis-(4-aminophénylamino)propane, le 1,3-bis-(4-aminophénylamino)-2-propanol, le 1,3-bis-[N-(4-aminophényl)-2-hydroxyéthylamino]-2-propanol, la N,N-bis-[2-(4-aminophénoxy)-éthyl]-méthylamine, la N-phényl-1,4-phénylènediamine et le bis-(5-amino-2-hydroxyphényl)-méthane.

8. Agent selon l'une quelconque des revendications 4 à 7, **caractérisé en ce que** les composés hétérocycliques azotés du composant C sont choisis parmi le groupe constitué par la 2-aminopyridine, la 3-aminopyridine, la 4-aminopyridine, la 2-amino-3-hydroxy-pyridine, la 2,6-diaminopyridine, la 2,5-diaminopyridine, la 2-(aminoéthylamino)-5-aminopyridine, la 2,3-diaminopyridine, la 2-diméthylamino-5-aminopyridine, la 2-méthylamino-3-amino-6-méthoxypyridine, la 2,3-diamino-6-méthoxypyridine, la 2,6-diméthoxy-3,5-diaminopyridine, la 2,4,5-triaminopyridine, la 2,6-dihydroxy-3,4-diméthylpyridine, la N-[2-(2,4-diaminophényl)aminoéthyl]-N-(5-amino-2-pyridyl)-amine, la N-[2-(4-aminophényl)aminoéthyl]-N-(5-amino-2-pyridyl)-amine, la 2,4-dihydroxy-5,6-diaminopyrimidine, la 4,5,6-triaminopyrimidine, la 4-hydroxy-2,5,6-triaminopyrimidine, la 2-hydroxy-4,5,6-triaminopyrimidine, la 2,4,5,6-tétraaminopyrimidine, la 2-méthylamino-4,5,6-triaminopyrimidine, la 2,4-diaminopyrimidine, la 4,5-diaminopyrimidine, la 2-amino-4-méthoxy-6-méthylpyrimidine, le 3,5-diaminopyrazole, le 3,5-diamino-1,2,4-triazole, le 3-aminopyrazole, le 3-amino-5-hydroxypyrazole, le 1-phényl-4,5-diaminopyrazole, le 1-(2-hydroxyéthyl)-4,5-diaminopyrazole, le 1-phényl-3-méthyl-4,5-diaminopyrazole, la 4-amino-2,3-diméthyl-1-phényl-3-pyrazolin-5-one (4-aminoantipyrine), la 1-phényl-3-méthylpyrazol-5-one, la 2-aminoquinoléine, la 3-aminoquinoléine, la 8-aminoquinoléine, la 4-aminoquinaldine, l'acide 2-aminonicotinique, l'acide 6-aminonicotinique, la 5-aminoisoquinoléine, le 5-aminoindazole, le 6-aminoindazole, le 5-aminobenzimidazole, le 7-aminobenzimidazole, le 5-aminobenzothiazole, le 7-aminobenzothiazole, la 2,5-dihydroxy-4-morpholino-aniline, ainsi que des dérivés d'indole et d'indoline, tels que le 4-aminoindole, le 5-aminoindole, le 6-aminoindole, le 7-aminoindole, le 5,6-dihydroxyindole, la 5,6-dihydroxyindoline, la 4-hydroxyindoline et des dérivés de l'hydroxypyrimidine, et les sels physiologiquement acceptables des composés susmentionnés.

9. Agent selon l'une quelconque des revendications 4 à 8, **caractérisé en ce que** les composés hydroxylés aromatiques du composant C sont choisis parmi le 2-, le 4-, le 5-méthylrésorcinol, le 2,5-diméthylrésorcinol, le résorcinol, le 3-méthoxyphénol, le pyrocatéchol, l'hydroquinone, le pyrogallol, la phloroglucine, l'hydroxyhydroquinone, le 2-, le 3-, le 4-méthoxy-, le 3-diméthylamino-, le 2-(2-hydroxyéthyl)-, le 3,4-méthylènedioxy-phénol, l'acide 2,4-, l'acide 3,4-dihydroxy-benzoïque, -phénylacétique, l'acide gallique, l'acide 2,4,6-trihydroxybenzoïque, la 2,4,6-trihydroxy-acétophénone, le 2-, le 4-chlororésorcinol, le 1-naphtol, le 1,5-, le 2,3-, le 2,7-dihydroxynaphtalène, l'acide 6-diméthylamino-4-hydroxy-2-naphtalènesulfonique et l'acide 3,6-dihydroxy-2,7-naphtalènesulfonique.

10. Agent selon l'une quelconque des revendications 4 à 9, **caractérisé en ce que** les acides aminés du composant C sont choisis parmi l'arginine, l'histidine, la tyrosine, la phénylalanine, la DOPA (dihydroxyphénylalanine), l'ornithine, la proline, la lysine, le tryptophane, l'acide 6-aminocaproïque et la β-alanine.

11. Agent selon l'une quelconque des revendications 4 à 10, **caractérisé en ce que** les oligopeptides du composant C sont choisis parmi le glutathion ou les oligopeptides contenus dans les hydrolysats du collagène, de la kératine, de la caséine, de l'élastine, de la protéine de soja, du gluten de froment ou de la protéine d'amande.

12. Agent selon l'une quelconque des revendications 4 à 11, **caractérisé en ce que** les composés que contient le composant C sont choisis parmi le p-toluènesulfonate de 4-formyl-1-éthylpyridinium, le p-toluènesulfonate de 4-formyl-1-méthylquinolinium, le p-toluènesulfonate de 2-formyl-1-méthylquinolinium, le salicylaldéhyde, la vanilline, le 4-hydroxy-3-méthoxycinnamaldéhyde, le 4-diméthylaminobenzaldéhyde, le 4-diméthylamino-2-hydroxybenzaldéhyde, le 4-hydroxynaphtaldéhyde, l'indol-3-carboxaldéhyde et l'isatine, ainsi que les sels physiologiquement acceptables respectifs de ces composés, formés de préférence avec des acides inorganiques.

13. Agent selon l'une quelconque des revendications 1 à 12, **caractérisé en ce que** les composés répondant à la formule I sont contenus en une quantité de 0,03 à 65 millimoles, en particulier de 1 à 40 millimoles, rapportés à 100 g de la teinture totale.

14. Agent selon l'une quelconque des revendications 1 à 13, **caractérisé en ce qu'**il contient des renforçateurs de couleurs choisis parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidon-5-carboxylique, le pyrazole, le 1,2,4-triazole, la pipérazidine ou n'importe lequel de leurs mélanges.

15. Agent selon l'une quelconque des revendications 1 à 14, **caractérisé en ce qu'**il contient des colorants montant directement sur la fibre choisie parmi le groupe des nitrophénylènediamines, des nitroaminophénols, des anthraquinones ou des indophénols de préférence en une quantité de 0,01 à 20 % en poids, rapportés à la teinture totale.

16. Agent selon l'une quelconque des revendications 1 à 15, **caractérisé en ce qu'**on lui ajoute des sels d'ammonium ou des sels métalliques choisis parmi le groupe des formiates, des carbonates, des halogénures, des sulfates, des butyrates, des valérates, des caproates, des acétates, des lactates, des glycollates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum, ou encore de l'aluminium, du manganèse, du fer, du cobalt, du cuivre ou du zinc.

17. Agent selon l'une quelconque des revendications 1 à 16, **caractérisé en ce qu'**il contient des agents d'oxydation, en particulier du H₂O₂, en une quantité de 0,01 à 6 % en poids, rapportés à la solution d'utilisation.

18. Agent selon l'une quelconque des revendications 1 à 17, **caractérisé en ce qu'**il contient des agents tensioactifs anioniques, zwitterioniques ou non ioniques.

19. Utilisation d'une combinaison de
A. au moins un composé hétérocyclique quaternisé répondant à la formule I : 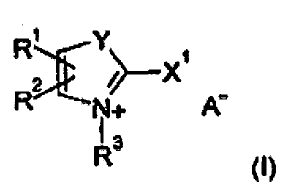 dans laquelle R¹, R², R³, X¹ et A⁻ sont tels qu'ils ont été définis ci-dessus,
avec cette mesure que, lorsque Y représente un groupe vinylène éventuellement substitué, les composés répondant à la formule II 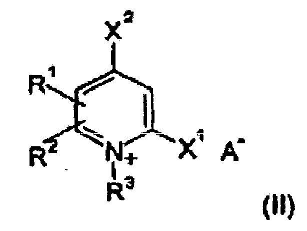 dans laquelle R¹, R², R³, X¹ X² et A⁻ sont tels qu'ils ont été définis ci-dessus;
sont également repris,
ainsi que les sels internes correspondants dans lesquels A- est absent, et
B. au moins un composé acide à liaison CH selon la revendication 1,
et, le cas échéant,
C. au moins un composé choisi parmi le groupe qui est formé par (a) des aldéhydes ou des cétones aromatiques ou hétéroaromatiques, (b) des acides aminés, (c) des oligopeptides constitués par de 2 à 9 acides aminés, (e) des composés d'ammonium quaternaires et (f) des composés comprenant des groupes amino primaires ou secondaires ou des groupes hydroxyle choisis parmi des composés hydroxylés aromatiques, des amines aromatiques primaires ou secondaires et des composés hétérocycliques azotés.
comme composant de coloration dans des teintures capillaires par oxydation.

20. Procédé pour la teinture de fibres kératiniques, en particulier de cheveux humains, dans lequel on applique une teinture selon les revendications 1 à 18 sur les fibres kératiniques, on la laisse agir pendant un certain temps, habituellement pendant environ 30 minutes sur les fibres et on l'élimine ensuite par rinçage ou bien on l'élimine par lavage avec un shampooing.
